(19) Europäisches Patentamt
European Patent Office
Office européen des brevets

(11) Numéro de publication : 0 419 327 B1

(12) # FASCICULE DE BREVET EUROPEEN

(45) Date de publication du fascicule du brevet : **27.07.94 Bulletin 94/30**

(21) Numéro de dépôt : **90402530.1**

(22) Date de dépôt : **14.09.90**

(51) Int. Cl.$^5$ : **C07C 323/60,** C07D 317/60, C07D 319/18, C07D 405/12, C07F 9/38, C07D 307/79, C07K 5/06, A61K 37/02

(54) **Dérivés d'amino-acides, leur procédé de préparation et leurs applications thérapeutiques.**

(30) Priorité : **15.09.89 FR 8912142**

(43) Date de publication de la demande : **27.03.91 Bulletin 91/13**

(45) Mention de la délivrance du brevet : **27.07.94 Bulletin 94/30**

(84) Etats contractants désignés : **AT BE CH DE DK ES FR GB GR IT LI LU NL SE**

(56) Documents cités :
**EP-A- 0 038 046**
**EP-A- 0 038 758**
**EP-A- 0 066 956**
**EP-A- 0 075 896**
**EP-A- 0 082 088**
**EP-A- 0 136 883**
**EP-A- 0 309 766**
**EP-A- 0 322 633**
**FR-A- 2 609 289**
**US-A- 4 401 677**
**PATENT ABSTRACTS OF JAPAN, vol. 10, no. 373 (C-391)[2430], 12 décembre 1986; & JP-A-61 165 362**
**CHEMICAL AND PHARMACEUTICAL BULLETIN, vol. 35, no. 6, juin 1987, pages 2388-2393, Tokyo, JP; K. TAKETOSHI et al.: "Sulfur-containing acylamino acids. II. Synthesis and angiotensin I converting enzyme-inhibitory activities of N-mercaptoalkanoyl-S-ethyl-L-cysteine"**

(73) Titulaire : **SOCIETE CIVILE BIOPROJET**
**30, rue des Francs Bourgeois**
**F-75003 Paris (FR)**

(72) Inventeur : **Plaquevent, Jean-Christophe**
**74, allée Messager**
**F-75960 Notre Dame de Bondeville (FR)**
Inventeur : **Danvy, Denis**
**16, Parc du Cailly**
**F-76130 Mont Saint Aignan (FR)**
Inventeur : **Monteil, Thierry**
**17, rue Henri-Vermont**
**F-76000 Rouen (FR)**
Inventeur : **Greciet, Hélène**
**16, Voie de Bas**
**F-27100 Val de Reuil (FR)**
Inventeur : **Duhamel, Lucette**
**32, rue Jacques Boutrolle**
**F-76130 Mont Saint Aignan (FR)**
Inventeur : **Duhamel, Pierre**
**32, rue Jacques Boutrolle**
**F-76130 Mont Saint Aignan (FR)**
Inventeur : **Gros, Claude**
**31, rue Robert de Flers**
**F-75015 Paris (FR)**
Inventeur : **Schwartz, Jean-Charles**
**9, villa Seurat**
**F-75014 Paris (FR)**
Inventeur : **Lecomte, Jeanne-Marie**
**30, rue des Francs-Bourgeois**
**F- 75003 - Paris (FR)**

(74) Mandataire : **Bernasconi, Jean et al**
**CABINET LEMOINE ET BERNASCONI**
**13, Boulevard des Batignolles**
**F-75008 Paris (FR)**

EP 0 419 327 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a trait à des dérivés d'aminoacides, inhibiteurs mixtes des enzymes enképhalinase (EC 3.4.24.11) et enzyme de conversion de l'angiotensine-convertase (EC 3.4.15.1 ; ACE).

Elle concerne également le procédé de préparation de ces dérivés d'amino-acides.

Elle concerne encore l'application de ces dérivés d'amino-acides à la préparation de médicaments.

Dans le brevet européen EP-A-0.038.758 (Roques et al.), il a été décrit des dérivés d'amino-acides présentant des propriétés inhibitrices de l'enképhalinase qui est une peptidase dégradant notamment les enképhalines. La méthionine et la leucine enképhaline sont des peptides qui ont été découverts dans le cerveau et qui sont des ligands endogènes du récepteur morphinique. Par ailleurs, le facteur auriculaire natriurétique (ANF) est un peptide endogène exerçant des effets vasorelaxant, diurétique et natriurétique potentiellement bénéfiques dans le traitement d'affections cardiovasculaires et rénales. L'ANF est un substrat de l'enképhalinase et les inhibiteurs de cette peptidase ralentissent sa dégradation, augmentent son taux plasmatique et induisent des effets antihypertenseur, diurétique et natriurétique (Lecomte et al, Proc. Natl. Ac. Sci., USA, sous presse).

On sait également, par exemple par le brevet français n° 2.623.498 déposé au nom de la demanderesse, que certains dérivés d'amino-acides exercent une activité inhibitrice sur l'enzyme de conversion de l'angiotensine I en angiotensine II (ACE), l'angiotensine II étant une substance vasomotrice active considérée comme l'agent responsable de diverses formes de l'hypertension. Ces composés sont donc utiles pour le traitement de l'hypertension et de l'insuffisance cardiaque.

Les dérivés d'amino-acides du type de ceux décrits dans le brevet européen EP-A-0.038.758 ou dans le brevet français n° 2.623.498 sont donc connus pour exercer une activité inhibitrice soit sur l'une ou l'autre des deux enzymes, enképhalinase et ACE, soit encore sur les deux enzymes à la fois. Mais dans ce dernier cas, leurs propriétés inhibitrices de l'enképhalinase et de l'ACE s'exercent à des degrés très différents. Aussi les recherches ont-elles jusqu'ici essentiellement porté sur la mise au point de dérivés d'amino-acides qui présentent une spécificité aussi grande que possible vis-à-vis de l'une ou de l'autre des deux activités enzymatiques précitées.

On conçoit, néanmoins, tout l'intérêt qu'il y aurait à disposer de dérivés d'amino-acides qui puissent inhiber avec la même efficacité les enzymes enképhalinase et ACE. Ces agents empêcheraient la formation de l'angiotensine II et favoriseraient à la fois les effets bénéfiques de l'ANF endogène. Qui plus est, l'inactivation d'un autre peptide endogène, la bradykinine, paraît dépendre à la fois de l'ACE et de l'enképhalinase : l'inhibition simultanée de ces deux peptidases est susceptible de favoriser les effets vasorelaxants connus de la bradykinine.

La demanderesse s'est donc attachée, dans le prolongement de ses précédents travaux, à mettre au point des dérivés d'amino-acides susceptibles d'inhiber, de façon équivalente, les deux enzymes, enképhalinase et ACE, en particulier grâce à un choix judicieux de certains substituants.

Un des buts de l'invention est donc de fournir des dérivés d'amino-acides qui soient des inhibiteurs mixtes des enzymes enképhalinase et ACE.

Un autre but de l'invention est de proposer un procédé de préparation de ces inhibiteurs mixtes.

Un autre but encore de l'invention est de fournir des compositions pharmaceutiques qui contiennent, à titre de principe actif, ces dérivés d'amino-acides.

Les dérivés d'amino-acides, inhibiteurs mixtes des enzymes enképhalinase et ACE, conformes à l'invention répondent aux formules générales :

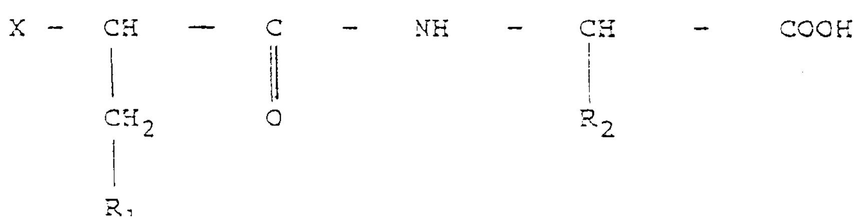

(Ia)

ou

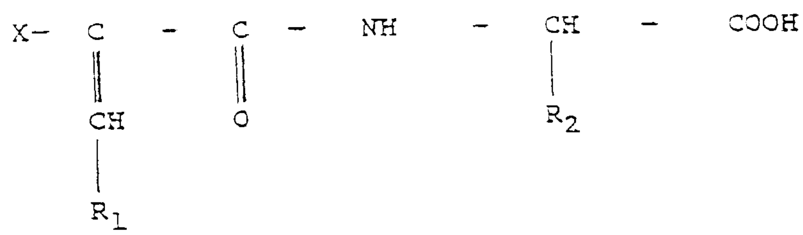

(Ib)

dans laquelle $R_1$ représente pour la formule (Ia) un groupe phényle mono- ou polysubstitué par un atome d'halogène, notamment le fluor ; Un groupe biphényle ou l'un des groupements suivants :

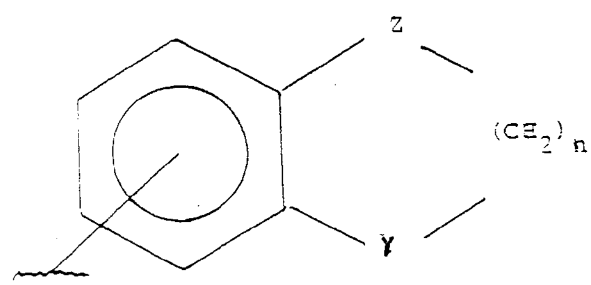

où Z, Y et n ont la signification donnée ci-dessous :

| Z | Y | n |
|---|---|---|
| O | O | 1 |
| O | $CH_2$ | 1 |
| $CH_2$ | $CH_2$ | 1 |
| O | O | 2 |
| $CH_2$ | $CH_2$ | 2 |
| O | $CH_2$ | 2 |

ou

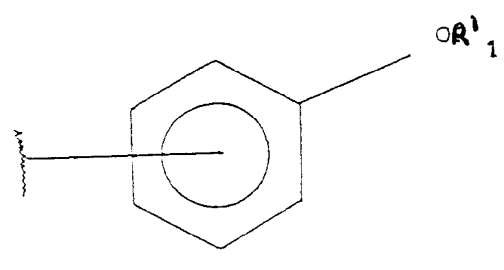

où

$R'_1$ représente un atome d'hydrogène, un groupe alkyle inférieur ; un groupe phényle ; un groupe phénylalkylène inférieur.

Pour la formule (Ib) , $R_1$ a la définition précitée et peut être également un groupe phényle,

$R_2$ représente un atome d'hydrogène ; un groupe alkyle inférieur ; un groupe hydroxyalkylène inférieur ; un

groupe phényle ; un groupe phénylalkylène inférieur ; un groupe hydroxyphénylalkylène inférieur ; un groupe aminoalkylène inférieur ; un groupe guanidinoalkylène inférieur ; un groupe mercaptoalkylène inférieur un groupe alkyle inférieur thioalkylène inférieur ; un groupe imidazolylalkylène inférieur un groupe indolylalkylène inférieur ; un groupe carbamylalkylène inférieur ; un groupe carboxyalkylène inférieur ou l'un des groupements suivants :

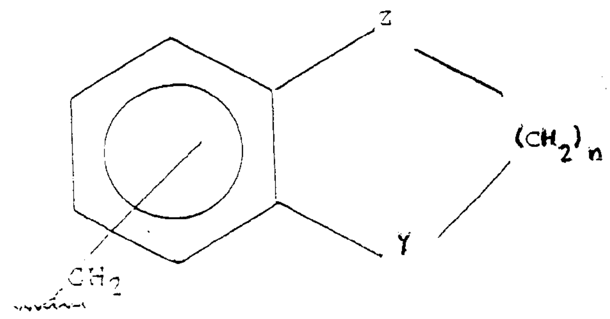

où Z et n ont les significations données ci-dessous :

| Z | Y | n |
|---|---|---|
| O | O | 1 |
| O | O | 2 |
| O | $CH_2$ | 1 |
| O | $CH_2$ | 2 |
| $CH_2$ | $CH_2$ | 1 |
| $CH_2$ | $CH_2$ | 2 |

X désigne un groupement responsable de la chélation de l'atome de zinc des enzymes, enképhalinase et ACE, et peut être choisi dans le groupe constitué par un mercaptométhyle ; l'acide hydroxamique ; un N-carboxyalkyle de formule

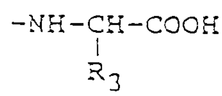

$R_3$ représente un radical alkyle inférieur, un radical alkyle inférieur benzyle ou un radical alcoxy inférieur benzyle ; des dérivés phosphorés de formule

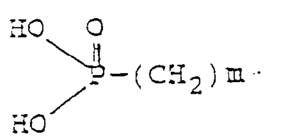

ou

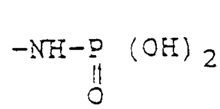

m = 0 ou 1

L'invention englobe également les sels d'addition pharmaceutiquement acceptables des dérivés d'amino-acides de formule (Ia) ou (Ib).

Par groupes alkyles inférieurs, on entend des groupes alkyles à chaine linéaire ou ramifiée contenant 1 à 6 atomes de carbone et, de préférence, 1 à 4 atomes de carbone.

Par groupes alkylènes inférieurs, on entend des groupes alkylènes contenant 1 à 6 atomes de carbone et, de

préférence, 1 à 4 atomes de carbone.

Les dérivés d'amino-acides conformes à l'invention comportent dans leur structure les amino-acides naturels et, plus particulèrement, la glycine, l'analine, la valine, la leucine, la sérine, la thréonine, la cystéine, la méthionine, l'acide aspartique, l'aspargine, l'acide glutamique, la glutamine, la lysine, l'arginine, la phénylalanine, la tyrosine, le tryptophane, l'histidine, à l'exception toutefois de la proline, ainsi que des amino-acides non naturels tels que la norvaline, la norleucine, la (méthylènedioxy-3,4 phényl)-3 alanine, la méthionine sulfoxyde.

Les dérivés d'amino-acides de formule (Ia) ou (Ib) conformes à l'invention sont des composés nouveaux, à l'exception des composés de formule (Ia) dans laquelle $R_1$ représente un groupe phényle mono- ou polysubstitué par un atome d'halogène ou encore un groupe phényle substitué par un radical hydroxy, déjà décrits dans les brevets européens EP-A-0038758 et EP-A-0 082 088.

Sont également connus par le brevet européen 0 066 956 (PFIZER INC.) et par l'article de T. KOMORI et al. paru dans Chem. Pharm. Bull. 35, 2388-2393 (1987) les composés de formule (Ia) dans laquelle X représente le groupe mercaptométhyle, $R_1$ un groupe phényle substitué par un groupe alcoxy ($C_1$-$C_3$) et $R_2$ un groupe alkyle ($C_1$-$C_2$) thioalkylène ($C_1$-$C_3$) et décrits pour leurs propriétés inhibitrices de l'enképhalinase.

Les dérivés d'amino-acides préférés conformes à l'invention sont les dérivés de formule (Ia) ou (Ib) dans lesquels le groupement X chélatant le zinc est constitué par un groupe mercaptométhyle.

Ces dérivés d'amino-acides peuvent être également utilisés sous une forme "prodrogue" où les fonctions mercaptométhyle et carboxyle sont protégées de la façon suivante :

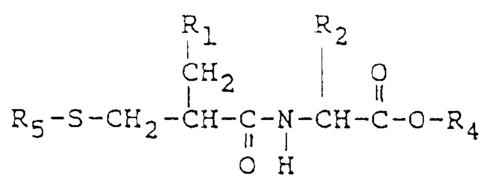

et

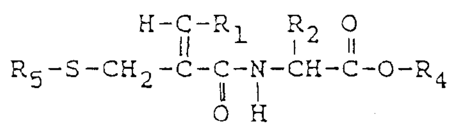

où $R_4$ représente, en particulier, un groupe alkyle linéaire ou ramifié, un groupe phényle ou phénylalkyle, ces deux derniers groupes étant éventuellement mono- ou polysubstitués sur le noyau phényle, ou des substituants linéaires ou ramifiés comportant un ou plusieurs atomes d'oxygène, et où $R_5$ représente en particulier un radical acyle aliphatique linéaire ou ramifié, un radical acyle aromatique éventuellement mono- ou polysubstitué ou un radical acyle linéaire ou ramifié comportant un ou plusieurs atomes d'oxygène.

Parmi les composés de formula (Ia) ou (Ib) plus particulièrement préférés, on peut citer :

- la N-(RS)-[oxo-1 (mercaptométhyl)-2 (méthylènedioxy-3,4 phényl)-3 propyl]glycine et ses formes optiquement pures,
- la N-(RS)-[oxo-1 (mercaptométhyl)-2 (méthylènedioxy-3,4 phényl)-3 propyl] -(S)-alanine, et ses formes optiquement pures,
- l'acide N-(RS)-[oxo-1 (mercaptométhyl)-2 (méthylènedioxy-3,4 phényl)-3 propyl] -(S)-amino-2-butyrique,
- la N-(RS)-[oxo-1 (mercaptométhyl)-2 (méthylènedioxy-3,4 phényl)-3 propyl]-(S)- norvaline,
- la N-(RS)-[oxo-1 (mercaptométhyl)-2 (méthylènedioxy-3,4 phényl)-3 propyl]-(S)-norleucine,
- la N-(RS)-[oxo-1 (mercaptométhyl)-2 (méthylènedioxy-3,4 phényl)-3 propyl]-(S)-leucine,
- le N-(RS)-[oxo-1 (mercaptométhyl)-2 (méthylènedioxy-3,4 phényl)-3 propyl]-(S)-tryptophane,
- la N-(RS)-[oxo-1 (mercaptométhyl)-2 (méthylènedioxy-3,4 phényl)-3 propyl]-(S)-phénylalanine,
- la N-(RS)-[oxo-1 (mercaptométhyl)-2 (méthylènedioxy-3,4 phényl)-3 propyl]-(S)-tyrosine,
- la N-(S)-[oxo-1 (mercaptométhyl)-2 (méthylènedioxy-3,4 phényl)-3 propyl]-(S)-sérine,
- la N-(S)-[oxo-1 (mercaptométhyl)-2 (méthylènedioxy-3,4 phényl)-3 propyl]-(S)-méthionine,
- la N-(S)-[oxo-1 (mercaptométhyl)-2 (méthylènedioxy-3,4 phényl)-3 propyl]-(RS)-méthionine sulfoxyde,
- la N-(S)-[oxo-1 (mercaptométhyl)-2 (méthylènedioxy-3,4 phényl)-3 propyl]-(RS)-(méthylènedioxy-3,4

phényl)-3 alanine,

- la N-(RS)-[oxo-1 (mercaptométhyl)-2 (éthylènedioxy-3,4 phényl)-3 propyl]-glycine,
- la N-(RS)-[oxo-1 (mercaptométhyl)-2 (éthylènedioxy-3,4 phényl)-3 propyl]-(S)-alanine,
- la N-(RS)-[oxo-1 (mercaptométhyl)-2 (méthylènedioxy-2,3 phényl)-3 propyl]-glycine,
- la N-(RS)-[oxo-1 (mercaptométhyl)-2 (phénoxy-4 phényl)-3 propyl]-glycine,
- la N-(RS)-[oxo-1 (mercaptométhyl)-2 (phénoxy-4 phényl)-3 propyl]-(S)-alanine,
- la N-(RS)-[oxo-1 (mercaptométhyl)-2 (phényl-4 phényl)-3 propyl]-glycine, et ses formes optiquement pures,
- la N-[oxo-1 (mercaptométhyl)-2 (phényl-4 phényl)-3 propyl]-(S)-alanine et ses formes optiquement pures,
- la N-[oxo-1 (mercaptométhyl)-2 (phényl-4 phényl)-3 propyl]-(S)-leucine,
- la N-(RS)-[oxo-1 (mercaptométhyl)-2 (fluoro-3 phényl)-3 propyl]-glycine,
- la N-(RS)-[oxo-1 (mercaptométhyl)-2 (fluoro-3 phényl)-3 propyl]-(S)-alanine,
- la N-(RS)-[oxo-1 (mercaptométhyl)-2 (difluoro-3,4 phényl)-3 propyl]-glycine,
- la N-(RS)- [oxo-1 (mercaptométhyl)-2 (difluoro-3,4 phényl)-3 propyl]-(S)-alanine,
- la N-(RS)-[oxo-1 (mercaptométhyl)-2 (difluoro-3,5 phényl)-3 propyl]-glycine, et ses formes optiquement pures,
- la N-(RS)-[oxo 1 (mercaptométhyl)-2 (difluoro-3,5 phényl)-3 propyl]-(S)-alanine,
- la N-(RS)-[oxo-1 (mercaptométhyl)-2 (indanyl-5′)-3 propyl]-glycine,
- la N-(RS)-[oxo-1 (mercaptométhyl)-2 (indanyl-5′)-3 propyl]-(S)-alanine,
- la N-(RS)-[oxo-1 (mercaptométhyl)-2 (dihydro-2′,3′benzofuranyl-5′)-3 propyl]-glycine,
- la N-(RS)-[oxo-1 (mercaptométhyl)-2 (dihydro-2′,3′benzofuranyl-5′)-3 propyl]-(S)-alanine,
- la N-(RS)-[oxo-1(mercaptométhyl)-2(méthoxy-4 phényl)-3 propyl]glycine,
- la N-(RS)-[oxo-1(mercaptométhyl)-2(méthoxy-4 phényl)-3 propyl]-(S)-alanine,
- la N-(S)-[oxo-1(mercaptométhyl)-2(méthoxy-4 phényl)-3 propyl]-glycine,
- la N-(S)-[oxo-1(mercaptométhyl)-2(méthoxy-4 phényl)-3 propyl]-(S)-alanine,
- la N-(RS)-[oxo-1(mercaptométhyl)-2(éthoxy-4 phényl)-3 propyl]-glycine,
- la N-(RS)-[oxo-1(mercaptométhyl)-2(éthoxy-4 phényl)-3 propyl]-(S)-alanine,
- la N-(E)-[oxo-1 mercaptométhyl)-2 ène-2 phényl-3 propyl]-(S)-alanine,
- la N-(E)-[oxo-1 (mercaptométhyl)-2 ène-2 phényl-3 propyl]-(S)-norvaline,
- la N-(E)-[oxo-1 (mercaptométhyl)-2 ène-2 phényl-3 propyl]-(S)-norleucine,
- la N-(E)-[oxo-1 (mercaptométhyl)-2 ène-2 phényl-3 propyl]-(RS)-(méthylènedioxy-3,4 phényl)-3 alanine,
- la N-(Z)-[oxo-1(mercaptométhyl)-2ène-2(méthylènedioxy-3,4 phényl)-3 propyl]glycine,
- le chlorhydrate de N-[N-(RS)-(carboxy-1 pentyl)-(RS)-(méthylènedioxy-3,4 phényl)-3 alanyl]-glycine,
- le chlorhydrate de N-[N-(RS)-(carboxy-1 phényl-2 éthyl)-(S)-phénylalanyl]-glycine,
- le chlorhydrate de N-[N-(RS)-(carboxy-1 phényl-2 éthyl)-(RS)-(méthylènedioxy-3,4 phényl)-3 alanyl]-glycine,
- la N-(RS)-[(dihydroxyphosphinyl) méthyl-2 oxo-1 (méthylènedioxy-3,4 phényl)-3 propyl]-(S)-alanine et son monosel de calcium,
- la N-(RS)-[(dihydroxyphosphinyl) méthyl-2 oxo-1 (méthylènedioxy-3,4 phényl)-3 propyl]-glycine et son monosel de calcium,
- la N-(RS)-[ (dihydroxyphosphinyl) méthyl-2 oxo-1 (phényl-4 phényl)-3 propyl]-(S) alanine et son monosel de calcium,
- la N-(RS)-[ (dihydroxyphosphinyl) méthyl-2 oxo-1 (phényl-4 phenyl)-3 propyl]-glycine et son monosel de calcium.

Les dérivés d'amino-acides de formule (Ia) ou (Ib) conformes à l'invention présentent un, deux ou trois atomes de carbone asymétriques et peuvent donc exister sous forme de mélange racémique ou sous des formes diastéréoisomères. Ces composés peuvent être utilisés sous forme racémique ou optiquement active. Le procédé de préparation de ces dérivés, décrit ci-après, utilise le racémique ou l'un des énantiomères comme produit de départ. Lorsque l'on utilise un produit de départ racémique, on peut séparer les stéréoisomères obtenus dans le produit par des procédés classiques de chromatographie ou de cristallisation fractionnée.

La présente invention concerne également le procédé de préparation des composés de formule (Ia) ou (Ib) dans laquelle X désigne, en particulier, le groupe mercaptométhyle.

Le procédé conforme à l'invention est caractérisé en ce qu'il consiste successivement :

a) à faire réagir un ester d'acide malonique tel que le malonate d'éthyle de formule

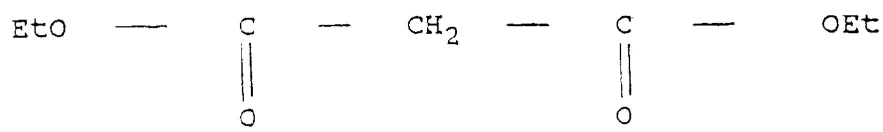

dans laquelle Et désigne le radical éthyle avec un composé halogéné de formule , $R_1$-$CH_2$-Y, $R_1$ ayant la signification précitée, en présence d'une solution alcoolique d'un métal alcalin, pour former un diester de formule (II)

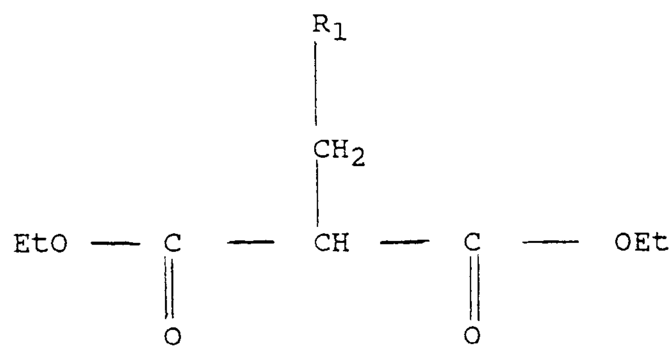

(II)

b) à monosaponifier le diester de formule (II) pour obtenir un monoacide de formule (III)

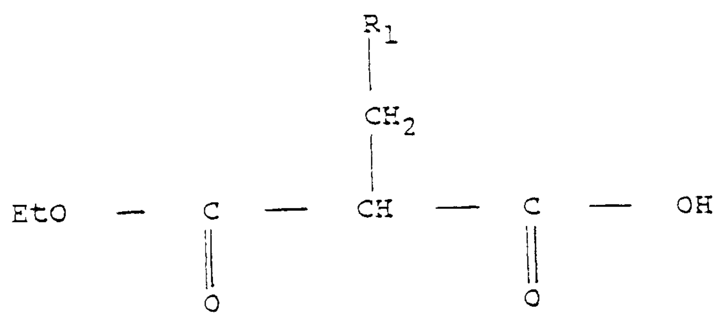

(III)

c) à préparer, par une réaction de Mannich, l'ester acrylique de formule (IV), réaction consistant à traiter le monoacide (III) avec une base organique telle que la diéthylamine, puis avec le formaldéhyde,

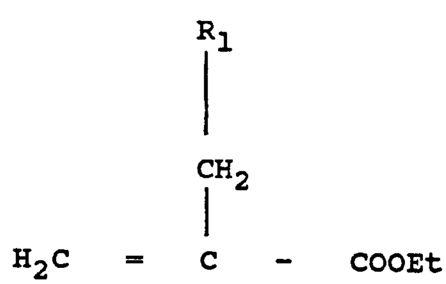

(IV)

d) à saponifier l'ester acrylique (IV) et à faire suivre la saponification d'une addition de Michaël avec l'acide thioacétique $CH_3CO$ SH pour former l'acide thioacétylé de formule (V)

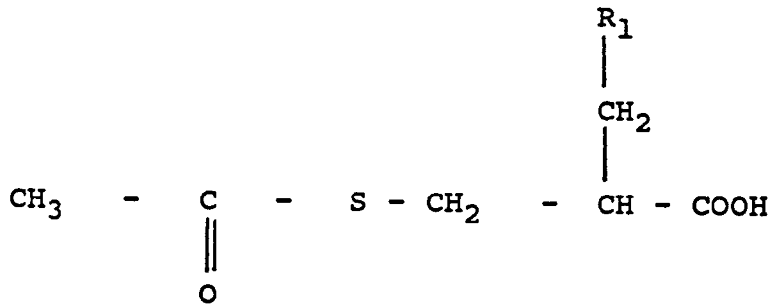 (V)

e) éventuellement à dédoubler l'acide thioacétylé (V),

f) à coupler l'acide thioacétylé de formule (V), sous forme racémique ou optiquement pure, avec l'amino-ester désiré tel qu'un amino-ester benzylique de formule (VI)

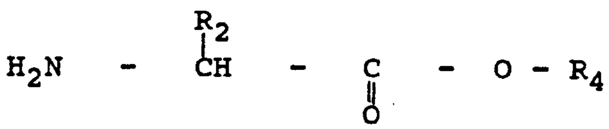 (VI)

où $R_2$ et $R_4$ ont les significations précitées pour former le composé de formule (VII)

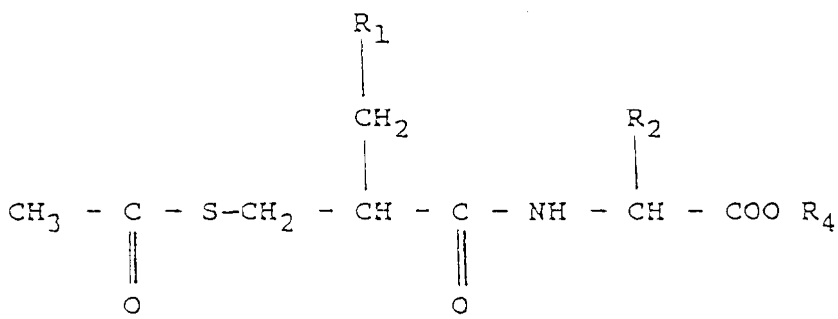 (VII)

en présence d'un agent de couplage tel que le dicyclohexylcarbodiimide,

g) puis à soumettre le composé (VII) à une déprotection alcaline pour former les inhibiteurs mixtes de formule (Ia).

Le dédoublement de l'acide thioacétyle (V) peut s'effectuer par un procédé tel que celui qui a été décrit dans le brevet français n° 2.623.498 précité, selon lequel on fait réagir l'acide avec l'éphédrine (+) ou selon le cas (-), on récupère le sel de l'énantiomorphe (+) ou (-) obtenu et on libère l'acide énantiomorphe. Le dédoublement de l'acide de formule (V) peut également s'effectuer avec une amine chirale telle que l'x-méthylbenzylamine.

La présente invention concerne également le procédé de préparation des composés éthyléniques de formule (Ib) dans laquelle X désigne, en particulier, le groupe mercaptométhyle.

Le procédé conforme à l'invention est caractérisé en ce qu'il consiste sucessivement :

a) à effectuer une bromation allylique d'un acide éthylénique (E) de formule (VIII)

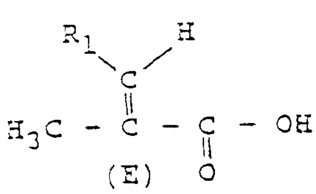 (VIII)

dans laquelle $R_1$ a la signification précitée, avec un agent de bromation tel que le N-bromo-succinimide, en présence d'une quantité catalytique de péroxyde de benzoyle, pour former un acide de formule (IX)

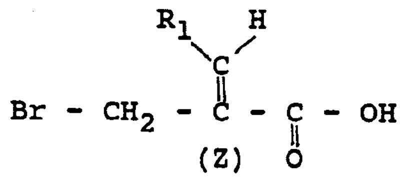

(IX)

b) à substituer le brome de l'acide éthylénique de formule (IX) par l'acide thioacétique pour former l'acide éthylénique thioacétyle (Z) de formule (X)

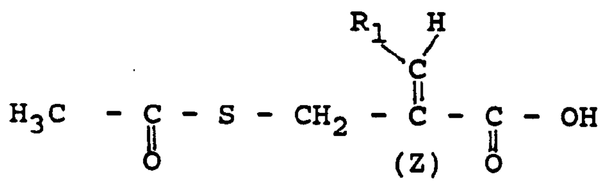

(X)

c) à isomériser l'acide de formule (X) à l'aide par exemple d'une lampe à ultra violet (U.V.) puis à séparer le mélange d'isomères (E/Z) obtenu par une amine telle que la cyclohexylamine pour obtenir l'acide éthylénique thioacétylé (E) de formule (XI)

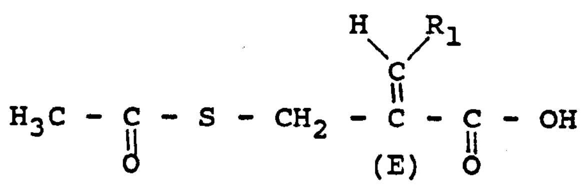

(XI)

d) à coupler l'acide éthylénique thioacétylé (E) de formule (XI), avec l'amino-ester désiré de formule (VI) en présence d'un agent de couplage tel que le dicyclohexylcarbodiimide pour obtenir le composé de formule (XII)

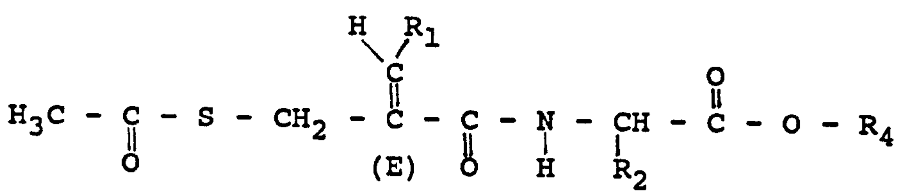

(XII)

e) puis à soumettre le composé de formule (XII) à une déprotection alcaline pour former les inhibiteurs mixtes de formule (Ib) (X = mercaptométhyl)

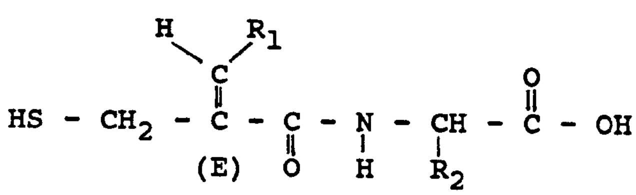

(Ib)

La présente invention concerne également le procédé de préparation des composés de formule (Ia) dans laquelle X désigne, en particulier, le groupe N-carboxyalkyle.

Le procédé conforme à l'invention est caractérisé en ce qu'il consiste successivement :

a) à effectuer une diazotation puis une hydrolyse d'un amino-acide de formule (XIII)

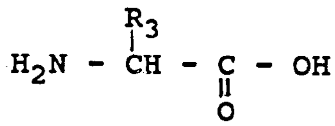 (XIII)

où $R_3$ a la définition précitée, pour former un hydroxyacide de formule (XIV)

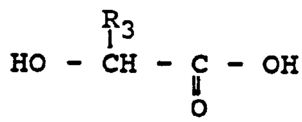 (XIV)

b) à effectuer une protection du groupe hydroxy du composé de formule (XIV) avec le chlorure d'acétyle pour former le composé de formule (XV)

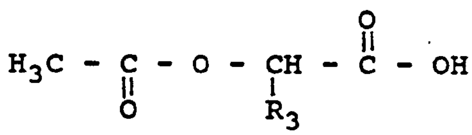 (XV)

c) à estérifier le composé de formule (XV), plus particulièrement par le tertiobutanol en présence d'oxychlorure de phosphore pour former l'ester de formule (XVI)

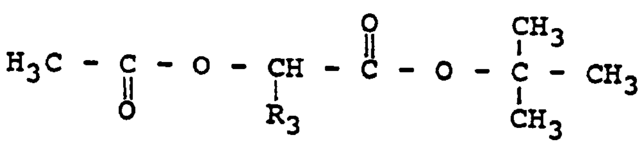 (XVI)

d) à libérer le groupe acétyle du composé (XVI) par une déprotection alcaline pour former l'hydroxyester de formule (XVII)

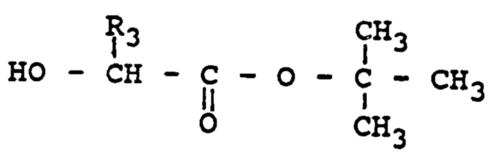 (XVII)

e) à activer la fonction alcool du composé de formule (XVII), par exemple par l'anhydride de triflurométhane sulfonique, en présence de pyridine, pour former le composé de formule (XVIII)

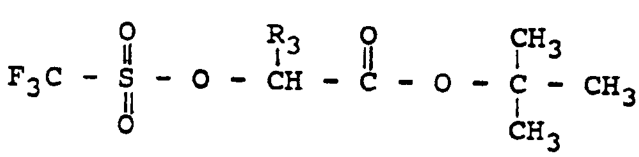 (XVIII)

f) à substituer le groupe trifluorométhanesulfonique du composé de formule (XVIII) par un aminoester de formule (XIX) :

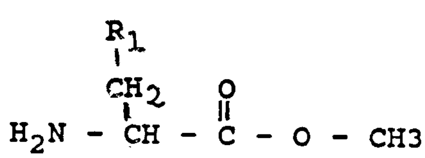 (XIX)

où $R_1$ a la définition précitée, en présence de bis (diméthylamino)-1,8 naphtalène, pour former le composé de formule (XX) :

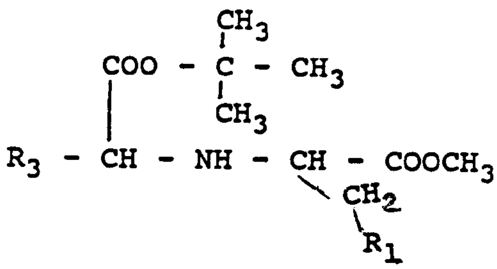

(XX)

g) à soumettre le composé de formule (XX) à une déprotection alcaline sélective pour former le composé de formule (XXI) :

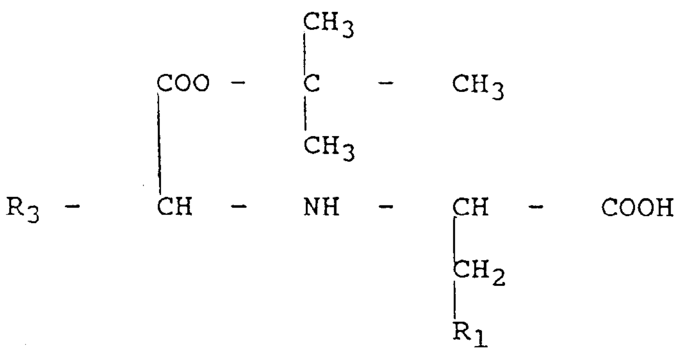

(XXI)

h) à coupler l'acide de formule (XXI) avec l'amino-ester désiré de formule (VI) où $R_4$ est un groupe benzyle, en présence d'un agent de couplage tel que le dicyclohexylcarbodiimide pour former le composé de formule (XXII) :

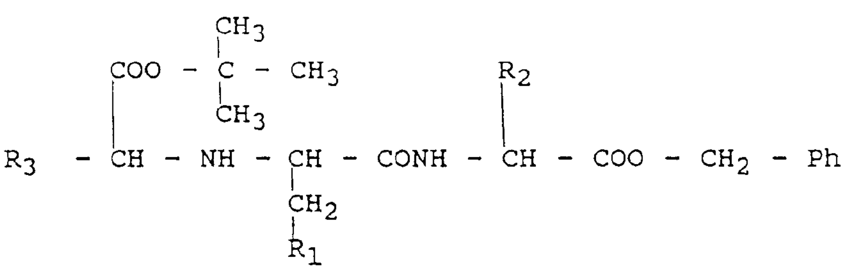

(XXII)

i) à hydrogéner le composé de formule (XXII) en présence d'un catalyseur d'hydrogénation tel que Pd/C à 10 % dans l'éthanol pour former le composé de formule (XXIII) :

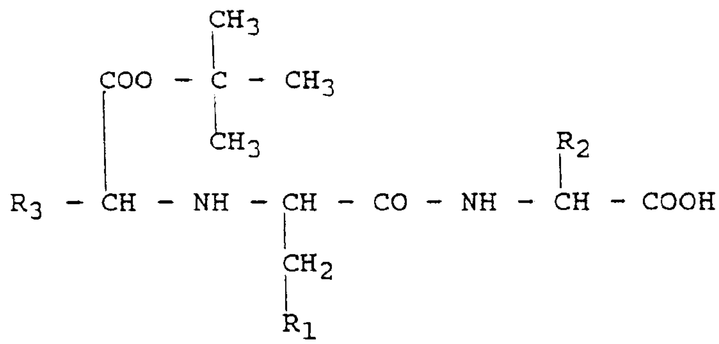

(XXIII)

j) puis, à hydrolyser la fonction ester tertiobutylique du composé de formule (XXIII), par exemple par une solution d'acide chlorhydrique dans l'acétate d'éthyle, pour former le diacide de formule (Ia) (X = N-carboxy-alkyle)

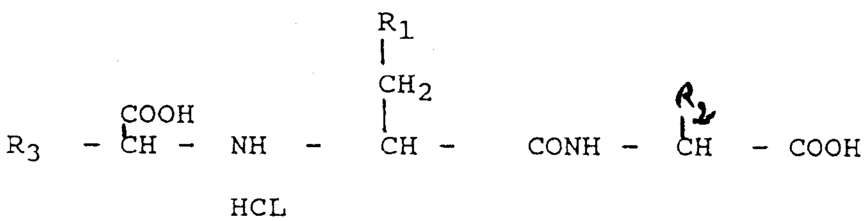

(Ia)

La présente invention concerne également le procédé de préparation des composés de formule (Ia) dans laquelle X désigne, en particulier, le groupe phosphonate.

Le procédé conforme à l'invention est caractérisé en ce qu'il consiste successivement :

a) à saponifier l'ester acrylique de formule (IV),

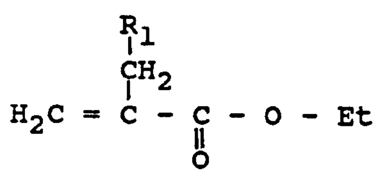

(IV)

où $R_1$ a la définition précitée, et à faire suivre la saponification par l'action du chlorure de thionyle pour former le chlorure d'acide acrylique de formule (XXIV)

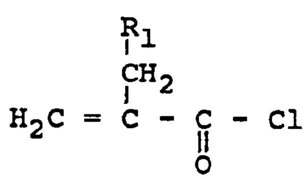

(XXIV)

b) à coupler le chlorure d'acide de formule (XXIV) avec l'amino-ester désiré de formule (VI), en présence par exemple de triéthylamine pour former le composé de formule (XXV)

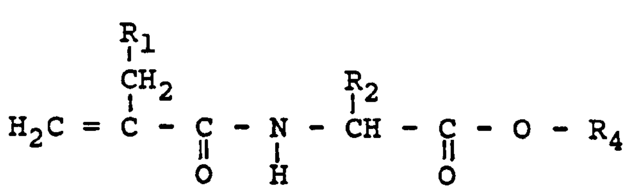

(XXV)

c) à effectuer une addition de Michaël avec un dialkylphosphite par exemple avec le diéthylphosphite en présence d'hydrure de sodium pour former le composé de formule (XXVI)

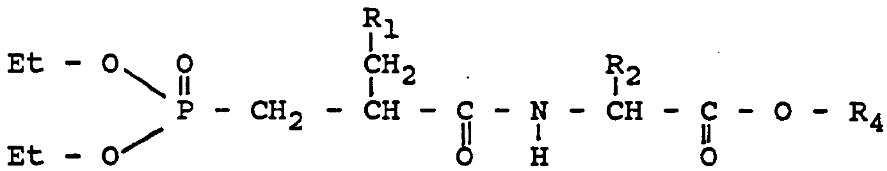

(XXVI)

d) puis à hydrolyser les fonctions protectrices du composé (XXVI) pour former les inhibiteurs de formule (Ia)

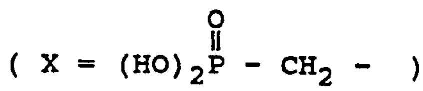

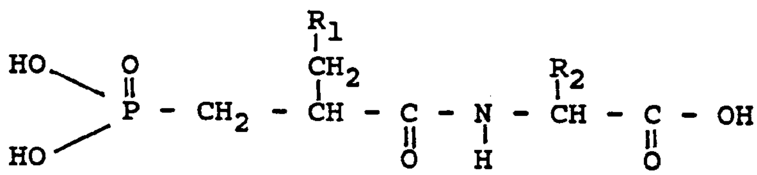

(Ia)

cette dernière étape pouvant avantageusement s'effectuer par le bromotriméthylsilane suivi d'un traitement par une solution aqueuse d'acide chlorhydrique 6 N.

Les composés conformes à l'invention se caractérisent par le fait qu'ils présentent des activités inhibitrices des enzymes enképhalinase et ACE, exprimées par les concentrations inhibitrices 50% ($IC_{50}$), inférieures à 10nM.

Selon une autre caractéristique importante de l'invention et qui s'applique plus particulièrement aux composés de formule (Ia) ou (Ib) dont les concentrations inhibitrices $IC_{50}$ sont comprises entre 1 et 10 nM, ces deux concentrations sont dans un rapport, de préférence, inférieur à 3-4, pour que le composé soit équipotent.

Il faut, toutefois, noter que dans le cas de composés très actifs, c'est-à-dire ceux présentant une concentration inhibitrice $IC_{50}$ de l'enképhalinase ou de l'ACE inférieure à 1nM, ces composés n'ont plus besoin d'être équipotents. Dans ce cas, en effet, et aux doses habituelles d'utilisation, une fraction seulement du dérivé d'amino-acide est utilisée pour inhiber l'une ou l'autre des deux activités enzymatiques et il subsiste toujours du composé libre en quantité suffisante pour inhiber l'autre activité.

La présente invention concerne donc également les compositions pharmaceutiques qui contiennent, à titre de principe actif, les composés de formule (Ia) ou (Ib) conformes à l'invention.

Ces compositions pharmaceutiques sont administrables à l'homme par voie orale, parentérale ou rectale.

Ces composés pharmaceutiques peuvent être sous forme solide ou liquide et se présenter sous les formes pharmaceutiques couramment utilisées en médecine humaine comme, par exemple, sous forme de comprimés simples ou dragéifiés, de gélules, de suppositoires, de préparations injectables.

Les compositions pharmaceutiques conformes à l'invention, sont administrables à des doses unitaires, de préférence, de 1 à 200 mg de principe actif et à une posologie quotidienne pouvant varier de 2 à 400 mg de principe actif.

Il va être donné, ci-dessous, à titre non limitatif, plusieurs exemples de mise en oeuvre de l'invention.

Exemple 1 : Préparation de la N-(RS)-[oxo-1 (mercaptométhyl)-2 (méthylènedioxy-3,4 phényl)-3 propyl]glycine

Etape a : Synthèse malonique

Préparation du (méthylènedioxy-3,4 phényl)-3 éthoxycarbonyl-2 propanoate d'éthyle

Dans un tricol d'un litre muni d'un réfrigérant, d'une ampoule à brome et d'une garde à chlorure de calcium,

on place 34,9 g (204,69 mmol) de chlorure de pipéronyle et 137,5 g (130,3 ml) (859,4 mmol) de diéthyle malonate. On agite et additionne ensuite une solution de 12,2 g (530,4 mmol) de sodium dans 312 ml d'éthanol anhydre (solution 1,7 M). On porte ce mélange à reflux (température du bain d'huile à 80°C) pendant 5 heures

On évapore l'éthanol à l'évaporateur rotatif, puis on reprend le résidu à l'eau (150 ml) et à l'éther éthylique (100 ml). On sépare la phase éthérée et on extrait encore la phase aqueuse à l'éther éthylique (2 fois 100 ml). Les phases éthérées réunies sont lavées à l'eau (1 fois 100 ml), séchées sur $MgSO_4$, filtrées et concentrées. On obtient un résidu huileux que l'on distille à la pompe à palettes afin d'éliminer l'excès de diéthyle malonate (60-70°C sous 0,2 mm Hg). Le résidu de distillation contient le pipéronyle diéthyle malonate (II).

Masse = 54,7 g

Rendement = 91%

RMN $^1H$ (CDCl3) : 6,9 à 6,55(m,3H) ; 5,95(s,2H) ; 4,1(q,4H,J = 6,8 Hz) ; 3,55(t,1H,J = 8 Hz) ; 3,1(d,2H,J = 8 Hz) ; 1,2(t,6H,J = 6,8 Hz).

IR : 1710 $cm^{-1}$

Etape b : Préparation de l'acide (méthylènedioxy-3,4 phényl)-3 éthoxycarbonyl-2 propanoïque

Dans un ballon muni d'une ampoule à brome et d'une garde à chlorure de calcium, on place une solution de 54,7 g (186,05 mmol) du produit obtenu à l'étape précédente dans 24 ml d'éthanol absolu. On refroidit à environ 0°C dans un bain de glace et on ajoute, sous agitation, en 30 minutes une solution de 10,7 g (190,69 mmol) de potasse dans 186 ml d'éthanol absolu. On agite ensuite entre 0°C et 10°C pendant 24 heures.

On évapore à sec (évaporateur rotatif) et on reprend le résidu à l'eau (150 ml). On lave à l'éther éthylique (3 fois 50 ml). La phase aqueuse est refroidie et acidifiée par une solution aqueuse d'acide chlorhydrique 3N jusqu'à pH 2. On extrait à l'éther éthylique (4 fois 50 ml). Les phases éthérées sont réunies, lavées à l'eau (1 fois 50 ml), avec une solution saturée de NaCl (1 fois 50 ml), sèchées sur $MgSO_4$, filtrées et concentrées. On obtient une huile :

Masse = 43,75 g

Rendement = 87%

RMN $^1H$ (CDCl3) : 10,1(s,1H) ; 6,9 à 6,3(m,3H) ; 5,85(s,2H) ; 4,1(q,2H,J = 7,5 Hz) ; 3,7(t,1H,J = 7,9 Hz) ; 3,1(d,2H,J = 7,9 Hz) ; 1,15(t,3H,J = 7,5 Hz).

IR : 1705 $cm^{-1}$

Etape c : Préparation du ((méthylènedioxy-3,4 phényl)-méthyl)-3 propénoate d'éthyle

Dans un ballon refroidi à 0-5°C par un bain de glace, on place 41,71 g (156,8 mmol) du monoester obtenu à l'etape précédente.

On ajoute goutte à goutte, sous agitation, à 0-5°C, 16,25 ml (157 mmol) de diéthylamine, puis 15,8 ml (210,8 mmol) de formol à 37% dans l'eau. On laisse revenir à température ambiante et on agite 24 heures.

Le mélange réactionnel est repris à l'eau (50 ml) puis extrait à l'éther (1 fois 200 ml). I,a phase organique est refroidie dans un bain de glace et est acifidiée sous agitation par une solution aqueuse d'acide chlorhydrique 1N, jusqu'à pH 2. La phase éthérée est alors séparée, lavée à l'eau (2 fois 50 ml), lavée par une solution aqueuse saturée de NaCl (1 fois 50 ml), séchée sur $MgSO_4$, filtrée et concentrée. On obtient une huile :

Masse = 31,6 g

Rendement = 84%

RMN $^1H$ (CDCl3) : 6,9 à 6,5(m,3H) ; 6,15(s,1H) ; 5,8(s,2H) ; 5,4(s,1H) ; 4,1(q,2H,J = 6,8 Hz) ; 3,5(s,2H) ; 1,2(t,3H,J = 6,8 Hz).

IR : 1700 ; 1620 $cm^{-1}$

Etape d : - Préparation de l'acide-(RS)-acétylthiométhyl-2 (méthylènedioxy-3,4 phényl)-3 propanoïque

Dans un ballon muni d'une ampoule à brome, on place 31,7 g (135 mmol) de l'ester acrylique obtenu à l'étape précédente en solution dans 190 ml d'un mélange acétone/eau, 75/25. On refroidit à environ 5°C par un bain de glace et on ajoute en 10 minutes environ, sous agitation, 270 ml (270 mmol) d'une solution aqueuse de NaOH 1N. On laisse remonter la température à l'ambiante et on agite 20 heures.

On élimine l'acétone à l'évaporateur rotatif et on lave la phase aqueuse à l'éther éthylique (3 fois 60 ml). La phase aqueuse est ensuite refroidie dans un bain de glace et acidifiée par une solution aqueuse d'HCl 1N jusqu'à pH 2. La phase aqueuse acide est alors extraite à l'éther éthylique (4 fois 60 ml). Les phases éthérées sont réunies, lavées à l'eau (1 fois 60 ml), lavées par une solution aqueuse saturée de NaCl (1 fois 60 ml), séchées sur $MgSO_4$, filtrées et concentrées. On obtient l'acide [(méthylènedioxy-3,4 phényl)-méthyl]-2 propénoïque sous forme d'un solide blanc :
Masse = 26,9 g
Rendement = 96%
F = 121°C RMN $^1H$ ($CDCl_3$) : 9,9(s,1H) ; 6,75(m,3H) ; 6,4(s,1H) ; 5,95(s,2H) ; 5,6(s,1H) ; 3,5(s,2H).
IR(nujol) : 1685 ; 1620 $cm^{-1}$

- Préparation de l'acide -(RS)-acétylthiométhyl-2 (méthylènedioxy-3,4 phényl)-3 propanoïque

Dans un ballon muni d'un réfrigérant et d'une garde à chlorure de calcium, on place 26,9 g (130,5 mmol) de l'acide obtenu à l'étape précédente et 15,9 g (209,2 mmol) d'acide thioacétique. Le mélange est chauffé pendant 24 heures à 70°C sous agitation.

On évapore sous vide (pompe à palettes 1 mm Hg, 60°C) l'excés d'acide thioacétique. Le résidu pâteux jaune est repris à trois reprises par 100 ml d'éther éthylique. A chaque fois, l'éther éthylique est éliminé à l'évaporateur rotatif, puis le résidu séché sous vide. On obtient une huile jaune visqueuse :
Masse = 36,7 g
Rendement = 100%
RMN $^1H$ ($CDCl_3$) ; 9,9(s,1H) ; 6,85 à 6,5(m,3H) ; 5,85(s,2H) ; 3,25 à 2,6(m,5H) ; 2,3(s,3H).
IR : 1700 $cm^{-1}$

Etape e : On procédé éventuellement, à ce stade, à un dédoublement de l'acide obtenu à l'étape précédente, selon l'exemple 2.

Etape f : Préparation du N-(RS)-[oxo-1(acétylthiométhyl)-2 (méthylènedioxy-3,4 phényl)-3 propyl]-glycinate de benzyle

Dans un ballon muni d'une garde à chlorure de calcium, on place 1,37 g (4,85 mmol) d'acide (RS) (acétylthiométhyl)-2 (méthylènedioxy-3,4 phényl)-3 propanoïque en solution dans 8 ml de THF anhydre. On refroidit le ballon à environ 0-5°C par un bain de glace et on ajoute successivement sous agitation 1,63 g (4,85 mmol) de sel de paratoluènesulfonate de glycinate de benzyle et 0,49 g (4,85 mmol) de triéthylamine dans 10 ml de chloroforme, une solution de 0,74 g (4,85 mmol) d'hydroxybenzotriazole monohydraté dans 8 ml de THF et une solution de 1,0 g (4,85 mmol) de dicyclohexylcarbodiimide dans 7 ml de chloroforme. On laisse le mélange revenir à la température ambiante et on agite pendant 6 heures.

Le précipité de dicyclohexylurée (DCU) est filtré et on évapore à sec. Le résidu pâteux est repris par l'acétate d'éthyle (12 ml). La DCU qui a à nouveau précipité est filtrée. La phase organique est successivement lavée à l'eau (1 fois 10 ml), par une solution aqueuse saturée d'hydrogénocarbonate de sodium (3 fois 10 ml), à l'eau (1 fois 10 ml), et par une solution aqueuse saturée de NaCl (1 fois 10 ml). On sèche sur $MgSO_4$, on filtre et on concentre.

On obtient un résidu solide blanc que l'on dissout dans le minimum de chloroforme. On ajoute, sous agitation, de l'éther de pétrole (25 ml). On laisse 15 heures au repos. On filtre, on lave à l'éther de pétrole, on essore et on sèche le solide sous vide :
Masse = 1,83 g
Rendement = 88% (recristallisé dans un mélange chloroforme/éther de pétrole)
F = 74°C
IR (nujol) : 3310, 1730, 1695, 1640 $cm^{-1}$
RMN $^1H$ ($CDCl_3$/TMS) : 7,5 à 7,3(m,5H) ; 6,7(s,3H) ; 6,6(s,large,1H) ; 5,9(s,2H) ; 5,25(s,2H) ; 4,0(d,2H,J = 5,3Hz) ; 3,2 à 2,4(m,5H) ; 2,3(s,3H)
RMN $^{13}C$ ($CDCl_3$) : 195,4(s) ; 172,9(s) ; 169,1(s) ; 147,3(s) ; 145,8(S) ; 134,9(s) ; 131,9(s) ; 128,2(d) ; 127,9(d) ; 121,6(d) ; 108,9(d) ; 107,9(d) ; 100,5(t) ; 66,6(t) ; 48,7(d) ; 41,0(t) ; 37,7(t) ; 30,6(t) ; 30,2(q) ;

Microanalyse : $C_{22}H_{23}O_6NS$

Calc % C = 61,54 N = 3,26 H = 5,36
Tr % C = 61,45 N = 3,36 H = 5,41

Etape g : Préparation de la N-(RS)-[oxo-1 (mercaptométhyl)-2 (méthylènedioxy-3,4 phényl)-3 propyl]-glycine

On place dans un ballon 0,43 g (1,0 mmol) du composé obtenu à l'étape précédente en solution dans 3 ml de méthanol. On purge avec de l'argon et on refroidit la solution par un bain de glace. On ajoute, à environ 5°C, 2,1 ml d'une solution aqueuse de soude 1N. On agite 2 heures à 20°C.

On évapore le méthanol sous vide à une température inférieure à 35°C. La phase aqueuse basique est lavée à l'éther (2 fois 10 ml). Elle est ensuite acidifiée par une solution aqueuse d'HCl 1N jusqu'à pH 1. On l'extrait à l'éther (2 fois 10 ml). Les phases d'extraction sont lavées une fois à l'eau, séchées sur $MgSO_4$, filtrées et concentrées sous vide. Le résidu est séché au dessicateur sur pentaoxyde de phosphore pour éliminer l'acide acétique. On purifie si nécessaire par chromatographie sur silice. On obtient la N-(RS)-[oxo-1 (mercaptométhyl)-2 (méthylènedioxy-3,4 phényl)-3 propyl]-glycine.
Masse = 0,29 g
Rendement = 72% (chromatographié)
F = 94°C (microscope)
IR (nujol) : 3390, 1740, 1620 $cm^{-1}$
RMN $^1H$ ($CDCl_3$/TMS) : 10,4(s,1H) ; 6,8 à 6,4(m,4H) ; 5,8(s,2H) ; 3,95(d,2H,J = 5,3Hz) ; 3,1 à 2,2(m,5H) ; 1,6(t,1H, J = 7,9Hz)
RMN$^{13}$ C ($CDCl_3$) : 174,4(s) ;173,1(s) ; 147,5(s) ; 146,1 (s) ; 131,9(s) ; 121,8(d) ; 109,0 (d) . 108,2(d) ; 100,7(t) ; 53,0(d) ; 41,2(t) ; 37,6(t) ; 25,8(t);

Microanalyse : $C_{13}H_{15}O_5NS$

| | | | | |
|---|---|---|---|---|
| Calc. | % | C = 52,52 | N = 4,71 | H = 5,05 |
| Tr. | % | C = 52,44 | N = 4,62 | H = 5,00 |

Exemple 2 : Préparation de l'acide (S)-(acétylthiométhyl)-2 (méthylènedioxy-3,4 phényl)- 3 propanoïque

I. Dédoublement par l' méthylbenzylamine

On place dans un ballon 32,2 g (114,2 mmol) d'acide (acétylthiométhyl)-2 (méthylènedioxy-3,4 phényl)-3 propanoïque racémique en solution dans 200 ml d'éther éthylique. On ajoute goutte à goutte, sous agitation, 13,85 g (114,2 mmol) de (R)-α- méthylbenzylamine. Il y a alors précipitation. On laisse le mélange au repos pendant 17 heures.

On filtre, on lave le sel à l'éther (50 ml) et on le sèche sous vide. On obtient ainsi le sel de l'acide :
Masse = 37,45 g
Rendement = 81%
F = 118°C
$[\alpha]D^{25}$ = + 2,2° ( c = 1,1 dans le méthanol)

Recristallisations

On place dans un ballon muni d'un réfrigérant 37,45 g du sel précédent et 100 ml de dichlorométhane. On agite et on chauffe jusqu'à dissolution complète du sel. On ajoute alors 100 ml d'éther de pétrole (40-60°C). Après retour à la température ambiante, on laisse au repos pendant 24 heures.

On filtre, on lave le sel à l'éther de pétrole (50 ml), on l'essore et on le sèche sous vide :
Masse = 18,45 g
Rendement = 50%
$[\alpha]D^{25}$ = -7,89° (c = 1,3 dans le méthanol)
On répète cette opération 4 fois.
Le rendement global de ces 5 recristallisations est de 20%. Le point de fusion du sel optiquement pur est de 122°C.
$[\alpha]D^{25}$ = -23,0° (c = 1,2 ; MeOH).

Libération de l'acide (S) optiquement pur

On place dans un ballon 7,4 g (18,36 mmol) de sel optiquement pur. On ajoute de l'eau (50 ml), du dichlorométhane (50 ml) et une solution aqueuse d'HCl 1N jusqu'à pH 1. On agite jusqu'à dissolution complète du sel. On sépare la phase organique et on extrait la phase aqueuse acide par le dichlorométhane (2 fois 25 ml). Les phases organiques sont réunies, lavées à l'eau (2 fois 25 ml), séchées sur $MgSO_4$, filtrées et concentrées. On obtient un résidu huileux qui cristallise :
Masse = 4,97 g
Rendement = 96%
F = 60°C
$[\alpha]D^{25}$ = -23,0° (c = 1,3 dans le méthanol)
IR(nujol) : 1685 $cm^{-1}$
RMN $^1H$ ($CDCl_3$/TMS) : 10,4(s, 1H) ; 6,75(s,3H) ; 5,95(s,2H) ; 3,3 à 2,7(m,5H) ; 2,3(s,3H).

II. Dédoublement par l'éphédrine

On place dans un ballon 35,46 mmoles d'acide racémique en solution dans 50 ml d'éther. On ajoute en agitant 17,73 mmoles de (+) éphédrine en solution dans 60 ml d'éther. On laisse cristalliser sans agiter à température ambiante. On filtre, on lave le sel avec de l'éther et on le sèche sous vide.

Le sel de l'acide est obtenu avec un rendement de 84%, F = 102-116°C, [ ]$D^{25}$ = +7,7° (c = 1,2 ; MeOH).

Recristallisations

On place dans un ballon 10 g de sel précédent. On le dissout dans 50 ml de chloroforme puis on ajoute 100 ml d'éther éthylique. On laisse au repos pendant 24 heures.

On filtre, on lave le sel à l'éther de pétrole, on essore et on sèche sous vide.
Masse = 7,8 g
Rendement = 78%
$[\alpha]D^{25}$ = +4,3° (c = 1,3 ; MeOH)
On répète cette opération 9 fois.
Rendement global des recristallisations = 40%, F = 122°C $[\alpha]D^{25}$ = -5,3° (c = 1,2 ; MeOH)

Libération de l'acide (S) optiquement pur

On opère comme dans le cas du dédoublement par l'α-méthylbenzylamine.
Rendement = 95%
$[\alpha]D^{25}$ = -25,7° (c = 1,3 ; MeOH).

Exemple 3 : Préparation du N-(S)-[oxo-1 (acétylthiométhyl)-2 (méthylènedioxy-3,4 phényl)-3 propyl]-glycinate de benzyle

On couple l'acide (S) (acétylthiométhyl)-2 (méthylènedioxy-3,4 phényl)-3 propanoïque avec le glycinate de benzyle en utilisant le mode opératoire décrit à l'exemple 1 (étape f).
Rendement = 76% (chromatographié)
F = 92°C (Microscope)
$[\alpha]_D$ = -15,8° (c = 1,2 dans le méthanol)
IR (nujol) : 3280, 1725, 1690, 1640 $cm^{-1}$
RMN $^1H$ ($CDCl_3$/TMS) : 7,3(s,5H) ; 6,65(s,3H) ; 6,1(s large, 1H) ; 5,85(s,2H) ; 5,15(s,2H) ; 3,95(d,2H,J = 5, 3Hz) ; 3,15 à 2,5(m,5H) ; 2,3(s,3H).

Le spectre RMN[13] est identique à celui du produit racémique (exemple 1).

**Microanalyse** : $C_{22}H_{23}O_6NS$

| | | | |
|---|---|---|---|
| Calc. | % C = 61,54 | N = 3,26 | H = 5,36 |
| Tr. | % C = 61,38 | N = 3,19 | H = 5,30 |

Exemple 4 : Préparation de la N-(S)-[oxo-1 (mercaptométhyl)-2 (méthylènedioxy-3,4 phényl)-3 propyl]-glycine (+)

On déprotège le produit de l'exemple 3 selon le mode opératoire décrit dans l'exemple 1 (étape g).
Rendement = 60% (chromatographié)
$[\alpha]_D$ = +54,4° (c = 1,0 dans le méthanol)
IR ($CHCl_3$) : 1730, 1670 $cm^{-1}$
RMN $^1H$ ($CDCl_3$/TMS) : 9,6(s,1H) ; 6,8 à 6,45(m,4H) ; 5,95(s,2H) ; 4,05(d,2H,J = 4Hz) ; 3,3 à 2,3(m,5Hz) ; 1,65(t,1H,J = 7,3Hz)

Microanalyse : $C_{13}H_{15}O_5NS$

| | | | | |
|---|---|---|---|---|
| Calc. | % | C = 52,52 | N = 4,71 | H = 5,05 |
| Tr. | % | C = 52,35 | N = 4,90 | H = 5,17 |

Exemple 5 : Préparation du N-(RS)-[oxo-1 (acétylthiométhyl)-2 (méthylènedioxy-3,4 phényl)-3 propyl] (S)-alaninate de benzyle

On couple l'acide (acétylthiométhyl)1-2 (méthylènedioxy-3,4 phényl)-3 propanoïque sous sa forme racémique (exemple 1, étape d) avec l'alaninate de benzyle de configuration (S) selon le mode opératoire de l'exemple 1 (étape f).
Rendement = 82% (chromatographié) (mélange 50/50 de deux diastéréoisomères) ;
F = 68°C (Microscope)
IR (nujol) : 3290, 1730, 1690, 1640 $cm^{-1}$
RMN $^1H$ ($CDCl_3$/TMS) : 7,25(s,5H) ; 6,6(s,3H) ; 6,4(m,IH) ; 5,8(s,2H) ; 5,1(s,2H) ; 4,6(quintuplet, IH,J = 7,3Hz) ; 3,2 à 2,35(m,5H) ; 2,2(s,3H) ; 1,3 et 1,15(2 doublets, 3H,J = 7,3Hz)
RMN$^{13}C$ ($CDCl_3$) : 195,3(s) ; 195,0(s) ; 171,9(s) ; 147,1(s) ; 145,7(S) ; 135,0(s) ; 131,9(s) ; 131,7(s) ; 128,0(d) ; 127,6(d) ; 121,5(d) ; 108,9(d) ; 107,7(d) ; 100,3(t) ; 66,4(t) ; 48,8(d) ; 48,3(d) ; 47,6(d) ; 37,7(t) ; 31,6(t) ; 30,5(q) ; 17,8(q)

Microanalyse : $C_{23}H_{25}O_6NS$

| | | | | |
|---|---|---|---|---|
| Calc. | % | C = 62,30 | N = 3,16 | H = 5,64 |
| Tr | % | C = 62,21 | N = 3,11 | H = 5,55 |

Exemple 6 : Préparation de la N-(RS)-[oxo-1 (mercaptométhyl)-2 (méthylènedioxy-3,4 phényl)-3 propyl]-(S)-alanine

On effectue la déprotection selon le mode opératoire de l'exemple 1 (étape g).
Rendement = 72% (mélange 50/50 de deux diastéréoisomères)
F <50°C
IR (nujol) : 3280, 1725, 1640 $cm^{-1}$
RMN $^1H$ ($CDCl_3$/TMS) / 8,8(s,1H) ; 6,8 à 6,2(m,4H) ; 5,8(s,2H) ; 4,5 (quintuplet, 1H,J = 7Hz) ; 3,25 à 2,15(m,5H) ; 1,65(t,1H,J = 6,5Hz) ; 1,4 et 1,25(2 doublets, 3H,J = 7Hz)
RMN$^{13}C$ ($CDCl_3$) : 175,6(s) : 173,5(s) ; 147,5(s) ; 146,0(s) ; 132,0(s) ; 121,7(d) ; 109,0(d) ; 108,1(d) ; 100,7(t) ; 53,4 (d) ; 52,9(d) ; 48,0(d) ; 37,9(t) ; 37,7(t) ; 25,9(t) ; 25,7(t) ; 18,0(q) ; 17,6(q)

Microanalyse : $C_{14}H_{17}O_5NS$

| | | | | |
|---|---|---|---|---|
| Calc. | % | C = 54,02 | N = 4,50 | H = 5,46 |
| Tr. | % | C = 53,73 | N = 4,39 | H = 5,40 |

Exemple 7 : Préparation du N-(S)-[oxo-1 (acétylthiométhyl)-2(méthylènedioxy-3,4 phényl)-3 propyl] (S)-alaninate de benzyle

A. Préparation par couplage de l'acide (S)-(acétylthiométhyl)-2 (méthylènedioxy-3,4 phényl)-3 propanoïque optiquement pur (exemple 2).
Rendement = 77% (chromatographié)
F = 104°C ; un seul diastéréoisomère (Microscope)
$[\ ]_D$ = -50,6° (c = 1,35 dans le méthanol)
IR (nujol) : 3280, 1740, 1695, 1640 $cm^{-1}$
RMN $^1H$ ($CDCl_3$/TMS) : 7,25(s,5H) ; 6,6(s,3H) ; 6,0(d,1H,J = 7,5 Hz) ; 5,85(s,2H) ; 5,0(s,2H) ; 4,5(quintuplet,1H,J = 7,5Hz) ; 3,05(d,2H,J = 6,1Hz) ; 3,0 à 2,4(m,3H) ; 2,25(s,3H) ; 1,3(d,3H,J = 7,5Hz).

Microanalyse : $C_{23}H_{25}O_6NS$

| | | | |
|---|---|---|---|
| Calc. % | C = 62,30 | N = 3,16 | H = 5,64 |
| Tr. % | C = 62,20 | N = 3,20 | H = 5,30 |

B. Préparation par séparation des diastéréoisomères de l'exemple 5

On place dans un ballon 5,7 g (12,86 mmol) du composé obtenu dans l'exemple 5 dans 20 ml de chloroforme. On ajoute ensuite à cette solution, sous agitation, 80 ml d'éther éthylique et 80 ml d'éther de pétrole. On laisse 24 heures au repos.

On filtre, on essore et on sèche le solide sous vide :
Masse = 1,7 g
Rendement = 30%
Produit contenant 80% de l'isomère (S,S)

On répète l'opération précédente. On dissout 1,7 g (3,83 mmol) de sel (enrichi à 80% en diastéréoisomère (S,S)) dans le minimum de chloroforme (7 ml). On ajoute ensuite, sous agitation, 25 ml d'éther éthylique et 25 ml d'éther de pétrole. On laisse 24 heures au repos.

On filtre, on essore et on sèche le solide blanc sous vide. On obtient :
Masse = 1,2 g
Rendement de recristallisation = 70%
Produit enrichi à plus de 95% en isomère (S,S)
Les caractéristiques physiques et spectrales sont identiques à celles obtenues pour le composé de l'exemple 7.A.

Le rendement global de ces deux recristallisations est de 21%.

Exemple 8 : Préparation du N-(S)-[oxo-1 (mercaptométhyl)-2 (méthylènedioxy-3,4 phényl)-3 propyl]-(S)-alanine

On effectue la déprotection selon le mode opératoire de l'exemple 1 (étape g).
Rendement = 81%
$[\alpha]_d^{20}$ = +12,9° (c = 1,35 ; MeOH)
RMN $^1H$ ($CDCl_3$/TMS) : 9,05(s,1H) ; 6,8 à 6,6(m,3H) ; 6,45(d,1H,J = 7Hz) ; 5,85(s,2H) ; 4,55(quintuplet, 1H,J = 7Hz) ; 3,1 à 2,25(m,3H) ; 1,5(t,1H,J = 8,5Hz) ; 1,4(d,3H,J = 7Hz)

Microanalyse : ($C_{14}H_7NO_5S$)

| | | | |
|---|---|---|---|
| Calc.% | C = 54,08 | H = 5,50 | N = 4,50 |
| Tr. % | C = 53,65 | H = 5,78 | N = 4,38 |

Exemple 9 : Préparation du N-(RS)-[oxo-1 (acétylthiométhyl)-2 (méthylènedioxy-3,4 phényl)-3 propyl]-(S)-amino-2 butyrate de benzyle

On couple l'acide (acétylthiométhyl)-2 (méthylènedioxy-3,4 phényl)-3 propanoïque sous sa forme racémique (exemple 1, étape d) avec l'amino-2 butyrate de benzyle de configuration (S) selon le mode opératoire

de l'exemple 1 (étape f).
Rendement = 87%
F = 61°C (Microscope) (Mélange 50/50 le diastéréoisomères)
RMN $^1H$ ($CDCl_3$, TMS) : 7,3(s,5H) ; 6,65(s,3H) ; 6,0(m,1H) ; 5,8(s,2H) ; 5,15(s,2H) ; 4,55(m,1H) ; 3,2-2,4(m,5H) ; 2,3(s,3H) ; 1,65(m,2H) ; 0,8(t,J = 7,5 Hz,3/2 H) ; 0,6(t,J = 7,5Hz,3/2H).
RMN$^{13}C$ ($CDCl_3$) : 195,5 ; 172,3 ; 171,6 ; 147,6 ; 146,1 ; 135,3 ; 132,2 ; 128,3 ; 128,0 ; 121,7 ; 109,1 108,1 ; 100,7 ; 66,7 ; 53,1 ; 52,9 ; 49,6 ; 49,35 ; 37,9 ; 31,1 ; 30,9 ; 30,3 ; 25,4 ; 25,2
IR (nujol) : 3300, 1730, 1690, 1640 $cm^{-1}$

Microanalyse : ($C_{24}H_{27}NO_6S$)

| | | | |
|---|---|---|---|
| Calc.% | C = 63,00 | H = 5,95 | N = 3,06 |
| Tr. % | C = 62,89 | H = 6,22 | N = 3,33 |

Exemple 10 : Préparation de l'acide N-(RS)-[oxo-1 (mercaptométhyl)-2 (méthylènedioxy-3,4 phényl)-3 propyl]-(S)-amino-2 butyrique

On effectue la déprotection du composé obtenu dans l'exemple 9 selon le mode opératoire de l'exemple 1 (étape g).
Rendement = 69%
F = 118°C (microscope) (mélange 50/50 de diastéréoisomères)
RMN $^1H$ $CDCl_3$/TMS) : 9,1(s,1H) . 6,65(s,3H) ; 6,3(m, 1H) ; 5,85(s,2H) ; 4,55(m,1H) ; 3,0-2,2(m,5H) ; 1,6(m,3H) ; 0,9(t,J = 7,5 Hz, 3/2H) ; 0,7(t,J = 7,5 Hz,3/2H).
RMN$^{13}C$ ($CDCl_3$) : 175,6 ; 173,5 ; 147,8 ; 146,4 ; 132,3 ; 121,95 ; 109,2 ; 108,4 ; 100,8 ; 53,8 ; 53,2 ; 37,9 ; 26,2 ; 25,8 ; 25,3 ; 24,8.
IR (nujol) : 350, 1730, 1630 $cm^{-1}$

Microanalyse : ($C_{15}H_{19}NO_5S$)

| | | | |
|---|---|---|---|
| Calc. % | C = 55,37 | H = 5,89 | N = 4,30 |
| Tr. % | C = 55,32 | H = 5,64 | H = 4,22 |

Exemple 11 : Préparation du N-(RS)-[oxo-1 (acétylthiométhyl)-2 (méthylènedioxy-3,4 phényl)-3 propyl](S)-norvalinate de benzyle

On couple l'acide acétylthiométhyl-2 (méthylènedioxy-3,4 phényl)-3 propanoïque sous sa forme racémique (exemple 1, étape d) avec le norvalinate de benzyle de configuration (S) selon le mode opératoire de l'exemple 1 (étape f).
Rendement = 88%
F = 92°C (Microscope) (mélange 50/50 de diastéréoisomères)
RMN$^1H$ ($CDCl_3$/TMS) : 7,3(s,5H) ; 6,6(s,3H) ; 6,0(m,1H) ; 5,85(s,2H) ; 5,1(s,2H) ; 4,55(m,1H) ; 3,2-2,3(m,5H) ; 2,1(s,3H) ; 1,6-0,9(m,4H) ; 0,6(m,3H.
RMN$^{13}C$ ($CDCl_3$) : 195,7 ; 195,5 ; 172,3 ; 171,8 ; 147,6 ; 146,1 ; 135,3 ; 132,4 ; 132,1 ; 128,3 ; 128,0 ; 121,8 ; 109,1 ; 108 ; 100,7 ; 66,7 ; 51,7 ; 49,6 ; 49,2 ; 37,9 ; 34,3 ; 31,1 ; 30,9 ; 30,3 ; 18,2 ; 17,8 ; 13,3.
IR (nujol) : 3300, 1730, 1690, 1635 $cm^{-1}$

Microanalyse : ($C_{25}H_{29}NO_6S$)

| | | | |
|---|---|---|---|
| Calc. % | C = 63,68 | H = 6,20 | N = 2,97 |
| Tr. % | c = 63,47 | H = 6,13 | N = 3,19 |

Exemple 12 : Préparation de la N-(RS)-[oxo-1 (mercaptométhyl)-2 (méthylènedioxy-3,4 phényl)-3 propyl]-(S)-norvaline (mélange 50/50 de diastéréoisoméres)

On effectue la déprotection du composé de l'exemple 11 selon le mode opératoire de l'exemple 1 (étape g).
Rendement = 75%
Rf = 0,35 (50/49/1 éther de pétrole/acétate d'éthyle/acide acétique)
RMN$^1$H ($CDCl_3$/TMS) : 9,4(s,1H) ; 6,6(s,3H) ; 6,2(m,1H) ; 5,85(s,2H) ; 4,5(m,1H) ; 3,0-2,3(m,5H) ; 1,9-0,8(m,8H). RMN$^{13}$C ($CDCl_3$) : 175,7 ; 173,5 ; 147,8 ; 146,4 ; 132,3 ; 121,9 ; 109,2 ; 108,4 ; 100,8 ; 54,0 ; 53,5 ; 51,9 ; 38,1 ; 34,0 ; 33,7 ; 26,3 ; 25,8 ; 18,5 ; 18,2 ; 13,4.
IR (nujol) : 3350, 1740, 1630 $cm^{-1}$

Microanalyse : ($C_{16}H_{21}NO_5S$)

| | | | |
|---|---|---|---|
| Calc. % | C = 56,62 | H = 6,23 | N = 4,13 |
| Tr. % | C = 56,41 | H = 6,08 | N = 4,25 |

Exemple 13 : Préparation du N-(RS)-[oxo-1 (acétylthiométhyl)-2 (méthylènedioxy-3,4 phényl)-3 propyl]-(S)-norleucinate de benzyle

On couple l'acide acétylthiométhyl-2 (méthylènedioxy-3,4 phényl)-3 propanoïque sous sa forme racémique (exemple 1, etape d) avec le norleucinate de benzyle de configuration (S) selon le mode opératoire de l'exemple 1 (étape f).
Rendement = 85%
F = 104°C-126°C (Microscope) (mélange 50/50 de diastéréoisomères)
RMN$^1$H ($CDCl_3$,TMS) : 7,3(s,5H) ; 6,6(s,3H) ; 5,9(s,3H) ; 5,1(s,2H) ; 4,5(m,1H) ; 3,2-2,4(m,5H) ; 2,3(s,3H) ; 1,6(m,2H) ; 1,2(m,4H) ; 0,8(m,3H).
RMN$^{13}$C($CDCl_3$) : 195,5 ; 172,3 ; 171,8 ; 147,7 ; 146,6 ; 135,3 ; 132,4 ; 132,2 ; 128,3 ; 128,0 ; 121,8 ; 109,1 ; 108,1 ; 100,7 ; 66,7 ; 51,8 ; 49,6 ; 49,4 ; 37,9 ; 31,9 ; 30,9 ; 30,2 ; 26,8 ; 22,1 ; 13,7.
IR (nujol) : 3300, 1740, 1680, 1640 $cm^{-1}$

Microanalyse : ($C_{26}H_{31}NO_6S$)

| | | | |
|---|---|---|---|
| Calc. % | C = 64,31 | H = 6,43 | N = 2,88 |
| Tr. % | C = 64,50 | H = 6,53 | N = 3,06 |

Exemple 14 : Préparation de la N-(RS)-[oxo-1 (mercaptométhyl)-2 (méthylènedioxy-3,4 phényl)-3 propyl]-(S)-norleucine

On effectue la déprotection selon le mode opératoire de l'exemple 1 (étape g).
Rendement = 75%
Rf = 0,35 (50/49/1 éther de pétrole/acétate d'éthyle/acide acétique)
(mélange 50/50 de diastéréoisomères)
RMN$^1$H ($CDCl_3$/TMS) : 8,2(s,1H) ; 6,6(s,3H) ; 6,4(m,1H) ; 5,8(s,2H) ; 4,5(m,1H) ; 2,9-2,3(m,5H) ; 1,9-0,7(m,10H) RMN13C ($CDCl_3$) : 175,1 ; 173,5 ; 147,8 ; 146,4 ; 132,3 ; 121,8 ; 109,2 ; 108,4 ; 100,8 ; 53,9 ; 53,6 ; 52,1 ; 37,9 ; 31,6 ; 31,3 ; 27,0 ; 26,3 ; 25,9 ; 22,0 ; 13,5
IR ($CDCl_3$) :3400, 1700, 1640 $cm^{-1}$

Microanalyse : ($C_{17}H_{23}NO_5S$)

| | | | |
|---|---|---|---|
| Calc. % | C = 57,77 | H = 6,56 | N = 3,96 |
| Tr. % | C = 57,56 | H = 6,34 | N = 3,69 |

Exemple 15 : Préparation du N-(RS)-[oxo-1 (acétylthiométhyl)-2 (méthylènedioxy-3,4 phényl)-3 propyl]-(S)-leucinate de benzyle

On couple l'acide acétylthiométhyl-2 (méthylènedioxy-3,4 phényl)-3 propanoïque sous sa forme racémique (exemple 1, etape d) avec le leucinate de benzyle de configuration (S) selon le mode opératoire de l'exemple 1 (étape f).

Rendement = 82%

F = 74°C (Microscope) (Mélange 50/50 de diastéréoisomères)

RMN$^1$H ($CDCl_3$, TMS) : 7,3(s,5H) ; 6,6(s,3H) ; 5,9(m,3H) ; 5,1(d,J = 3Hz,2H) ; 4,55(m,1H) ; 3,15-2,45(m,5H) ; 2,3(s,3H) ; 1,3(m,3H) ; 0,9(d,J = 5Hz,3H) ; 0,75(d,J = 5Hz,3H).

RMN$^{13}$C ($CDCl_3$) : 195,6 ; 195,4 ; 172,3 ; 172,1 ; 147,7 ;146,1 ; 135,4 ; 132,4 ; 132,15 ; 128,4 ; 128,1 ; 121,8 ; 109,2 ; 108,0 ; 100,6 ; 66,7 ; 50,7 ; 50,4 ; 49,6 ; 49,2 ; 41,5 ; 37,8 ; 31,3 ; 30,9 ; 30,2 ; 24,6 ; 24,2 ; 22,4 ; 21,7 ; 21,4.

IR (nujol) : 3300, 1730, 1690, 1640 $cm^{-1}$

Microanalyse : ($C_{26}H_{31}NO_6S$)

| | | | |
|---|---|---|---|
| Calc. % | C = 64,31 | H = 6,43 | N = 2,88 |
| Tr. % | C = 64,25 | H = 6,38 | N = 2,90 |

Exemple 16 : Préparation de la N-(RS)-[oxo-1 (mercaptométhyl)-2 (méthylènedioxy-3,4 phényl)-3 propyl]-(S)-leucine (Mélange 50/50 de diastéréoisomères)

On effectue la déprotection selon le mode opératoire de l'exemple 1 (étape g).

Rendement = 69%

Rf = 0,7 (acétate d'éthyle/acide acétique 98/2)

RMN$^1$H ($CDCl_3$/TMS) : 10,1(s,1H) ; 6,65(s,3H) ; 6,3(t,J = 7Hz,1H) ; 5,85(s,2H) ; 4,5(m,1H) ; 3,1-2,2(m,5H) ; 1,65(m,4H) ; 0,9(m,6H).

RMN$^{13}$C ($CDCl_3$) : 176,2 ; 173,7 ; 147,8 ; 146,35 ; 132,4 ; 132,15 ; 121,95 ; 109,2 ; 108,2 ; 100,8 ; 53,95 ; 53,35 ; 50,8 ; 50,6 ; 41,2 ; 40,85 ; 37,95 ; 26,4 ; 25,8 ; 24,7 ; 24,45 ; 22,65 ; 21,7 ; 21,4

IR (nujol) : 3340, 1730, 1630 $cm^{-1}$

Microanalyse : ($C_{17}H_{23}NO_5S$)

| | | | |
|---|---|---|---|
| Calc. % | C = 57,77 | H = 6,56 | N = 3,96 |
| Tr. % | C = 57,48 | H = 6,43 | N = 3,62 |

Exemple 17 : Préparation du N-(RS)-[oxo-1 (acétylthiométhyl)-2 (méthylènedioxy-3,4 phényl)-3 propyl]-(S)-tryptophanate de méthyle

On couple l'acide acétylthiométhyl-2 (méthylènedioxy-3,4 phényl)-3 propanoïque sous sa forme racémique (exemple 1, étape d) avec le (S) tryptophanate de méthyle.

Rendement = 81%

F = 93°C-130°C (Microscope) (mélange 50/50 de diastéréoisomères)

RMN$^1$H ($CDCl_3$, TMS) : 8,3(m,1H) ; 7,65-6,85(M,4H) ; 6,6(m,3H) ; 6,0(t,J = 6,7Hz,1H) ; 5,85(s,2H) ; 4,9(m,1H) ; 3,6(s,3H) ; 3,25(d,J = 6,7Hz,2H) ; 3,2-2,3(m,5H) ; 2,25(s,3H).

RMN$^{13}$ C ($CDCl_3$) : 195,6 ; 172,5 ; 171,8 ; 147,55 ; 146,1 ; 136,15 ; 132,4 132,0 ; 127,4 ; 122,9 ; 122,7 ; 121,9 ; 119,4 ; 118,4 ; 111,15 ; 109,3 ; 109,1 ; 108,1 ; 100,7 ; 52,5 ; 51,9 ; 49,35 ; 49,1 ; 37,8 ; 31,0 ; 30,65 ; 30,2 ; 27,7 ; 27,5.

IR (nujol) : 3420, 3320, 1730, 1680, 1640 $cm^{-1}$

Microanalyse : ($C_{25}H_{26}N_2O_6S$)

| | | | |
|---|---|---|---|
| Calc. % | C = 62,23 | H = 5,43 | N = 5,80 |
| Tr. % | C = 62,01 | H = 5,74 | N = 5,46 |

Exemple 18 : Préparation du N-(RS)-[oxo-1 (mercaptométhyl)-2 (méthylènedioxy-3,4 phényl)-3 propyl]-(S)-tryptophane

On effectue la déprotection selon le mode opératoire de l'exemple 1 (étape g).
Rendement = 71%
F = 66°C (Microscope) (mélange 50/50 de diastéréoisomères)
RMN$^1$H ($CDCl_3$, DMSO $d_6$,/TMS) : 8,6(s,1H) ; 8,35(s,1H) ; 7,6(m,1H) ; 7,4-6,85(m,4H) ; 6,75-6,2(m,4H) ; 5,75(d,J = 6,7Hz,2H) ; 4,35(m,1H) ; 3,1(m,2H) ; 2,9-2,0(m,5H) ; 1,4(t,J = 8 Hz,1H).
RMN$^{13}$ C ($CDCl_3$) : 175,1 ; 173,8 ; 147,7 ; 146,1 ; 136,0 ; 132,3 ; 131,8 ; 127,4 ; 123,3 ; 123,0 ; 122,2 ; 121,9 ; 119,6 ; 118,4 ; 118,2 ; 111,2 ; 109,2 ; 108,2 ; 100,7 ; 53,5 ; 52,8 ; 52,6 ; 37,5 ; 27,1 ; 26,8 ; 26,0 ; 25,4 IR (nujol) : 3400, 3350, 1720, 1635 cm$^{-1}$

Microanalyse : ($C_{22}H_{22}N_2O_5S$)
Calc. % C = 61,96 H = 5,20 N = 6,57
Tr. % C = 60,59 H = 5,46 N = 6,82

Exemple 19 : Préparation du N-(RS)-[oxo-1 (acétylthiométhyl)-2 (méthylènedioxy-3,4 phényl)-3 propyl]-(S)-phénylalaninate de benzyle

On couple l'acide acétylthiométhyl-2 (méthylènedioxy-3,4 phényl)-3 propanoïque sous sa forme racémique (exemple 1, étape d) avec le (S) phénylalaninate de benzyle selon le mode opératoire de l'exemple 1 (étape f).
Rendement = 73%
F = 99°C-105°C (Microscope) (mélange 50/50 de diastéréoisomères)
RMN$^1$H ($CDCl_3$,TMS) : 7,3(s,5H) ; 7,3-6,9(m,5H) ; 6,6(s,3H) ; 5,9(m,1H) ; 5,85(s,2H) ; 5,1(s,2H) ; 4,8(t,J = 7Hz,1H) ; 3,2-2,5(m,5H) ; 2,3(s,1/2H) ; 2,25(s,1/2H).
RMN$^{13}$ C ($CDCl_3$) : 195,4 ; 172,1 ; 170,8 ; 147,6 ; 146,1 ; 135,3 ; 134,8 ; 132,7 ; 131,9 ; 129,0 ; 128,3 ; 127,0 ; 121,5 ; 109,0 ; 108,0 ; 100,6 ; 66,8 ; 52,5 ; 49,5 ; 37,7 ; 31,15 ; 30,7 ; 30,2.
IR (nujol) : 3300, 1725, 1685, 1640 cm$^{-1}$

Microanalyse : ($C_{29}H_{29}NO_6S$)
Calc. % C = 67,03 H = 5,63 N = 2,69
Tr. % C = 67,02 H = 5,51 N = 2,90

Exemple 20 : Préparation de la N-(RS)-[oxo-1 (mercaptométhyl)-2 (méthylènedioxy-3,4 phényl)-3 propyl]-(S)-phénylalanine (Mélange 50/50 de diastéréoisomères)

On effectue la déprotection selon le mode opératoire de l'exemple 1 (étape g).
Rendement = 79%
Rf = 0,7 (acétate d'éthyle/acide acétique : 98:2)
RMN$^1$H ($CDCl_3$/TMS) : 8,8(s,1H) ; 7,3-6,7(m,5H) ; 6,6(m,3H) ; 6,1(d,J = 7,3 Hz,1H) ; 5,85(m,2H) ; 4,85(m,1H) ; 3,3-2,1(m,7H) ; 1,5(t,J = 8,5 Hz, 1H).
RMN$^{13}$ C ($CDCl_3$) : 174,5 ; 173,3 ; 147,7 ; 146,2 ; 135,3 ; 132,1 ; 129,3 ; 129,1 ; 128,5 ; 127,1 ; 121,9 ; 109,2 ; 108,9 ; 108,2 ; 100,8 ; 53,6 ; 53,3 ; 52,9 ; 52,6 ; 37,7 ; 37,2 ; 26,0 ; 25,5.
IR (CDC$^{13}$) : 3420, 1720, 1660 cm$^{-1}$

Microanalyse : ($C_{20}H_{21}NO_5S$)
Calc. % C = 62,00 H = 5,46 N = 3,61
Tr. % C = 61,71 H = 5,19 N = 3,40

Exemple 21 : Préparation du N-(RS)[oxo-1 (acétylthiométhyl)-2 (méthylènedioxy-3,4 phényl)-3 propyl]-(S)tyrosinate de benzyle (mélange 50/50 de diastéréoisomères)

On couple l'acide acétylthiométhyl-2 (méthylènedioxy-3,4 phényl)-3 propanoïque sous sa forme racémique (exemple 1, étape d) avec le (S) tyrosinate de benzyle selon le mode opératoire de l'exemple 1 (étape f).
Rendement = 90% (chromatographié)
Rf = 0,7 (50/50 éther de pétrole/acétate d'éthyle)
F <45°C
RMN$^1$H ($CDCl_3$,TMS) : 7,25(s,5H) ; 7,15-6,3(m,8H dont 1H échangeable par $D_2O$) ; 6,1(d,J = 8Hz,1H) ; 5,8(s,2H) ; 5,1(s,2H) ; 4,8(t,J=6,3Hz,1H) ; 3,2-2,4(m,7H) ; 2,23(s,1,5H) ; 2,19(s,1,5H).
RMN$^{13}$ C ($CDCl_3$) : 196,0 ; 172,7 ; 171,0 ; 155,3 ; 147,7 ; 146,2 ; 134,9 ; 131,9 ; 130,3 ; 128,5 ; 126,8 ; 126,6 ; 121,9 ; 115,4 ; 109,1 ; 108,2 ; 100,7 ; 67,0 ; 53,2 ; 53,0 ; 49,7 ; 49,4 ; 37,9 ; 37,0 ; 31,1 ; 30,6 ; 30,3
IR (nujol) : 3300, 1730, 1690, 1635 $cm^{-1}$

Microanalyse : ($C_{29}H_{29}NO_7S$)
Calc. % C = 65,03 H = 5,45 N = 2,61
Tr. % C = 65,19 H = 5,39 = 2,73

Exemple 22 : Préparation de la N-(RS)-[oxo-1 (mercaptométhyl)-2 (méthylènedioxy-3,4 phényl)-3 propyl]-(S)-tyrosine (mélange 50/50 de diastéréoisomères

On effectue la déprotection du composé de l'exemple 21 selon le mode opératoire de l'exemple 1 (étape g).
Rendement = 85% (chromatographié)
Rf = 0,5 (50/49/1 éther de pétrole/acétate d'éthyle/acide acétique)
F = 62-65°C (Microscope)
RMN$^1$ H($CDCl_3$, $DMSOd_6$, TMS) : 7,3-6,00(m,10H) ; 5,85(s large,2H) ; 4,65(m,1H) ; 3,15-2,00(m,7H) ; 1,65(t,J = 8Hz, 1H)
IR (nujol) : 3300, 1730, 1640 $cm^{-1}$

Microanalyse : $C_{20}H_{21}NO6_S$
Calc. % C = 59,54 H = 5,24 N = 3,47
Tr. % C = 59,36 H = 5,30 N = 3,38

Exemple 23 : Préparation du N-(S)-[oxo-1 (acétylthiométhyl)-2 (méthylènedioxy-3,4 phényl)-3 propyl]-(S)-sérinate de méthyle

On couple l'acide (S)-(acétylthiométhyl)-2 (méthylènedioxy-3,4 phényl)-3 propanoïque (exemple 2) avec le (S) sérinate de méthyle selon le mode opératoire de l'exemple 1 (étape f).
Rendement = 89% (chromatographié)
F = 120°C
$[\alpha]_D^{20}$ = -15,5° (c = 1,1 ; $CHCl_3$)
IR (nujol) : 3460, 3260, 1725, 1690, 1630 $cm^{-1}$
RMN$^1$H ($CDCl_3$/TMS) : 6,85(m,3H) ; 6,4(d,1H,J = 6,6Hz) ; 5,85(s,2H) ; 4,7 à 4,4(m,1H) ; 4,0 à 3,8(m,2H) ; 3,7(s,3H) ; 3,2 à 2,4(m,6H) ; 2,25(s,3H).

Microanalyse : $C_{17}H_{21}O_7NS$
Calc. % C = 53,26 H = 5,48 N = 3,65
Tr. % C = 53,46 H = 5,59 N = 3,85

Exemple 24 : Préparation de la N-(S)-[oxo-1 (mercaptométhyl)-2 (méthylènedioxy-3,4 phényl)-3 propyl]-(S)-sérine

On effectue la déprotection du composé de l'exemple 23 selon le mode opératoire de l'exemple 1 (étape g).
Rendement = 65% (chromatographié)
F = 124°C
$[\alpha]_D^{20}$ = +20,3° (c = 1,0 ; EtOH)
IR(nujol) : 3340, 1750, 1630 cm-1
RMN[1] H($CDCl_3$/DMSO/TMS) : 7,0(d,1H,J = 7,7Hz) ; 6,7(s,3H) ; 5,85(s,2H) ; 5,2 à 4,4(m,3H) ; 3,9(d,2H,J = 3,5 Hz) ; 3,0 à 2,4(m,5H) ; 1,6(t,1H,J = 8,6 Hz)

Microanalyse : $C_{14}H_{17}O_6NS$

| | | | | |
|---|---|---|---|---|
| Calc. | % | C = 51,37 | H = 5,19 | N = 4,28 |
| Tr. | % | C = 51,08 | H = 5,24 | N = 4,26 |

Exemple 25 : Préparation du N-(S)-[oxo-1 (acétylthiométhyl)-2 (méthylènedioxy-3,4 phényl)-3]-(S) méthioninate de méthyle

On couple l'acide (S)-acétylthiométhyl-2 (méthylènedioxy-3,4 phényl)-3 propanoïque (exemple 2) avec le (S) méthioninate de méthyle selon le mode opératoire de l'exemple 1 (étape f).
Rendement = 71% (chromatographié)
F = 85°C
$[\alpha]_D^{20}$ = -33,3° (c = 1,3 ; $CHCl_3$)
IR (nujol) : 3280, 1730, 1690, 1640 $cm^{-1}$
RMN1H ($CDCl_3$/TMS) : 6,85(m,3H) ; 6,2 à 5,9(m,1H) ; 5,85(s,2H) ; 4,6(quintuplet, 1H,J = 6,6 Hz) ; 3,65(s,3H) ; 3,1(d,2H,J = 6,6Hz) ; 3,0 à 2,3(m,7H) ; 2,25(s,3H) ; 2,0(s,3H)

Microanalyse : $C_{19}H_{25}O_6NS_2$

| | | | | |
|---|---|---|---|---|
| Calc. | % | C = 53,38H = 5,89 | | N = 3,28 |
| Tr. | % | C = 53,74 | H = 5,67 | N = 3,53 |

Exemple 26 : Préparation de la N-(S)-[oxo-1 (mercaptométhyl)-2 (méthylènedioxy-3,4 phényl)-3 propyl]-(S)-méthionine

On effectue la déprotection du composé de l'exemple 25 selon le mode opératoire de l'exemple 1 (étape g).
Rendement = 56% (chromatographié)
$[\alpha]_D^{20}$ = +2,2° (c = 0,9 ; EtOH)
IR : 3300, 1720, 1640 $cm^{-1}$
RMN[1]H ($CDCl_3$/TMS) : 9,5(s,1H) ; 6,6(m,4H) ; 5,8(s,2H) ; 4,9 à 4,4(m,1H) ; 3,0 à 1,3(m,10H) ; 2,0(s,3H)

Microanalyse : $C_{16}H_{21}O_5NS_2$

| | | | | |
|---|---|---|---|---|
| Calc. | % | C = 51,73 | H = 5,70 | N = 3,77 |
| Tr. | % | C = 51,97 | H = 5,79 | N = 3,75 |

Exemple 27 : Préparation du N-(S)-[oxo-1 (acéthylthiométhyl)-2 (méthylènedioxy-3,4 phényl)-3 propyl]-(RS)-méthioninate sulfoxyde de méthyle

On couple l'acide (S)-(acétylthiométhyl)-2 (méthylènedioxy-3,4 phényl)-3 propanoïque (exemple 2) avec le (RS) méthioninate sulfoxyde de méthyle selon le mode opératoire de l'exemple 1 (étape f).
Rendement = 27%
RMN$^1$H ($CDCl_3$/TMS) : 6,8 à 6,5(m,3H) ; 6,2(d,2H,J=8Hz) ; 5,85(D,2H) ; 4,75 à 4,4(m,1H) ; 3,65 et 3,6(s,3H) ; 3,05 (d,2H,J = 6,7 Hz) ; 3,0 à 1,5 (m,7H) ; 2,25 (s,3H) ; 2,05 et 1,95 (s,3H).
IR(nujol) : 3300, 1740, 1690, 1650 $cm^{-1}$.

Microanalyse : $C_{19}H_{25}O_7NS_2$
Calc. % C = 51,45 N = 3,16 H = 5,68
Tr. % C = 51,39 N = 3,23 H = 5,52.

Exemple 28 : Préparation de la N-(S)-[oxo-1 (mercaptométhyl)-2 (méthylènedioxy-3,4 phényl)-3 propyl]-(RS)-méthionine sulfoxyde

On effectue la déprotection du composé de l'exemple 27 selon le mode opératoire de l'exemple 1 (étape g).
Rendement = 71%
RMN$^1$H ($CDCl_3$/TMS) : 8,2(s,1H) ; 6,8 à 6,2(m,4H) ; 5,9 et 5,85(s,2H) ; 4,8 à 4,5(m,1H) ; 3,2 à 1,3(m,9H) ; 2,05 et 1,95(s,3H) ; 1,55(t,1H,J=8Hz).
IR(nujol) : 1725, 1630 $cm^{-1}$

Microanalyse : $C_{16}H_{21}O_6NS_2$
Calc. % C = 49,60 N = 3,61 H = 5,46
Tr. % C = 50,00 N = 3,72 H = 5,39

Exemple 29 : Préparation du N-(S)-[oxo-1 (acétylthiométhyl)-2 (méthylènedioxy-3,4 phenyl)-3 propyl]-(RS)-(méthylènedioxy-3,4 phényl)-3 alaninate de méthyle

On couple l'acide (S)-(acétylthiométhyl)-2 (méthylènedioxy-3,4 phényl)-3 propanoïque (exemple 2) avec le (RS)-(méthylènedioxy-3,4 phényl)-3 alaninate de méthyle selon le mode opératoire de l'exemple 1 (étape f).
Rendement = 58%
F = 98°C
RMN$^1$H ($CDCl_3$/TMS) : 6,8 à 6,0(m,6H) ; 5,85(s,4H) ; 5,8 à 5,6(m,1H) ; 4,95 à 4,55(m,1H) ; 3,65 et 3,6(s,3H) ; 3,15 à 2,4(m,7H) ; 2,3 et 2,25(s,3H).
IR (nujol) : 3300, 1730, 1700 à 1680, 1650, 1645 $cm^{-1}$

Microanalyse : $C_{24}H_{25}O_8NS$
Calc. % C = 59,13 N = 2,87 H = 5,17
Tr. % C = 59,50 N = 2,90 H = 5,23.

Exemple 30 : Préparation de la (N-(S)-[oxo-1 (mercaptométhyl)-2 (méthylènedioxy-3,4 phényl)-3 propyl]-(RS)-(méthylènedioxy-3,4 phényl)-3 alanine

On effectue la déprotection du composé de l'exemple 29 selon le mode opératoire de l'exemple 1 (étape g).
Rendement = 74%
F = 121°C
RMN$^1$H ($CDCl_3$/TMS) : 9,4(s,1H) ; 7,0 à 6,0(m,6H) ; 5,8(s,4H) ; 5,0 à 4,6(m,1H) ; 3,9 à 3,6(m,1H) ; 3,25 à

2,2(m,7H) ; 1,5 et 1,4(t,1H,J=8Hz).
IR (nujol) : 1705, 1640 $cm^{-1}$

Microanalyse : $C_{21}H_{21}O_7NS$

Calc. % C = 58,46 N = 3,25 H = 4,91
Tr. % C = 58,76 N = 3,39 H = 4,97.

Exemple 31 : Préparation du N-(RS)-[oxo-1 (acétylthiométhyl)-2 (éthylènedioxy-3,4 phényl)-3 propyl]-glycinate de benzyle

A. Préparation de l'acide -(RS)-acétylthiométhyl-2 (éthylènedioxy-3,4 phényl)-3 propanoïque

On l'obtient comme dans l'exemple (étape d), par addition de l'acide thioacétique avec l'acide ((éthylènedioxy-3,4 phényl)méthyl)-2 propénoïque.
Rendement = 98%
IR ($cm^{-1}$) : 1720, 1690
RMN¹H ($CDCl_3$/TMS) : 8,5(s,1H) ; 6,8 à 6,5(m,3H) ; 4,2(s,4H) ; 3,15 à 2,7(m,5H) ; 2,3(s,3H).

B. Préparation du N-(RS)-[oxo-1 (acétylthiométhyl)-2 (éthylènedioxy-3,4 phényl)-3 propyl]-glycinate de benzyle

On couple l'acide obtenu sous A avec le glycinate de benzyle en présence d'HOBT et de DCC selon le mode opératoire décrit dans l'exemple 1.
Rendement = 92% (chromatographié)
F = 62°C (Microscope)
IR (nujol) : 3290, 1745, 1670, 1640 $cm^{-1}$
RMN¹H ($CDCl_3$/TMS) : 7,4(s,5H) ; 7,0 à 6,55(m,3H) ; 5,9(s,1H) ; 5,2(s,2H) ; 4,2(s,4H) ; 4,0(dd,1H,J = 5,3Hz) ; 3,95(dd,1H,J = 5,3Hz) ; 3,2 à 2,4(m,5H) ; 2,3(s,3H).
RMN¹³C($CDCl_3$) : 196,4(s) ; 173,1(s) ; 169,3(s) ; 143,2(s) ; 142,1(s) ; 135,3(s) ; 131,5(s) ; 128,4(d) ; 128,0 (d) ; 121,6(d) ; 117,3(d) ; 116,9(d) ; 66,7(t) ; 63,9(t) ; 48,6(d) ; 41,1(t) ; 37,2(t) ; 30,7(t) ; 30,2(q).

Microanalyse : $C_{23}H_{25}O_6NS$
Calc. % C = 62,30 N = 3,16 H = 5,64
Tr. % C = 62,15 N = 3,02 H = 5,60

Exemple 32 : Préparation de la N-(RS)-[oxo-1 (mercaptométhyl)-2 éthylènedioxy-3,4 phényl)-3 propyl]-glycine

On effectue la déprotection du composé de l'exemple 31 selon le mode opératoire de l'exemple 1 (étape g).
Rendement = 66%
F = 138°C (Microscope)
IR (nujol) : 3480, 1745, 1630 $cm^{-1}$
RMN¹H ($CDCl_3$-DMSO $d_6$/TMS) : 7,6(s,1H) ; 6,75(s,3H) ; 5,8(m,1H) ; 4,2(S,4H) ; 3,9(d,2H,J = 5,3Hz) ; 3,15 à 2,1(m,5H) ; 1,7(t,1H,J = 6,6Hz).

Microanalyse : $C_{14}H_{17}O_5NS$
Calc. % C = 54,02 N = 4,50 H = 5,47
Tr. % C = 53,76 N = 4,26 H = 5,38

Exemple 33 : Préparation du N-(RS)-[oxo-1 (acétylthiométhyl)-2 (éthylènedioxy-3,4 phényl)-3 propyl]-(S)-alaninate de benzyle (mélange 50/50 de diastéréoisomères)

On couple l'acide acétylthiométhyl-2 (éthylènedioxy-3,4 phényl)-3 propanoïque sous la forme racémique (exemple 1, étape d) avec le (S) alaninate de benzyle selon le mode opératoire de l'exemple 1 (étape f).
Rendement = 78%
Rf = 0,2 (50/50 éther/éther de pétrole)
RMN$^1$H (CDCl$_3$/TMS) : 7,3(s,5H) ; 6,8-6,5(m,3H) ; 6,0(m,1H) ; 5,1(s,2H) ; 4,5(m,1H) ; 4,2(s,4H) ; 3,2-2,4(m,5H) ; 2,3(s,3H) ; 1,35(d,J=7Hz,1,5H) ; 1,15(d,J=7Hz,1,5H) ;
RMN $^{13}$C(CDCl$_3$) : 195,9(C$_5$) ; 172,3(C$_1$, C$_{16}$) ; 143,2 ; 142,2(C$_{10}$, C$_{11}$ ) ; 135,3(C$_{18}$) ; 131,8 ; 131,5(C$_7$) ; 128,5 ; 128,3 ; 128,0(C$_{19}$, C$_{20}$, C$_{21}$) ; 121,7(C$_8$) ; 117,5 ; 117,1(C$_9$, C$_{12}$) ; 66,8(C$_{17}$) ; 64,2(C$_{13}$, C$_{14}$) ; 49,4 ; 49,0 ; 48,0 ; 47,8(C$_2$,C$_{15}$) ; 37,7 ; 37,5(C$_3$) ; 30,9 ; 30,7 ; 30,3(C$_4$,C$_6$) ; 18,3 ; 18,0(C$_{22}$).
IR(CCl$_4$) : 3400, 1735, 1675 cm$^{-1}$

Microanalyse : $C_{24}H_{27}NO_6S$

| | | | |
|---|---|---|---|
| Calc. % | C = 63,00 | N = 5,95 | N = 3,06 |
| Tr. % | C = 62,73 | H = 5,93 | N = 3,29 |

Exemple 34 : Préparation de la N-(RS)-[oxo-1 (mercaptométhyl)-2(éthylènedioxy-3,4 phényl)-3 propyl]-(S-alanine (mélange 50/50 de diastéréoisomères)

On effectue la déprotection du composé de l'exemple 33 selon le mode opératoire de l'exemple 1 (étape g).
Rendement = 86%
Rf = 0,3 (50/49/1 éther de pétrole/acétate d'éthyle/acide acétique)
RMN$^1$H (CDCl$_3$/TMS) : 9,05(s,1H) ; 6,9-6,4(m,3H) ; 6,25(m,1H) ; 4,55(m,1H) ; 4,2(s,4H) ; 3,05-2,3(m,5H) ; 1,6(t,J = 8,3Hz,1H) ; 1,4(d,J =6,7 Hz,1,5H) ; 1,25(d,J = 6,7Hz,1,5Hz).
RMN$^{13}$C (CDCl$_3$) : 175,9 ; 173,7 (C$_1$,C$_{14}$) ; 143,4 ; 142,3 (C$_8$,C$_9$) ; 131,8 ; 131,5 (C$_5$) ; 121,8 (C$_6$) ; 117,6 ; 117,3 (C$_7$,C$_{10}$) ; 64,3 (C$_{11}$,C$_{12}$) ; 53,6 ; 53,1 (C$_2$) ; 48,0 (C$_{13}$) ; 37,6 ; 37,3 (C$_3$) ; 26,0 ; 25,8 (C$_4$) ; 18,0 ; 17,7 (C$_{15}$). IR (CDCl$_3$) : 3400, 1720, 1650 cm$^{-1}$

Microanalyse : $C_{15}H_{19}NO_5S$

| | | | |
|---|---|---|---|
| Calc. % | C = 55,37 | H = 5,88 | N = 4,30 |
| Tr. % | C = 55,26 | H = 5,65 | N = 4,19 |

Exemple 35 : Préparation du N-(RS)[oxo-1(acétylthiométhyl)-2 (méthylènedioxy-2,3 phényl)-3 propyl]-glycinate de benzyle

A. Préparation de l'acide (RS)-acétylthiométhyl-2 (méthylènedioxy-2,3 phényl)-3 propanoïque.

On l'obtient comme dans l'exemple 1 (etape d, par addition de l'acide thioacétique avec l'acide ((méthylènedioxy-3,4 phényl)méthyl)-2 propénoïque.
Rendement = 85%
IR(cm$^{-1}$) : 1710 ; 1690
RMN$^1$H (CDCl$_3$/TMS) : 9(s,1H) ; 6,6 (s,3H) ; 5,8(s,2H) ; 3,2 à 2,4(m,5H) ; 2,2(s,3H).

B. Préparation du N-(RS)-[oxo-1(acétylthiométhyl)-2(méthylènedioxy-2,3 phényl)-3 propyl]-glycinate de benzyle

On couple le composé obtenu sous A avec le glycinate de benzyle selon le mode opératoire de l'exemple 1 (étape f).
Rendement = 68%
F = 60°C
IR (nujol) : 3320, 1740, 1690, 1650 cm$^{-1}$

RMN[1]H ($CDCl_3$/TMS) : 7,2(s,5H) ; 6,8(s,3H) ; 6,3-6(m,IH) ; 5,8(s,2H) ; 5,1(s,2H) ; 3,95(d,2H,J = 5Hz) ; 3,2-2,4(m,5H) ; 2,2(s,3H)
RMN[13]C($CDCl_3$) : 195,5 ; 172,9 ; 169,3 ; 146,9 ; 145,4 ; 134,9 ; 128,4 ; 122,9 ; 121,3 ; 120 ; 107 ; 100,3 ; 66,8 ; 46,3 ; 41,1 ; 32,1 ; 30,5 ; 30,2

Microanalyse : $C_{22}H_{23}NO_6S$

| | | | |
|---|---|---|---|
| Calc. % | C = 61,52 | H = 5,39 | N = 3,26 |
| Tr. % | C = 61,47 | H = 5,36 | N = 3,34 |

Exemple 36 : Préparation de la N-(RS)-[oxo-1 (mercaptométhyl)-2(méthylènedioxy-2,3 phényl)-3 propyl]glycine

On effectue la déprotection du composé de l'exemple 35 selon le mode opératoire de l'exemple 1 (étape g).
Rendement = 71%
IR ($CDCl_3$) : 3390, 1740, 1640 $cm^{-1}$
RMN[1]H ($CDCl_3$) : 9,4(s,1H) ; 6,8-6,4(m,4H) ; 5,8(s,2H) ; 4,0-3,8(m,2H) ; 3,1-2,3(m,5H) ; 1,65(t,1H,J = 7,9Hz)
RMN[13]C($CDI_3$) : 173,2 ; 171,85 ; 147 ; 145,5 ; 122,9 ; 121,45 ; 120 ; 107 ; 100,4 ; 50,3 ; 41,1 ; 32 ; 25,9.

Microanalyse : $C_{13}H_{15}NO_5S$

| | | | |
|---|---|---|---|
| Calc. % | C = 52,51 | H = 5,08 | N = 4,71 |
| Tr. % | C = 52,38 | H = 4,87 | N = 4,51 |

Exemple 37 : Préparation du N-(RS)-[oxo-1 (acétylthiométhyl)-2 (phénoxy-4 phényl)-3 propyl]-glycinate de benzyle

A. Préparation de l'acide (RS)-acétylthiométhyl-2 (phénoxy-4 phényl)-3 propanoïque

On l'obtient comme dans l'exemple 1 (étape d), par addition de l'acide thioacétique avec l'acide [(phénoxy-4 phényl)méthyl]-2 propénoïque.
Rendement = 98%
IR (film) : 1700 $cm^{-1}$
RMN[1]H ($CDCl_3$/TMS) : 9,5-9,1(m,1H) ; 7,5-6,8 (m,9H) ; 3,2-2,8 (m,5H) ; 2,2(s,3H).

B. Préparation du N-(RS)-[oxo-1 (acétylthiométhyl)-2 (phénoxy-4 phényl)-3 propyl]-glycinate de benzyle

On couple le composé obtenu sous A avec le glycinate de benzyle selon le mode opératoire de l'exemple 1 (étape f).
Rendement = 52%
F = 66°C (Microscope)
RMN[1]H ($CDCl_3$/TMS) : 7,2(s,5H) ; 7,2-6,7(m,9H) ; 5,8(t,J = 6,7Hz, 1H) ; 5,0(s,2H) ; 4-3,8(m,2H) ; 3,1-2,4(m,5H) ; 2,2(s,3H).
IR (nujol) : 3300, 1730, 1680, 1640 $cm^{-1}$

Microanalyse : ($C_{27}H_{27}NO_5S$)

| | | | |
|---|---|---|---|
| Calc. % | C = 67,90 | H = 5,69 | N = 2,93 |
| Tr. % | C = 67,59 | H = 5,55 | N = 3,03 |

Exemple 38 : Préparation de la N- (RS)-[oxo-1 (mercaptométhyl)-2 (phénoxy-4 phényl)-3 propyl]glycine

On effectue la déprotection du composé obtenu sous B dans l'exemple 37 selon le mode opératoire de l'exemple 1 (étape g).

Rendement = 60%
F = 94°C (Microscope)
RMN[1]H ($CDCl_3$/TMS) : 7,6-6,2(m,11H) ; 4,2-3,8(m,2H) ; 3,1-2,2(m,5H) ; 1,8-1,4(m,1H)
IR (nujol) : 3300, 1740, 1630 $cm^{-1}$

Microanalyse : ($C_{18}H_{19}NO_4S$)

| | | | | |
|---|---|---|---|---|
| Calc. | % | C = 62,58 | H = 5,54 | N = 4,05 |
| Tr. | % | C = 62,80 | H = 5,67 | N = 4,12 |

Exemple 39 : Préparation du N-(RS)-[oxo-1 (acétylthiométhyl)-2(phénoxy-4 phényl)-3 propyl] (S)-alaninate de benzyle

On couple le composé obtenu sous A avec le (S)-alaninate de benzyle selon le mode opératoire de l'exemple 1 (étape f).
Rendement = 73% (mélange 50/50 de diastéréoisomères)
IR ($CDCl_3$) : 3300, 1740, 1690, 1650 $cm^{-1}$
RMN[1]H ($CDCl_3$/TMS) : 7,2(s,5H) ; 7,2-6,7(m,9H) ; 6(t,J = 6,6Hz, 1H) ; 5,1(s, large,2H) ; 4,7-4,4(m,1H) ; 3,2-2,8(m,4H) ; 2,8-2,4(m,1H) ; 2,25(s,3H) ; 1,3 et 1,15(2d,3H,J = 6,6Hz)
$RMN_{13}C$ ($CDCl_3$) : 195,9 ; 195,6 ; 172,2 ; 157,4 ; 155,7 ; 135,2 ; 133,5 ; 130,2 ; 129,6 ; 128,5 ; 128 ; 122,9 ; 118,9 ; 118,5 ; 66,9 ; 49,5 ; 49,1 ; 47,7 ; 37,4 ; 30,9 ; 30,3 ; 18,3 ; 18.

Microanalyse : $C_{28}H_{29}NO_5S$

| | | | | |
|---|---|---|---|---|
| Calc. | % | C = 68,4 | N = 2,85 | H = 5,94 |
| Tr. | % | C = 68,15 | N = 3,15 | H =6,06 |

Exemple 40 : Préparation de la N-(RS)-[oxo-1 (mercaptométhyl)-2(phénoxy-4 phényl)-3 propyl]-(S)-alanine

On effectue la déprotection du composé de l'exemple 39 selon le mode opératoire de l'exemple 1 (étape g).
Rendement = 63% (mélange 50/50 de diastéréoisomères)
IR ($CDCl_3$) : 3420, 1720, 1650 $cm^{-1}$ ;
RMN[1]H ($CDCl_3$/TMS) : 9,3 (s,1H) ; 7,4-.6,7(m,9H) ; 6,3(t,J = 8 Hz,1H) ; 4,8-4,4 (m,1H) ; 3,1-2,2(m,5H) ; 1,45 et 1,25 (2d, 3H, J = 7,1 Hz)
RMN[13]C ($CDCl_3$) : 174,9 ; 173,1 ; 157,3 ; 155,7 ; 133,25 ; 130,1 ; 129,6 ; 123 ; 118,9 ; 118,5 ; 53,2 ; 52,75 ; 48,15 ; 47,9 ; 37,45 ; 37,1 ; 26 ; 25,8 ; 18,1 ; 17,8.

Microanalyse : $C_{19}H_{21}NO_4S$

| | | | | |
|---|---|---|---|---|
| Calc. | % | C = 63,48 | N = 3,89 | H = 5,88 |
| Tr. | % | C = 63,25 | N = 3,72 | H = 6,1 |

Exemple 41 : Préparation du N -(RS)-[oxo-1 (acétylthiométhyl)-2 (phényl-4 phényl)-3 propyl]-glycinate de benzyle

A. Préparation de l'acide -(RS)-acétylthiométhyl-2 (phényl-4 phényl)-3 propanoïque

On l'obtient comme dans l'exemple 1 (étape d), par addition de l'acide thioacétique avec l'acide [(phényl-4 phényl)méthyl]-2 propénoïque.
Rendement = 97%
IR($cm^{-1}$) : 1740, 1690
RMN[1]H ($CDCl_3$/TMS) : 9,6(s,1H) ; 7,7 à 7,1(m,9H) ; 3,2 à 2,8(m,5H) ; 2,25(s,3H)

B. Préparation du N-(RS)-[oxo-1 (acétylthiométhyl)-2 (phényl-4 phényl)-3 propyl]-glycinate de benzyle

On couple le composé obtenu sous A avec le glycinate de benzyle selon le mode opératoire de l'exemple 1 (étape f).
Rendement = 64% (chromatographié)
F = 99 - 103°C (Microscope)
IR (nujol) : 3290, 1745, 1690, 1650 $cm^{-1}$
RMN$^1$H ($CDCl_3$/TMS) : 7,7 à 7,1(m,14H) ; 5,9(m,1H) ; 5,1(s,2H) ; 3,95(m,2H) ; 3,2 à 2,8(m,5H) ; 2,3(s,3H)
RMN$^{13}$C($CDCl_3$) : 196,1(s) ; 173,3(s) ; 169,6(s) ; 140,9(s) ; 139,6(s) ; 137,8(s) ; 135,2(s) ; 129,4(d) ; 128,7(d) ; 128,3(d) ; 127,3(d) ; 127,0(d) ; 66,9(t) ; 48,9(d) ; 41,1(t) ; 37,7(t) ; 30,9(t) ; 30,3(q).

Microanalyse : $C_{27}H_{27}O_4NS$

| | | | |
|---|---|---|---|
| Calc. % | C = 70,28 | N = 3,03 | H = 5,85 |
| Tr. % | C = 70,10 | N = 3,03 | H = 6,01 |

Exemple 42 : Préparation de la N-(RS)-[oxo-1 (mercaptométhyl)-2 (phényl-4 phényl)-3 propyl]-glycine

On effectue la déprotection du composé de l'exemple 41 selon le mode opératoire de l'exemple 1 (étape g).
Rendement = 78% (chromatographié)
F = 70-73°C (Microscope)
IR(nujol) : 1735, 1670 $cm^{-1}$
RMN$^1$H ($CDCl_3$ -DMSO $d_6$/TMS) : 7,9 à 7,2(m,9H) ; 6,0(s,large,2H) ; 4,0(d,2H,J = 6,8 Hz) ; 3,4 à 2,4(m,5H) ; 1,95(s,large,1H)
RMN$^{13}$C ($CDCl_3$-DMSO $d_6$) : 173,6(s) ; 170,5(s) ; 139,7(s) ; 138,0(s) ; 137,4(s) ; 128,6(d) ; 127,9(d) ; 126,0(d) ; 125,9(d) ; 51,0(d) ; 40,2(t) ; 36,7(t) ; 25,3(t)

Microanalyse : $C_{18}H_{19}O_3NS$

| | | | |
|---|---|---|---|
| Calc. % | C = 65,65 | N = 4,25 | H = 5,77 |
| Tr. % | C = 65,63 | N = 4,13 | H = 5,93 |

Dédoublement de l'acide acétylthiométhyl-2 (phényl-4 phényl)-3 propanoïque

A une solution de 3 g (9,55 mmol) d'acide acétylthiométhyl-2 (phényl-4 phényl)-3 propanoïque racémique, on ajoute une solution de 0,79 g (4,78 mmol) de (+) éphédrine dans 25 ml d'éther. On laisse reposer 41 heures, on filtre, on lave les cristaux à l'éther et on essore.
Masse = 2,15 g
F = 132 - 142°C
$[\alpha]_D^{25}$ = +14,5° (c = 1,3 ; MeOH)

Recristallisations

On place dans un ballon 2,10 g de sel (+) et on ajoute 17 ml de chloroforme et 28 ml d'éther de pétrole. Après 22 heures, on filtre, on lave le sel à l'éther et on essore.
Masse = 1,38 g
Rendement = 66%
F = 138 - 148°C
$[\alpha]_D^{25}$ = +11,6° (c = 1,4 ; MeOH)

On répète cette opération 4 fois.
Rendement global des 5 recristallisations = 16%
F = 148°C
$[\alpha]_D^{25}$= + 5,9° (c = 1,1 ; MeOH)

Libération de l'acide (s) optiquement pur

On dissout 0,33 g de sel optiquement pur (+) dans du chloroforme, on ajoute de l'eau et on acidifie par une solution aqueuse d'HCl 1N jusqu'à pH = 1. On sépare la phase organique et on extrait encore une fois au $CHCl_3$. Les phases organiques sont réunies, lavées à l'eau, séchées sur $MgSO_4$, filtrées et concentrées.
Masse = 0,22 g
Rendement = 100%
F = 121°C
$[\alpha]_D^{25}$ = -10,1° (c = 1,3 dans le méthanol)
IR (nujol) : 1710, 1690 $cm^{-1}$
RMN$^1$ H ($CDCl_3$/TMS) : 10,95(s,1H) ; 7,7 à 6,95(m,9H) ; 3,3 à 2,6(m,5H) ; 2,2(s,3H)
RMN$^{13}$C ($CDCl_3$) : 195,1(s) ; 179,5(s) ; 140,5(s) ; 139,4(s) ; 136,4(s) ; 129,2(d) ; 128,5(d) ; 127,0(d) ; 126,8(d) ; 47,7(d) ; 36,9(t) ; 30,3(t) ; 29,5(q).

Exemple 43 : Préparation du N-(S)-[oxo-1 (acétylthiométhyl)-2 (phényl-4 phényl)-3 propyl]-glycinate de benzyle

On couple l'acide acétylthiométhyl-2 (phényl-4 phényl)-3 propanoïque sous sa forme (S) avec le glycinate de benzyle selon le mode opératoire de l'exemple 1 (étape f).
Rendement = 62% (recristallisé dans l'éther)
F = 108°C-109°C (microscope)
$[\alpha]_D^{25}$ = -6,7° (c = 1,1 dans le $CHCl_3$)
IR (nujol) : 3300, 1730, 1680, 1645 $cm^{-1}$
RMN$^1$H ($CDCl_3$/TMS) : 7,7 à 7,1(m,14H) ; 5,9(t,1H,J = 4Hz) ; 5,05(s,2H) ; 3,95(dd,1H,J = 4Hz) ; 3,9(dd,1H,J = 4Hz) ; 3,2 à 2,4(m,5H) ; 2,25(s,3H).
Le spectre RMN$^{13}$C est identique à celui du produit racémique.

Microanalyse : $C_{27}H_{27}O_4NS$

| | | | | |
|---|---|---|---|---|
| Calc. | % | C = 70,28 | N = 3,03 | H = 5,85 |
| Tr. | % | C = 69,96 | N = 3,24 | H = 5,93 |

Exemple 44 : Préparation du N-(S)-[oxo-1 (acétylthiométhyl)-2 (phényl-4 phényl)-3 propyl]-(S)-alaninate de benzyle)

On couple l'acide acétylthiométhyl-2 (phényl-4 phényl)-3 propanoïque sous sa forme (S) avec le (S) alaninate de benzyle selon le mode opératoire de l'exemple 1 (étape f).
Rendement = 67% (recristallisé dans un mélange $CHCl_3$/éther de pétrole)
F = 110°C ; un seul diastéréoisomère (Microscope)
$[\alpha]_D^{25}$ = -11,7° (c = 1,1 dans le $CHCl_3$)
IR (nujol) : 3320,1725, 1680, 1640 $cm^{-1}$
RMN$^1$H ($CDCl_3$/TMS) : 7,7 à 7,0(m,14H) ; 5,95(d,1H,J = 6,8Hz) ; 6,0(s,2H) ; 4,5(quintuplet, 1H,J = 6,8Hz) ; 3,2 à 2,4(m,5H) ; 2,25(s,3H) ; 1,3(d,3H,J = 6,8Hz)
RMN13C (CDCl3) : 195,7(s) ; 172,3(s) ; 171,9(s) ; 140,5(s) ; 139,2(s) ; 137,5(s) ; 135,0(s) ; 129,2(d) ; 128,5(d) ; 128,3(d) ; 128,2(d) ; 127,8(d) ; 126,9(d) ; 126,7(d) ; 66,8(t) ; 49,2(t) ; 47,7(d) ; 38,0(t) ; 31,1(t) ; 30,3(q) ; 17,9(q)

Microanalyse : $C_{23}H_{29}O4NS$

| | | | | |
|---|---|---|---|---|
| Calc. | % | C = 70,73 | N = 2,94 | H = 6,10 |
| Tr. | % | C = 70,33 | N = 2,97 | H = 6,10 |

Exemple 45 : Préparation de la N-(S)[oxo-1 (mercaptométhyl)-2 (phényl-4 phenyl)-3 propyl]-(S)-alanine

On effectue la déprotection du composé de l'exemple 44 selon le mode opératoire de l'exemple 1 (exemple g).
Rendement = 62% (chromatographié)
F = 131°C, un seul diastéréoisomère (Microscope)
$[\alpha]_D^{25}$ = +36,4° (c = 1,0 dans le $CHCl_3$)
IR ($CHCl_3$) : 1720, 1675 $cm^{-1}$
RMN$^1$H ($CDCl_3$/TMS) : 8,8(s,1H) ; 7,7 à 7,0(m,9H) ; 6,15(d,1H,J = 8Hz) ; 4,5(quintuplet,1H,J = 8Hz) ; 3,2 à 2,3(m,5H) ; 1,5(t,1H,J =7,2Hz) ; 1,35(d,3H,J = 8Hz)
RMN$^{13}$C ($CDCl_3$) : 176,0(s) ; 173,1(s) ; 140,5(s) ; 139,3(s) ; 137,2(s) ; 129,2 (d) ; 128,6(d) ; 127,1(d) ; 126,8(d) ; 52,8(d) ; 48,2(d) ; 37,6(t) ; 25,9(t) ; 18,0(q).

Microanalyse : $C_{31}H_{35}O_4NS$
Calc. % C = 71,95 N = 2,70 H = 6,76
Tr. % C = 72,07 N = 2,70 H = 6,80

Exemple 46 : Préparation du N-(S)-[oxo-1 (acétylthiométhyl)-2 (phényl-4 phenyl)-3 propyl]-(S)-leucinate de benzyle

On couple l'acide acétylthiométhyl-2 (phényl-4 phényl)-3 propanoïque sous sa forme (S) avec le (S)-leucinate de benzyle.
Rendement = 42% (chromatographié)
F = 100°C ; un seul diastéréoisomère (Microscope)
$[\alpha]_D^{25}$ = -20,7° (c = 1,1 dans le $CHCl_3$)
IR (nujol) : 3320, 1720, 1700, 1640 $cm^{-1}$
RMN$^1$H ($CDCl_3$/TMS) : 7,8 à 7,0(m, 14H) ; 5,9(d,1H, J = 8Hz) ; 4,95(s,2H) ; 4,75 à 4,35(m,1H) ; 3,2 à 2,4(m,5H) ; 2,2(s,3H) ; 1,75 à 1,3(m,3H) ; 0,8(d,6H,J = 4Hz)
RMN$^{13}$C ($CDCl_3$) : 195,6(s) ; 172,3(s) ; 172,0(s) ; 140,6(s) ; 139,2(s) ; 137,3(s) ; 135,1(s) ; 129,1(d) ; 128,5(d) ; 128,1(d) ; 127,8(d) ; 127,0(d) ; 126,8(d) ; 66,6(t) ; 50,6(d) ; 49,0(d) ; 41,4(t) ; 37,8(t) ; 31,0(t) ; 30,3(q) ; 24,5(d) ; 22,6(q) ; 21,7(q).

Exemple 47 : Préparation de la N-[oxo-1 (mercaptométhyl)-2 (phényl-4 phényl)-3 propyl-(S)-leucine (+)

On effectue la déprotection du composé de l'exemple 46 selon le mode opératoire de l'exemple 1 (étape g).
Rendement : 71%
F < 50°C, un seul diastéréoisomère
$[\alpha]_D^{25}$ = +33,5° (c = 1,1 dans le $CHCl_3$)
IR (nujol) : 3290,1720, 1630 $cm^{-1}$
RMN$^1$H ($CDCl_3$/TMS) : 10,2(s,1H) ; 7,7 à 7,0(m,9H) ; 6,0(d,1H,J = 8,8Hz) ; 4,75 à 4,35(m,1H) ; 3,2 à 2,3(m,5H) ; 1,9 à 1,3(m,3H) ; 1,55(t,1H,J = 7, 9Hz) ; 0,8(d,6H,J = 4Hz) ;
RMN$^{13}$C ($CDCl_3$) : 176,8(s) ; 173,4(s) ; 140,7(s) ; 139,3(s) ; 137,8(s) ; 129,2(d) ; 128,6(d) ; 127,3(d) ; 127,1(d) ; 126,9(d) ; 53,0(d) ; 50,6(d) ; 40,9(t) ; 37,6(t) ; 26,0(t) ; 24,6(d) ; 22,7(q) ; 21,5(q).

Microanalyse : $C_{22}H_{26}O_3NS$
Calc. % C = 68,75 N = 3,64 H = 6,77
Tr. % C = 67,9 N = 3,46 H = 7,22

Exemple 48 : Préparation du N-(RS)-[oxo-1 (acétylthiométhyl)-2 (fluoro-3 phényl)-3 propyl]-glycinate de benzyle

A. Préparation de l'acide (RS)-acétylthiométhyl-2 (fluoro-3 phényl)-3 propanoïque

On l'obtient comme dans l'exemple 1 (étape d), par addition de l'acide thioacétique avec l'acide [(fluoro-3 phényl)méthyl]-2 propénoïque.
Rendement = 98%
IR (film) : 1700, 1610, 1590 $cm^{-1}$
$RMN^1H$ ($CDCl_3$/TMS) : 11,25(s,1H) ; 7,45-6,75(m,4H) ; 3,3-2,6(m,5H) ; 2,3(s,3H) ;

B. Préparation du N-(RS)-[oxo-1 (acétylthiométhyl)-2 (fluoro-3 phényl)-3 propyl]-glycinate de benzyle

On couple le composé obtenu sous A avec le glycinate de benzyle selon le mode opératoire de l'exemple 1 (étape f).
Rendement = 83%
F = 60°C (Microscope)
$RMN^1H$ ($CDCl_3$/TMS) : 7,35(s,5H) ; 7,3-6,7(m,4H) ;
6,05(m,1H) ; 5,10(s,2H) ; 3,95(m,2H) ; 3,25-2,55(m,5H) ; 2,3(s,3H).
$RMN^{13}C$ ($CDCl_3$) : 195,8 ; 172,8 ; 169,3 ; 162,8(d,J = 246,6Hz) ; 141,1(d,J = 7,3Hz) ; 135,1 ; 129,8(d,J = 7,3Hz) ; 128,5 ; 124,5 ; 115,0(d,J = 46,5Hz) ; 114,0(d,J = 46,5 Hz) ; 66,9 ; 48,7 ; 41,2 ; 37,7 ; 31,0 ; 30,3.
IR (nujol) : 3300, 1730, 1680, 1640 $cm^{-1}$

Microanalyse : ($C_{21}H_{22}FNO_4S$)

| | | | |
|---|---|---|---|
| Calc. % | C = 62,51 | H = 5,49 | N = 3,47 |
| Tr. % | C = 62,70 | H = 5,35 | N = 3,64 |

Exemple 49 : Préparation de la N-(RS)-[oxo-1 (mercaptométhyl)-2 (fluoro-3 phényl)-3 propyl]-glycine

On effectue la déprotection du composé obtenu sous B dans l'exemple 48 selon le mode opératoire de l'exemple 1 (étape g).
Rendement = 79%
F = 125°C (microscope)
$RMN^1H$ ($CDCl_3$-$DMSOd_6$/TMS) : 7,65(m,1H) ; 7,45-6,65(m,4H) ; 6,3(m,1H) ; 3,90(m,2H) ; 3,2-2,3(m,5H) ; 1,75(t,J = 8,5Hz,1H).
$RMN^{13}C$ ($CDCl_3$-$DMSOd_6$, 400MHz) : 172,9 ; 171,1, ; 162,5(d,$J^1$= 244 Hz ) ; 141,2(d,$J^3$=7,3 Hz) ; 129,5 (d,$J^3$=7,3Hz) ; 124,4 ; 115,5(d,$J^2$=22Hz) ; 113,0(d,$J^2$=22Hz) ; 51,8 ; 40,9 ; 37,3; 25,8.
IR (nujol) : 3380, 1745, 1620 $cm^{-1}$

Microanalyse : ($C_{12}H_{14}FNO_3S$)

| | | | |
|---|---|---|---|
| Calc. % | C = 53,13 | H = 5,20 | N = 5,16 |
| Tr.% | C = 53,26 | H = 5,11 | N = 5,01 |

Exemple 50 : Préparation du N-(RS)-[oxo-1 (acétylthiométhyl)-2 (fluoro-3 phényl)-3 propyl]-(S)-alaninate de benzyle

On couple l'acide acétylthiométhyl-2 (fluoro-3 phényl)-3 propanoïque sous sa forme racémique avec le (S)-alaninate de benzyle selon le mode opératoire de l'exemple 1 (étape f).
Rendement = 90%
F = 52-55°C (microscope) (mélange 50/50 de diastéréoisomères)
$RMN^1H$ ($CDCl_3$,TMS) = 7,3(s,5H) ; 7,3-6,65(m,4H) ; 6,0(m,1H) ; 5,1(s,2H) ; 4,5(quintuplet,J = 7Hz,1H) ; 3,2-2,4(m,5H) ; 2,3(s,3H) ; 1,35(t,J = 7Hz,1,5H) ; 1,15(t,J = 7Hz,1,5H).
$RMN^{13}C$ ($CDCl_3$) = 195,5 ; 172,3 ; 171,8 ; 162,6(d,J = 244Hz ; 141,0 ; 135,0 ; 129,7(d,J = 9,7Hz) ; 128,4 ; 128,1 ; 127,9 ; 124,5 ; 115,6(d,J = 21Hz) ; 113,2 (d,J = 21Hz) ; 66,8 ; 50,5 ; 48,5 ; 47,9 ; 47,6 ; 37,8 ; 30,8 ; 30,2 ;

18,0 ; 17,8.
IR (nujol) : 3290, 1740, 1720, 1680, 1645 $cm^{-1}$.

Microanalyse : ($C_{22}H_{24}FNO_4S$)
Calc. % C = 63,29 H = 5,79 N = 3,35
Tr. % C = 63,52 H = 5,90 N = 3,40

Exemple 51 : Préparation de la N-(RS)-[oxo-1 (mercaptométhyl)-2 (fluoro-3 phényl)-3 propyl]-(S)-alanine

On effectue la déprotection du composé de l'exemple 50 selon le mode opératoire de l'exemple 1 (étape g).
Rendement = 71%
F = 120-122°C (microscope) (mélange 50/50 de diastéréoisomères)
RMN$^1$H (CDCl3, DMSOd6/TMS) : 7,55-6,4(m6H) ; 4,4(m,1H) ; 3,15-2,3(m,5H) ; 1,65(m,1H) ; 1,4(d,J = 8Hz,1,5H) ; 1,2(d,J = 8Hz, 1,5H).
IR (nujol) : 3300, 2580, 1715, 1640 $cm^{-1}$

Microanalyse : ($C_{13}H_{16}FNO_3S$)
Calc. % C = 54,72 H = 5,65 N = 4,91
Tr. % C = 54,57 H = 5,44 N = 4,82

Exemple 52 : Préparation du N-(RS)-[oxo-1 (acétylthiométhyl)-2 (difluoro-3,4 phényl)-3 propyl]-glycinate de benzyle

A. Préparation de l'acide -(RS)-acétylthiométhyl-2 (difluoro-3,4 phényl)-3 propanoïque

On l'obtient comme dans l'exemple 1 (étape d) par addition de l'acide thioacétique à l'acide [(difluoro-3,4 phényl)méthyl]-2 propénoïque.
Rendement = 98%
IR (film) : 1700, 1610 $cm^{-1}$
RMN$^1$H ($CDCl_3$/TMS) : 10,6(s,1H) ; 7,35-6,7(m,3H) ; 3,3-2,6(m,5H) ; 2,3(s,3H) ;

B. Préparation du N-(RS)-[oxo-1 (acétylthiométhyl)-2 (difluoro-3,4 phényl)-3 propyl]-glycinate de benzyle

On couple le composé obtenu sous A avec le glycinate de benzyle selon le mode opératoire de l'exemple 1 (étape f).
Rendement = 87%
F = 78°C (microscope)
RMN$^1$H ($CDCl_3$,TMS) : 7,3(s,5H) ; 7,25-6,75(m,3H) ; 6,0(m,1H) ; 5,15(s,2H) ; 4,0(m,2H) ; 3,2-2,55 (m,5H) ; 2,3(s,3H) ;
RMN$^{13}$C ($CDCl_3$) : 195,6 ; 172,6 ; 169,2 ; 149,6(dd,$J^1$ = 247Hz, $J^2$ = 12Hz) ; 148,5(dd, $J^1$ = 247Hz, $J^2$ = 12Hz) ; 135,4 ; 134,9 ; 128,4 ; 124,7 ; 117,5(d,$J^2$ = 17Hz) ; 116,9(d,$J^2$ = 17Hz) ; 66,8 ; 48,5 ; 40,9 ; 36,8 ; 30,9 ; 30,2.
IR (nujol) : 3300, 1740, 1680, 1640 $cm^{-1}$

Microanalyse : ($C_{21}H_{21}F_2NO_4S$)
Calc. % C = 59,85 H = 5,02 N = 3,32
Tr. % C = 59,53 H = 5,17 N = 3,50

Exemple 53 : Préparation de la N-(RS)-[oxo-1 (mercaptométhyl)-2 (difluoro-3,4 phényl)-3 propyl]-glycine

On effectue la déprotection du composé obtenu sous B dans l'exemple 52 selon le mode opératoire de l'exemple 1 (étape g).

Rendement = 82%
F = 139°C (microscope)
RMN[1]H ($CDCl_3$-DMSO$d_6$/TMS) : 9,0(s,1H) ; 7,2-6,6(m,3H) ; 3,9(d,J = 6Hz,2H) ; 3,1-2,2(m,5H) ; 1,75(t,J = 8,5Hz,1H) ;
RMN[13]C ($CDCl_3$/DMSO$d_6$,400MHz) : 172,6 ; 171,0; 149,6(dd,$J^1$ = 247Hz, $J^2$= 12Hz) ; 148,5(dd,$J^1$ = 247Hz, $J^2$ = 12Hz) ; 135,5 ; 124,6 ; 117,3(d,$J^2$ = 16Hz) ; 116,6(d,$J^2$ = 16Hz) ; 51,7 ; 40,7 ; 36,6 ; 25,8.
IR (nujol) : 3300, 1720, 1640 $cm^{-1}$

Microanalyse : ($C_{12}H_{13}F_2NO_3S$)

| | | | |
|---|---|---|---|
| Calc. % | C = 49,82 | H = 4,53 | N = 4,84 |
| Tr. % | C = 49,70 | H = 4,34 | N = 4,63 |

Exemple 54 : Préparation du N-(RS)-[oxo-1 (acétylthiométhyl)-2 (difluoro-3,4 phényl)3 propyl]-(S)-alaninate de benzyle

On couple l'acide acétylthiométhyl-2 (difluoro-3,4 phényl)-3 propanoïque sous sa forme racémique avec le S-alaninate de benzyle selon le mode opératoire de l'exemple 1 (étape f).
Rendement = 93%
F = 76-79°C (microscope) (mélange 50/50 de diastéréoisomères)
RMN[1]H ($CDCl_3$, TMS) : 7,3(s,5H) ; 7,2-6,7(m,3H) ; 6,1(d,J = 7Hz,1H) ; 5,1(s,2H) ; 4,5 (quintuplet,J = 7Hz,1H) ; 3,1-2,4(m,5H) ; 2,3(s,3H) ; 1,3(d,J = 7Hz,1,5H) ; 1,15(d,J = 7Hz,1,5H) ;
RMN[13]C ($CDCl_3$) : 195,8 ; 172,3 ; 171,8 ; 149,8(dd,$J^1$ = 247Hz, $J^2$ = 17Hz) ; 148,6(dd,$J^1$ = 247Hz, $J^2$ = 17Hz) ; 135,6 ; 135,2 ; 128,5 ; 128,0 ; 124,9 ; 117,5(d,$J^2$ = 17Hz) ; 116,9(d,$J^2$ = 17Hz) ; 66,9 ; 49,1 ; 48,7 ; 47,9 ; 37,1 ; 31,0 ; 30,3 ; 18,1.
IR (nujol) : 3295, 1740, 1680, 1640 $cm^{-1}$

Microanalyse : ($C_{22}H_{23}F_2NO_4S$)

| | | | |
|---|---|---|---|
| Calc. % | C = 60,68 | H = 5,32 | N = 3,22 |
| Tr. % | C = 61,02 | H = 5,23 | N = 3,27 |

Exemple 55 : Préparation de la N-(RS)-[oxo-1 (mercaptométhyl)-2 (difluoro-3,4 phényl)-3 propyl]-(S)-alanine

On effectue la déprotection du composé de l'exemple 54 selon le mode opératoire de l'exemple 1 (étape g).
Rendement = 72%
F = 103-107°C (microscope) (mélange 50/50 de diastéréoisomères)
RMN[1]H ($CDCl_3$, DMSO$d_6$,TMS) : 7,8(s,1H) ; 7,5-6,65(m,3H) ; 4,45(quintuplet,J = 8Hz,1H) ; 3,1-2,2(m,5H) ; 1,65(m,1H) ; 1,35(d,J = 8Hz,1,5H) ; 1,2(d,J = 8Hz,1,5H) ;
IR (nujol) : 3300,2560, 1720, 1645 $cm^{-1}$

Microanalyse : ($C_{13}H_{15}F_2NO_3S$)

| | | | |
|---|---|---|---|
| Calc. % | C = 51,47 | H = 4,98 | N = 4,62 |
| Tr. % | C = 51,33 | H = 4,87 | N = 4,46 |

Exemple 56 : Préparation du N-(RS)-[oxo(acétylthiométhyl)-2 (difluoro-3,5 phényl)-3 propyl]-glycinate de benzyle

A. Préparation de l'acide -(RS)-acétylthiométhyl-2 (difluoro-3,5 phényl)-3 propanoïque

On l'obtient comme dans l'exemple 1 (étape d), par addition de l'acide thioacétique à l'acide [(difluoro-3,5 phényl)méthyl]-2 propénoïque.

Rendement = 98%
IR (film) : 1700, 1620, 1590 $cm^{-1}$
RMN$^1$H ($CCl_4$) : 11,1(s,1H) ; 6,9-6,35 (m,3H) 3,2-2,7(m,5H) ; 2,3(s,3H)

B. Préparation du N-(RS)-[oxo-1 (acétylthiométhyl)-2 (difluoro-3,5 phényl)-3 propyl]-glycinate de benzyle

On couple le composé obtenu sous A avec le glycinate de benzyle selon le mode opératoire de l'exemple 1 (étape f).
Rendement = 89%
F = 67°C (microscope)
RMN$^1$H ($CCl_4$) : 7,25 (s,5H) ; 6,85-6,40(m,3H) ; 6,2(m,1H) ; 5,05(s,2H) ; 3,85(m,2H) ; 3,2-2,4(m,5H) ; 2,15(s,3H) ;
RMN$^{13}$C ($CDCl_3$) : 195,7 ; 172,4 ; 169,3 ; 162,6(dd,$J^1$= 249Hz,$J^3$=12Hz) ; 142,5 ; 135,1 ; 128,5 ; 128,3 ; 111,1(d,$J^2$=24Hz) ; 102,0(t,$J^2$=24Hz) ; 67,1 ; 48,5 ; 41,1 ; 37,5 ; 31,1 ; 30,3.
IR (nujol) : 3300, 1750, 1690, 1650, 1620, 1595 $cm^{-1}$

Microanalyse : ($C_{21}H_{21}F_2NO_4S$)
Calc. % C = 59,85 H = 5,02 N = 3,32
Tr % C = 60,0 H = 5,14 N = 3,33

Exemple 57 : Préparation de la N-(RS)-[oxo-1 (mercaptométhyl)-2 (difluoro-3,5 phényl)-3 propyl]-glycine

On effectue la déprotection du composé obtenu sous B dans l'exemple 56 selon le mode opératoire de l'exemple 1 (étape g).
Rendement = 69%
F = 85°C (microscope)
RMN$^1$H ($CDCl_3$,$DMSOd_6$,TMS) : 7,3(s,1H) ; 6,9-6,35(m,3H) ; 3,9(d,J = 5Hz,2H) ; 3,15-2,2(m,5H) ; 1,75(t,J = 8,5Hz,1H). RMN$^{13}$C($CDCl_3$,$DMSOd_6$, 400MHz) : 172,5 ; 171,0 ; 162,4 (dd,$J^1$=248,3Hz, $J_3$=12,5Hz) ; 142,6 ; 111,4 (d,$J^2$=24Hz) ; 101,4(t,$J^2$=24Hz) ; 51,4 ; 40,7 ; 37,2 ; 26,0.
IR (nujol) : 3290, 1720, 1640, 1620, 1595 $cm^{-1}$

Microanalyse : ($C_{12}H_{13}F_2NO_3S$)
Calc. % C = 49,82 H = 4,53 N = 4,84
Tr. % C = 50,00 H = 4,54 N = 4,71

Exemple 58 : Préparation du N-(RS)-[oxo-1 (acétylthiométhyl)-2 (difluoro-3,5 phényl)-3 propyl]-(S)-alaninate de benzyle

On couple l'acide acétylthioréthyl-2 (difluoro-3,5 phényl)-3 propanoïque sous sa forme racémique avec le (S)-alaninate de benzyle selon le mode opératoire de l'exemple 1 (étape f).
Rendement = 96%
F = 64-73°C (microscope) (mélange 50/50 de diastéréoisomères)
RMN$^1$H ($CDCl_3$/TMS) : 7,25 (s,5H) ; 6,95-6,30(m,4H) ; 5,1(s,2H) ; 4,45(quintuplet,J = 7Hz,1H) ; 3,2-2,4(m,5H) ; 2,3(s,3H) ; 1,3(d,J = 7Hz,1,5H) ; 1,1(d,J = 7Hz,1,5H) ;
RMN$^{13}$C ($CDCl_3$) : 195,8 ; 172,3 ; 171,5 ; 162,7(dd,$J^1$=247Hz, $J^3$=12Hz) ; 142,8 ; 135 ; 128,4 ; 127,9 ; 111,6(d,$J^2$=24Hz) ; 101,8(t,$J^2$=24Hz) ; 66,8 ; 48,6 ; 47,7 ; 37,5 ; 31,0 ; 30,3 ; 18.
IR (nujol) : 3300, 1740, 1670, 1640, 1620, 1590 $cm^{-1}$

Microanalyse : ($C_{22}H_{23}F_2NO_4S$)
Calc. % C = 60,68 H = 5,32 N = 3,22
Tr. % C = 60,93 H = 5,26 N = 3,29

Exemple 59 : Préparation de la N-(RS)-[oxo-1(mercaptométhyl)-2 (difluoro-3,5 phényl)-3 propyl]-(S)-alanine

On effectue la déprotection du composé de l'exemple 58 selon le mode opératoire de l'exemple 1 (étape g).
Rendement = 62%
F = 115-118°C (microscope) (mélange 50/50 de diastéréoisomères)
RMN$^1$H ($CDCl_3$, $DMSOd_6$/TMS) : 8,15(s,1H) ; 7,15-6,4(m,3H) ; 4,45(quintuplet, J = 7Hz, 1H) ; 3,1-2,25(m,5H) ; 1,65(m,1H) ; 1,4(d,J = 7Hz,1,5H) ; 1,25(d,J = 7Hz,1,5H) ;
RMN$^{13}$C ($CDCl_3$,$DMSOd_6$) : 174,5 ; 171,9 ; 163,2(dd,$J^1$= 249Hz, $J^3$=12Hz) ; 142,6 ; 111,6(d,$J^2$=24,4Hz) ; 101,9 (t,$J^2$=24,4Hz) ; 52,6 ; 52,1 ; 47,8 ; 37,4 ; 26,3 ; 26,1 ; 18,15 ; 17,8.
IR (nujol) : 3300, 1715, 1640, 1620, 1595 $cm^{-1}$

Microanalyse : ($C_{13}H_{15}F_2NO_3S$)
Calc. % C = 51,47 H = 4,98 N = 4,62
Tr. % C = 50,98 H = 4,87 N = 4,43

Exemple 60 : Préparation du N-(S)-[oxo-1 (acétylthiométhyl)-2 (difluoro-3,5 phényl)-3 propyl]-(S)-alaninate de benzyle(isomère (-))

Il est obtenu par recristallisation fractionnée du mélange de diastéréoisomères de l'exemple 58 dans un mélange éther/éther de pétrole.
$[\alpha]_D^{25}$ = -58,3° (c = 1,2 ; MeOH)
F = 113°C (microscope)
RMN$^1$H ($CDCl_3$/TMS) : 7,25(s,5H) ; 6,95-6,4(m,3H) ; 6,2(m,1H) ; 5,1(s,2H) ; 4,45(quintuplet, J = 7Hz,1H) ; 3,2-2,4(m,5H) ; 2,3(s,3H) ; 1,35(d,J = 7Hz,3H).
IR (nujol) : 3300,1735, 1670, 1640, 1590 $cm^{-1}$

Exemple 61 : Préparation du N-(RS)-[oxo-1 (acétylthiométhyl)-2 (indanyl-5')-3 propyl]-glycinate de benzyle

A. Préparation de l'acide -(RS)-acétylthiométhyl-2 (indanyl-5')-3 propénoïque

On l'obtient comme dans l'exemple 1 (étape d) par addition de l'acide thioacétique à l'acide [(indanyl 5')méthyl]-2 propénoïque.
Rendement = 96%
IR ($cm^{-1}$) : 1700
RMN$^1$H ($CCl_4$/TMS) : 11,2 (s,1H) ; 7,2-6,8(m,3H) ; 3,2-2,4(m,9H) ; 2,2(s,3H) ; 2,2-1,8(m,2H)

B. Préparation du N-(RS)-[oxo-1 (acétylthiométhyl)-2(indanyl-5')-3 propyl] glycinate de benzyle

On couple le composé obtenu sous A avec le glycinate de benzyle selon le mode opératoire de l'exemple 1 (étape f).
Rendement = 67%
F = 71-72°C
IR (nujol) : 3300, 1740, 1690, 1650 $cm^{-1}$
RMN$^1$H ($CCl_4$/TMS) : 7,2(s,5H) ; 7,1-6,8(m,4H) ; 5(s,2H) ; 3,9-3,7(m,2H) ; 3,1-2,5(m,9H) ; 2,1(s,3H) ; 2,1-1,8(m,2H)

Microanalyse : $C_{24}H_{27}NO_4S$
Calc. % C = 67,73 H = 6,39 N = 3,79
Tr. % C = 68,02 H = 6,44 N = 3,50

Exemple 62 : Préparation de la N-(RS)-[oxo-1(mercaptométhyl)-2 (indanyl-5')-3 propyl]glycine

On effectue la déprotection du composé obtenu sous B dans l'exemple 61 selon le mode opératoire de l'exemple 1 (etape g).
Rendement = 68%
F = 90°C
IR (nujol) : 3380, 1740, 1620 $cm^{-1}$
RMN$^1$H($CDCl_3$,/TMS) : 8(s,1H) ; 7,2-6,8(m,3H) ; 6,4-6,2(m,1H) ; 4,1-3,8(m,2H) ; 3,0-2,4(m,9H) ; 2,25-1,8(m,2H) ; 1,6(t,1H,J = 7,8Hz).
RMN$^{13}$C ($CDCl_3$) : 174,6 ; 172,6 ; 144,6 ; 142,5 ; 135,9 ; 126,5 ; 124,7 ; 124,2 ; 53,1 ; 41,2 ; 37,8 ; 32,6 ; 32,2 ; 25,8 ; 25,2.

Microanalyse : $C_{15}H_{19}NO_3S$

| | | | |
|---|---|---|---|
| Calc. % | C = 61,4 | H = 6,52 | N = 4,77 |
| Tr. % | C = 61,35 | H = 6,50 | N = 4,82 |

Exemple 63 : Préparation du N-(RS)-[oxo-1 (acétylthiométhyl)-2(indanyl-5')-3 propyl]-(S)-alaninate de benzyle

On couple l'acide obtenu sous A dans l'exemple 61 avec le (S)-alaninate de benzyle selon le mode opératoire de l'exemple 1 (étape f).
Rendement = 67%
IR : 3300, 1740, 1680, 1650 $cm^{-1}$
RMN$^1$H($CDCl_3$/TMS) : 7,2(s,5H) ; 7,1-6,8(m,3H) ; 6,5 et 6,2(2d, 1H, J = 6,6Hz) ; 5(s,2H) ; 4,7-4,3(m,1H) ; 3,2-2,5(m,9H) ; 2,2(s,3H) ; 2,1-1,8(m,2H) ; 1,3 et 1,1 (2d,3H,J = 6,7Hz) ;
RMN$^{13}$C ($CDCl_3$) : 195,8 ; 172,2 ; 144,3 ; 142,2 ; 136,1 ; 135,2 ; 128,4 ; 128,1 ; 127,8 ; 126,4 ; 124,7 ; 124 ; 66,7 ; 49,3 ; 48,7 ; 47,9 ; 47,5 ; 38,2 ; 37,8 ; 32,3 ; 32,1 ; 30,9 ; 30,6 ; 30,2 ; 25,2 ; 18 ; 17,8.

Microanalyse : $C_{25}H_{29}NO_4S$

| | | | |
|---|---|---|---|
| Calc. % | C = 68,3 | H = 6,65 | N = 3,18 |
| Tr. % | C = 68,1 | H = 6,60 | N = 3,05 |

Exemple 64 : Préparation de la N-(RS)-[oxo-1 (mercaptométhyl)-2(indanyl-5')-3 propyl]-(S)-alanine

On effectue la déprotection du composé de l'exemple 63 selon le mode opératoire de l'exemple 1 (étape g).
Rendement = 86%
F = 40-45°C
IR (nujol) : 3440, 1720, 1660 $cm^{-1}$
RMN$^1$H ($CDCl_3$/TMS) : 8,4(s,1H) ; 7,2-6,8(m,3H) ; 6,3-5,8(m,1H) ; 4,6-4,4(m,1H) ; 3,0-2,3(m,9H) ; 2,2-1,8(m,2H) ; 1,6(t,1H,J = 9Hz) ; 1,45 et 1,2(2d,3H,J = 7,3Hz)
RMN$^{13}$C ($CDCl_3$) : 175,6 ; 173,8 ; 144,5 ; 142,5 ; 135,9 ; 126,55 ; 124,9 ; 124,25 ; 53,5 ; 52,9 ; 48,1 ; 47,9 ; 38,1 ; 37,9 ; 32,5 ; 32,2 ; 25,9 ; 25,3 ; 17,9 ; 17,55.

Microanalyse : $C_{16}H_{21}NO_3S$

| | | | |
|---|---|---|---|
| Calc. % | C = 62,51 | H = 6,88 | N = 4,55 |
| Tr. % | C = 62,30 | H = 6,81 | N = 4,38 |

Exemple 65 : Préparation du N-(RS)-[oxo-1 (acétylthiométhyl)-2 (dihydro-2′, 3′ benzofuranyl-5′)-3 propyl]-glycinate de benzyle

A. Préparation de l'acide -(RS)-acétylthiométhyl-2 (dihydro 2′,3′ benzofuranyl 5′)-3 propanoïque

On l'obtient comme dans l'exemple 1 (etape d), par addition de l'acide thioacétique à l'acide [(dihydro-2′,3′ benzofuranyl-5′)méthyl]-2 propénoïque.
Rendement = 98%
IR ($cm^{-1}$) : 1700
RMN[1]H ($CDCl_3$/TMS) : 10,2(s,1H) ; 7,1-6,8(m,2H) ; 6,65(d,1H,J = 8,1Hz) ; 4,5(t,2H,J = 8Hz) ; 3,3-2,4(m,7H) ; 2,2(s,3H) .

Préparation du N- (RS)-[oxo-1 (acétylthiométhyl)-2(dihydro 2′,3′ benzofuranyl-5′)-3 propyl]glycinate de benzyle

On couple le composé obtenu sous A avec le glycinate de benzyle selon le mode opératoire de l'exemple 1 (étape f).
Rendement = 85%
F = 85°C
IR (nujol) : 3300, 1720, 1680, 1635 $cm^{-1}$
RMN[1]H ($CDCl_3$/TMS) : 7,2(s,5H) ; 7-6,75(m,2H) ; 6,65(d,1H,J = 8Hz) ; 6-5,8(m,1H) ; 5,1(s,2H) ; 4,5(t,2H,J = 8,8Hz) ; 3,9(t,2H,J = 5Hz) ; 3,3-2,4(m,7H) ; 2,2(s,3H).

Microanalyse : $C_{23}H_{25}NO_5S$

| | | | |
|---|---|---|---|
| Calc. % | C = 64,61 | H = 5,89 | N = 3,27 |
| Tr. % | C = 64,60 | H = 5,87 | N = 3,36 |

Exemple 66 : Préparation de la N-(RS)-[oxo-1 (mercaptométhyl)-2(dihydro 2′,3′ benzofuranyl-5′)-3 propyl]glycine

On effectue la déprotection du composé obtenu sous B dans l'exemple 65 selon le mode opératoire de l'exemple 1 (étape g).
Rendement = 70%
F = 121°C
IR (nujol) : 3380, 1740, 1610 $cm^{-1}$
RMN[1]H ($CDCl_3$/TMS) : 9,4(s,1H) ; 7,05-6,8(m,2H) ; 6,65(d,1H,J = 8Hz) ; 6,65-6,4(m,1H) ; 4,5(t,2H,J = 8Hz) ; 3,9(t,2H, J = 5Hz) ; 3,3-2,4(m,7H) ; 1,6(t,1H,J = 8Hz).

Microanalyse : $C_{14}H_{17}NO_4S$

| | | | |
|---|---|---|---|
| Calc. % | C = 56,92 | H = 5,80 | N = 4,74 |
| Tr. % | C = 56,84 | H = 5,70 | N = 4,51 |

Exemple 67 : Préparation du N-(RS)-[oxo-1 (acétylthiométhyl)-2(dihydro-2′,3′ benzofuranyl-5′)-3 propyl]-(S)-alaninate de benzyle

On couple le composé obtenu sous A dans l'exemple 65 avec le (S)-alaninate de benzyle selon le mode opératoire de l'exemple 1 (étape f).
Rendement = 66%
F = 63-67°C
IR (nujol) : 3280, 1720, 1680, 1640 $cm^{-1}$
RMN[1]H($CDCl_3$/TMS) : 7,2(s,5H) ; 7 à 6,7(m,2H) ; 6,6(d,1H, J = 8Hz) ; 6,2-5,7(m,1H) ; 5(s,2H) ; 4,6-4,3(m,3H) ; 3,3-2,4(m,7H) ; 2,2(s,3H) ; 1,3 et 1,1(2d, 3H, J = 8Hz).

Microanalyse : $C_{24}H_{27}NO_5S$

Calc. % C = 65,28 H = 6,16 N = 3,17

Tr. % C = 64,97 H = 6,27 N = 3,19

Exemple 68 : Préparation de la N-(RS)-[oxo-1 (mercaptométhyl)-2(dihydro-2′ 3′ benzofuranyl-5′)-3 propyl]-(S)-alanine

On effectue la déprotection du composé de l'exemple 67 selon le mode opératoire de l'exemple 1 (étape g).
Rendement = 81%
F = 40-45°C
IR ($CDCl_3$) : 3420, 1720, 1640 $cm^{-1}$
RMN$^1$H($CDCl_3$/TMS) : 8,8(s,1H) ; 7-6,75(m,1H) ; 6,65 (d,1H,J = 8Hz) ; 6,3(t, 1H,J = 8Hz) ; 4,7-4,3(m,1H) ; 4,5(t,2H,J = 8Hz) ; 3,3-2,4(m,7H) ; 1,6(t,1H,J = 9Hz) ; 1,4 et 1,2(2d,3H,J = 6,7Hz).
RMN$^{13}$C($CDCl_3$) : 175,6 ; 173,65 ; 158,9 ; 130,3 ; 128,3 ; 127,2 ; 125,35 ; 109,1 ; 70,95 ; 53,7 ; 53,2 ; 47,9 ; 37,6 ; 37,3 ; 29,4 ; 25,9 ; 25,7 ; 17,9 ; 17,5.

Microanalyse : $C_{15}H_{19}NO_4S$

Calc. % C = 58,23 H = 6,19 N = 4,52

Tr. % C = 58,08 H = 6,10 N = 4,44

Exemple 69 : Préparation du N-(RS)-[oxo-1(acétylthiométhyl)-2 (méthoxy-4 phényl)-3 propyl]-glycinate de benzyle

A. Préparation de l'acide (RS)-acétylthiométhyl-2 (méthoxy-4 phényl)-3 propanoïque

On l'obtient comme dans l'exemple 1 (étape d) par addition de l'acide thioacétique à l'acide [(méthoxy-4 phényl)-méthyl]-2 propénoïque.
Rendement = 84%
IR = 1740-1685 $cm^{-1}$
RMN$^1$H ($CDCl_3$/TMS) : 11,0(s,1H) ; 7,2 et 6,9 (AB, 4H, J = 8Hz) ; 3,8(s,3H) ; 3,3 à 2,8 (m,5H) ; 2,3S (s,3H).

B. Préparation du N-(RS)-[oxo-1(acétylthiométhyl)-2(méthoxy-4 phényl]-3 propyl]-glycinate de benzyle

On couple le composé obtenu sous A avec le glycinate de benzyle selon le mode opératoire de l'exemple 1 (étape f).
Rendement = 68% (chromatographié)
F = 62-64°C
IR (nujol) : 3300, 1735, 1690, 1650 $cm^{-1}$
RMN$^1$H ($CDCl_3$/TMS) : 7,4(s,5H) ; 7,15 et 6,8(AB,4H, J = 9,3) ; 6,3(t, large, 1H) ; 5,15(s,2H) ; 4,1 à 3,85(m,2H) ; 3,7(s,3H) ; 3,2 à 2,45(m,5H) ; 2,25(s,3H)
RMN$^{13}$C ($CDCl_3$) : 195,4(s) ; 173,0(s) ; 169,0(s) ; 157,9(s) ; 134,7(s) ; 130,1(s) ; 129,5(d) ; 128,1(d) ; 113,5(d) ; 66,6(t) ; 54,7(q) ; 48,6(d) ; 40,9(t) ; 37,0(t) ; 30,6(t) ; 30,2(q)

Microanalyse : $C_{22}H_{25}O_5NS$

Calc % C : 63,61 N : 3,37 H : 6,02

Tr. % C : 63,80 N : 3,53 H : 6,12

Exemple 70 : Préparation de la N-(RS)-[oxo-1(mercaptométhyl)-2(méthoxy-4 phényl)-3 propyl]-glycine

On effectue la déprotection du composé obtenu sous B dans l'exemple 69 selon le mode opératoire de l'exemple 1 (étape g).

Rendement = 85%
F = 122-123°C
IR (nujol) : 3390, 1750, 1620 $cm^{-1}$
$RMN^1H$ (DMSO $D_6$) : 8,0(m,1H) ; 6,8 et 6,6(AB,4H, J = 9,3) ; 3,4(m,5H) ; 2,6 à 1,8(m,6H)
$RMN^{13}C$ (DMSO $D_6$) : 174,3(s) ; 172,4(s) ; 158,8(s) ; 132,1(s) ; 131,0(d) ; 114,8(d) ; 56,1(q) ; 52,0(d) ; 41,7(t) ; 37,5(t) ; 26,6(t)

Microanalyse : $C_{13}H_{17}O_4NS$

| | | | |
|---|---|---|---|
| Calc. | % C : 55,12 | N : 4,94 | H : 6,00 |
| Tr. | % C : 54,78 | N : 4,85 | H : 5,95 |

Exemple 71 : Préparation de la N-(RS)-[oxo-1(acétylthiométhyl)-2(méthoxy-4 phényl)-3 propyl]-(S)alaninate de benzyle

On couple l'acide (RS)-(acétylthiométhyl)-2(méthoxy-4 phényl)-3 propanoïque avec le (S)-alaninate de benzyle selon le mode opératoire de l'exemple 1 (étape f).
Rendement = 50% (chromatographié)
F = 50-51°C
IR (nujol) : 3320, 1740, 1690 à 1645 $cm^{-1}$
$RMN^1H$ ($CDCl_3$/TMS) : 7,25(s,5H) ; 7,1 à 6,75(AB,4H,J = 8 Hz) ; 6,3 et 6,15 (2 doublets, J = 7,2 Hz) ; 5,05(s,2H) ; 4,5(quintuplet,1H,J = 7,2 Hz) ; 3,6(s,3H) ; 3,15 à 2,4(m,5H) ; 2,2(s,3H) ; 1,3 et 1,1(2 doublets,3H,J = 7,2 Hz).
$RMN^{13}C$ ($CDCl_3$) : 195,2(s) ; 172,0(s) ; 157,8(s) ; 135,0 (s) ; 130,2(s) ; 129,5(d) ; 128,1(d) ; 127,9(d) ; 127,6(d) ; 113,4(d) ; 66,5(t) ; 54,7(q) ; 48,9(d) ; 48,4(d) ; 47,7(d) ; 47,4(d) ; 37,3(t) ; 30,6(t) ; 30,1(q) ; 17,8(q).

Microanalyse : $C_{23}H_{27}O_5NS$

| | | | |
|---|---|---|---|
| Calc. | % C : 64,33 | N : 3,26 | H : 6,29 |
| Tr. | % C : 63,98 | N : 3,30 | H : 6,26 |

Exemple 72 : Préparation de la N-(RS)-[oxo-1(mercaptométhyl)-2(méthoxy-4phényl)-3propyl]-(S)-alanine

On effectue la déprotection du composé de l'exemple 71 selon le mode opératoire de l'exemple 1 (étape g).
Rendement = 83% (chromatographié)
IR : 3320, 1730, 1630 $cm^{-1}$
$RMN^1H$ ($CDCl_3$/TMS) : 9,7(s,1H) ; 7,05 et 6,75(AB,4H,J = 8 Hz) ; 6,55 et 6,4(2 doublets,1H,J = 7,6 Hz) ; 4,5(2 quintuplets, 1H,J = 7,6 Hz) ; 3,7(s,3H) ; 3,1 à 2,2(m,5H) ; 1,6(t,1H,J = 8 Hz) ; 1,4 et 1,2(2 doublets,3H,J = 7,6Hz).
$RMN^{13}C$ ($CDCl_3$) : 173,6(s) ; 158,1(s) ; 155,5(s) ; 130,3(s) ; 129,7(d) ; 113,8(d) ; 55,1(q) ; 53,5(d) ; 53,0(d) ; 48,1(d) ; 37,3(t) ; 37,0(t) ; 26,0(t) ; 25,7(t) ; 17,9(q) ; 17,6(q)

Microanalyse : $C_{14}H_{19}O_4NS$

| | | | |
|---|---|---|---|
| Calc. | % C : 56,56 | N : 4,71 | H : 6,39 |
| Tr. | % C : 56,21 | N : 4,57 | H : 6,22 |

Dédoublement de l'acide acétylthiométhyl-2 (méthoxy-4 phényl)-3 propanoïque

A une solution de 10 g d'acide acétylthiométhyl-2(méthoxy-4 phényl)-3 propanoïque en solution dans 60 ml d'éther, on ajoute 3,05 g de (-)éphédrine en solution dans 25 ml d'éther. On laisse au repos pendant sept jours et on filtre.
Masse obtenue = 6,1 g
F = 126°C
$[\alpha]_D^{25}$= -23,3°

Recristallisations

On place dans un ballon 6 g de sel (-) et on ajoute 25 ml de chloroforme et 35 ml d'éther. On laisse 15 heures et on filtre. On répète cette opération trois fois.
Masse obtenue = 4,35 g
Rendement global des quatre recristallisations = 73%
F = 122°C
$[\alpha]_D^{25}$= -40,7° (C = 1,2 ; MeOH)

Libération de l'acide (S) optiquement pur

On utilise un processus expérimental analogue à celui décrit dans l'exemple 2 (II).
Rendement = 98%
$[\alpha]_D^{25}$= -24,4° (C = 1,3 ; MeOH)

Exemple 73 : Préparation du N-(S)-[oxo-1(acétylthiométhyl)-2 (méthoxy-4 phényl)-3 propyl]-glycinate de benzyle

On couple l'acide acétylthiométhyl-2(méthoxy-4 phényl)-3 propanoïque sous sa forme (S) avec le glycinate de benzyle obtenu selon le mode opératoire de l'exemple 1 (étape f).
Rendement = 90% (chromatographié)
F = 84°C
$[\alpha]_D^{25}$= -14,7° (c = 1,3 dans le méthanol)

Microanalyse : $C_{22}H_{25}O_5NS$

| | | | |
|---|---|---|---|
| Calc. | % C : 63,61 | N : 3,37 | H : 6,02 |
| Tr. | % C : 63,43 | N : 3,50 | H : 6,05 |

Exemple 74 : Préparation de la N-(S)-[oxo-1(mercaptométhyl)-2(méthoxy-4 phényl]-3 propyl]-glycine

On effectue la déprotection du composé obtenu dans l'exemple 73 selon le mode opératoire de l'exemple 1 (étape g).
Rendement = 84% (chromatographié)
$[\alpha]_D^{25}$= + 50,5° (c = 1,05 dans le méthanol)

Microanalyse : $C_{13}H_{17}O_4NS$

| | | | |
|---|---|---|---|
| Calc. | % C : 55,12 | N : 4,94 | H : 6,00 |
| Tr. | % C : 55,04 | N : 4,76 | H : 6,13 |

Exemple 75 : Préparation de la N-(S)-[oxo-1(acétylthiométhyl)-2(méthoxy-4 phényl)-3 propyl]-(S)-alaninate de benzyle

On couple l'acide acétylthiométhyl-2(méthoxy-4 phényl)-3 propanoïque sous sa forme (S) avec le (S)-alaninate de benzyle selon le mode opératoire de l'exemple 1 (étape f).
Rendement = 56% (recristallisé dans un mélange chloroforme/éther de pétrole)
F = 74°C
$[\alpha]_D^{25}$ = -47,1° (c = 1,2 dans le méthanol)
IR (nujol) : 3310, 1730, 1680, 1640 $cm^{-1}$
RMN$^1$H ($CDCl_3$/TMS) : 7,3(s,5H) ; 7,05 et 6,75(AB,4H,J = 8Hz) ; 6,1(d,1H,J = 7,2Hz) ; 5,05(s,2H) ; 4,5(quintuplet,1H,J = 7,2Hz) ; 3,7(s,3H) ; 3,2 à 2,4(m,5H) ; 2,2(s,3H) ; 1,3(d,3H,J = 7,2Hz).
RMN$^{13}$C ($CDCl_3$) : 195,5(s) ; 172,1(s) ; 157,9(s) ; 135,0(s) ; 130,3(s) ; 129,6(d) ; 128,1(d) ; 127,7 (d) ; 113,5(d) ; 66,6(t) ; 54,8(q) ; 49,1(d) ; 47,5(d) ; 37,4(t) ; 30,8(t) ; 30,2(q) ; 17,8(q).

Microanalyse : $C_{23}H_{27}O_5$ NS

| | | | |
|---|---|---|---|
| Calc % | C : 64,33 | N : 3,26 | H : 6,29 |
| Tr % | C : 64,03 | N : 3,18 | H : 6,50 |

Exemple 76 : Préparation de la N-(S)-[oxo-1(mercaptométhyl)-2(méthoxy-4 phényl)-3 propyl]-(S)-alanine

On effectue la déprotection du composé obtenu dans l'exemple 75 selon le mode opératoire de l'exemple 1 (étape g).
Rendement = 83% (chromatographié)
$[\alpha]_D^{25}$ = -5,8° (c = 1,2 dans le méthanol)
IR : 3290 à 3390, 1730, 1645 $cm^{-1}$
RMN$^1$H ($CDCl_3$/TMS) : 9,85(s,1H) ; 7,05 et 6,8(AB,4H, J = 8Hz) ; 6,3(d,1H,J = 7,3Hz) ; 4,5 (quintuplet,1H,J = 7,3Hz) ; 3,7(s,3H) ; 3,15 à 2,25(m,5H} ; 1,7 à 1,15(m,1H) ; 1,4(d,3H,J = 7,3Hz).
RMN$^{13}$C ($CDCl_3$) : 176,1(s) ; 173,6(s) ; 158,2(s) ; 130,2(s) ; 129,7(d) ; 113,9(d) ; 55,1(q) ; 53,0(d) ; 48,2(d) ; 37,1(t) ; 25,7(t) ; 18,0(q).

Microanalyse : $C_{14}H_{19}O_4$NS

| | | | |
|---|---|---|---|
| Calc. % | C : 56,56 | N : 4,71 | H : 6,39 |
| Tr. % | C : 56,45 | N : 4,70 | H : 6,28 |

Exemple 77 : Préparation du N-(RS)-[oxo-1(acétylthiométhyl)-2(éthoxy-4 phényl)-3 propyl]-glycinate de benzyle

A. Préparation de l'acide (RS)-acétylthiométhyl-2 (éthoxy-4phényl)-3 propanoïque

On l'obtient comme dans l'exemple 1 (étape d), par adition de l'acide thioacétique à l'acide [(éthoxy-4 phényl)-méthyl]-2 propénoïque.
Rendement = 95%
RMN$^1$H ($CDCl_3$/TMS) : 9,6(s,1H) ; 7,15 et 6,8 (AB,4H, J = 9,3 Hz) ; 4,0(9,2H, J = 6,6Hz) ; 3,25 à 2,7(m,5H) ; 2,3(s,3H) ; 1,4(t,3H,J = 6,6Hz).

B. Préparation du N-(RS)-[oxo-1(acétylthiométhyl)-2 éthoxy-4 phényl)-3 propyl]-glycinate de benzyle

On couple le composé obtenu sous A avec le glycinate de benzyle selon le mode opératoire de l'exemple 1 (étape f).
Rendement = 82% (chromatographié)
F = 78°C
IR : 3300, 1730, 1690, 1640 $cm^{-1}$
RMN$^1$H ($CDCl_3$/TMS) : 7,3(s,5H) ; 7,05 et 6,75(AB,4H,J = 7,5Hz) ; 5,85(m,1H) ; 5,1(s,2H) ; 4,3 à 3,6(m,4H) ; 3,2 à 2,3(m,5H) ; 2,25(s,3H) ; 1,3(t,3H,J = 6,5Hz).

Exemple 78 : Préparation du N-(RS)-[oxo-1(mercaptométhyl)-2(éthoxy-4 phényl)-3 propyl]-glycine

On effectue la déprotection du composé obtenu dans l'exemple 77 selon le mode opératoire de l'exemple 1 (étape g).
Rendement = 82%
F = 124°C
IR : 3380, 2560, 1745, 1620 $cm^{-1}$
RMN$^1$H (DMSO/TMS) : 7,8(d,1H,J = 4,7Hz) ; 7,1 et 6,75(AB, 4H,J = 7,5Hz) ; 4,2 à 3,5(m,4H) ; 3,1 à 2,2(m,5H) ; 1,8(t,1H,J = 6,7Hz) ; 1,3(t,3H,J = 6,7Hz).

Exemple 79 : Préparation du N-(RS)-[oxo-1(acétylthiométhyl)-(éthoxy-4 phényl)-3 propyl]-(S)-alaninate de benzyle

On couple l'acide acétylthiométhyl-2(éthoxy-4 phényl)-3 propanoïque avec le (S)-alaninate de benzyle selon le mode opératoire de l'exemple 1 (étape f).
Rendement = 75% (chromatographié)
F = 52°C
IR : 3280, 1725, 1675, 1640 $cm^{-1}$
RMN$^1$H ($CDCl_3$/TMS) 7,15(s,5H), 7,05 et 6,75(AB, 4H,J = 7,5Hz) ; 6,2 à 5,8(m,1H) ; 5,1(s,2H) ; 4,5(quintuplet,1H, J = 7Hz) ; 3,95(q,2H,J = 6Hz) ; 3,2 à 2,3 (m,5H) ; 2,25(s,3H) ; 1,3(t,3H,J = 6Hz) ; 1,3 et 1,1(d,3H,J = 7Hz).

Exemple 80 : Préparation du N-(RS)-[oxo-1(mercaptométhyl)-2(éthoxy-4 phényl)-3 propyl]-(S)-alanine

On effectue la déprotection du composé obtenu dans l'exemple 79 selon le mode opératoire de l'exemple 1 (étape g).
Rendement = 52%
IR : 3300, 1720, 1635 $cm^{-1}$
RMN$^1$H ($CDCl_3$/TMS) : 10,4(s,1H) ; 7,05 et 6,75(AB,4H,J = 8Hz) ; 6,6 à 6,15(m,1H) ; 4,55(quintuplet, 1H,J = 6Hz) ; 3,9(q,2H,J = 6,7Hz) ; 3,1 à 2,2(m,5H) ; 1,6(t,1H,J = 7,5Hz) ; 1,6 à 1,0(m,6H).

Exemple 81 : Préparation du N-(E)-[oxo-1(acétylthiométhyl)-2 ène-2 phényl-3 propyl]-(S)-alaninate de benzyle

A. Préparation de l'acide (Z)-bromométhyl-2 cinnamique

On chauffe à reflux pendant 6 heures un mélange de 2 g (12,34 mmol) d'acide (E) méthyl-2 cinnamique, 2,19 g (12,34 mmol) de N-bromosuccinimide (NBS) et une quantité catalytique de peroxyde de benzoyle dans 6 ml de $CCl_4$. On filtre et on lave le résidu à l'éther. On lave la phase organique successivement par une solution aqueuse d'HCl 1N, puis par de l'eau. On sèche sur $MgSO_4$, on filtre et on évapore à sec.
Rendement = 57% (recristallisé dans l'éther)
F = 168°C
IR (nujol) : 1665 $cm^{-1}$
RMN$^1$H ($CDCl_3$/TMS) : 10,0(s,1H) ; 7,9(s,1H) ; 7,8 à 7,2(m,5H) ; 4,0(s,2H).

B. Préparation de l'acide (Z) acétylthiométhyl-2 cinnamique

On mélange 2,1 g de l'acide obtenu précédemment (8,70 mmol), 0,73 g (8,70 mmol) de $NaHCO_3$ et 3 ml d'eau. On ajoute à 0°C, une solution de 0,67 g (8,8 mmol) d'acide thioacétique et de 1,44 g (10,43 mmol) de $K_2CO_3$ dans 21 ml d'eau. On agite pendant 15 heures à 20°C. On acidifie par une solution aqueuse d'HCl 6N. On extrait deux fois à l'éther. Les phases éthérées réunies sont lavées à l'eau, séchées sur $MgSO_4$, filtrées et concentrées.
Rendement = 67% (recristallisé dans l'éther)
F = 114°C
IR (nujol) : 1670 $cm^{-1}$
RMN$^1$H ($CDCl_3$/TMS) : 9,55(s,1H) ; 8,0(s,1H) ; 7,5(s,5H) ; 4,1(s,2H) ; 2,3(s,3H).

C. Préparation de l'acide (E) acétylthiométhyl-2 cinnamique

On irradie pendant 16 heures, avec une lampe TQ 150 Hanovia, 2 g d'acide (Z) précédent en solution dans 30 ml d'éthanol. Après évaporation, on obtient un mélange d'acide Z/E 60/40. Ce mélange est repris par 25 ml d'éther et on ajoute une solution de 0,39 g de cyclohexylamine (0,4 éq.) dans 5 ml d'éther. Après 30 minutes sous agitation, on filtre. Le sel récupéré est traité par une solution aqueuse d'HCl 3N. On extrait à l'éther. La phase organique est lavée par une solution aqueuse saturée de NaCl, séchée sur $MgSO_4$ filtrée et concentrée.
Rendement = 32%
F = 57°C
RMN$^1$H ($CDCl_3$/TMS) : 9,6(s,1H) ; 7,3(s,5H) ; 7,2(s,1H) ; 3,85(s,2H) ; 2,25(s,3H).

D. Préparation du N-(E)-[oxo-1 (acétylthiométhyl)-2 ène-2 phényl-3 propyl]-(S)-alaninate de benzyle

On couple l'acide (E) acétylthiométhyl-2 cinnamique obtenu sous C avec le (S)-alaninate de benzyle selon le mode opératoire décrit à l'exemple 1 (étape f).
Rendement = 83% (chromatographié)
F = 103°C
$[\alpha]_D^{20}$ = -25,2° (c = 1,8 ; $CHCl_3$).
IR (nujol) : 3280, 1730, 1690, 1630 $cm^{-1}$
RMN$^1$H ($CDCl_3$/TMS) : 7,3(s,5H) ; 7,25(s,5H) ; 6,9(s,1H) ; 6,05(d,1H,J = 6,6Hz) ; 4,6(m,1H) ; 3,85(s,2H) ; 2,3(s,2H) ; 1,2(d,3H,J = 6,6Hz).

Microanalyse : $C_{22}H_{23}O_4NS$

| | | | |
|---|---|---|---|
| Calc. % | C = 66,47 | H = 5,83 | N = 3,52 |
| Tr. % | C = 66,36 | H = 5,68 | N = 3,53 |

Exemple 82 : Préparation de la N-(E)-[oxo-1 (mercaptométhyl)-2 ène-2 phényl-3 propyl]-(S)-alanine

A une solution de 0,85 mmol du diester obtenu sous D dans l'exemple 81 en solution dans un mélange THF-$H_2O$ (75-25), on ajoute à 0°C, sous atmosphère d'argon, 3,4 mmol de LiOH (4 éq.) et on agite 2 heures. On évapore le THF, on lave la phase aqueuse à l'éther et on acidifie avec une solution aqueuse d'HCl 3N. On extrait à l'éther, on lave avec une solution aqueuse saturée de NaCl, on sèche sur $MgSO_4$, on filtre et on évapore.
Rendement = 60% (chromatographié)
F = 78°C
IR (nujol) : 3300, 1720, 1650, 1610 $cm^{-1}$
RMN$^1$H ($CDCl_3$/TMS) : 9,15(s,1H) ; 7,25(s,5H) ; 6,70(s,1H) ; 6,25(d,1H, J = 6,6Hz) ; 4,55(m,1H) ; 3,5(d,2H,J = 8Hz) ; 1,8(t,1H,J = 8Hz) ; 1,25(d,3H,J = 6,6Hz).

Microanalyse : $C_{13}H_{15}O_3NS$

| | | | |
|---|---|---|---|
| Calc. % | C = 58,84 | H = 5,69 | N = 5,28 |
| Tr. % | C = 58,86 | H = 5,83 | N = 5,14 |

Exemple 83 : Préparation du N-(E)-[oxo-1(acétylthiométhyl)-2 ène-2 phényl-3 propyl]-(S)-norvalinate de benzyle

On couple l'acide (E) acétylthiométhyl-2 cinnamique obtenu sous C dans l'exemple 81 avec le (S)-norvalinate de benzyle selon le mode opératoire décrit à l'exemple 1 (étape f).
Rendement = 83% (chromatographié)
F = 80° C
$[\alpha]_D^{20}$= -26,4° (c = 1,53 ; $CHCl_3$)
IR (nujol) : 3300, 1720, 1680, 1640, 1620 $cm^{-1}$
RMNH ($CDCl_3$/TMS) : 7,3(s,5H) ; 7,25(s,5H) ; 6,85(s,1H) ; 6(d,1H,J = 7,6Hz) ; 5,1(s,2H) ; 4,8 à 4,4(m,1H) ; 3,85(s,2H) ; 2,3(s,3H) ; 1,85 à 0,6(m,7H).

Microanalyse : $C_{24}H_{27}O_4NS$

| | | | |
|---|---|---|---|
| Calc. % | C = 67,73 | H = 6,39 | N = 3,29 |
| Tr. % | C = 67,71 | H = 6,35 | N = 3,38 |

Exemple 84 : Préparation du N-(E)-[oxo-1 (acétylthiométhyl)-2 ène-2 phenyl-3 propyl]-(S)-norleucinate de benzyle.

On couple l'acide (E) acétylthiométhyl-2 cinnamique (exemple 81 C) avec le (S)-norleucinate de benzyle selon le mode opératoire décrit à l'exemple 1 (étape f).
Rendement = 79%
F = 79°C
$[\alpha]_D^{20}$ = 22,1° (c = 1,5 ; $CHCl_3$)
IR (nujol) : 3280, 1720, 1670, 1640, 1620 $cm^{-1}$
RMN$^1$H ($CDCl_3$/TMS) : 7,3(s,5H) ; 7,25(s,5H) ; 6,85(s,1H) ; 6,0(d,1H,J = 7,4Hz) ; 5,1(s,2H) ; 4,8 à 4,45(m,1H) ; 3,85(s,2H) ; 2,3(s,3H) ; 1,8 à 0,6(m,9H).

Microanalyse : $C_{25}H_{29}O_4NS$

| | | | | |
|---|---|---|---|---|
| Calc. | % | C = 68,30 | H = 6,65 | N = 3,18 |
| Tr. | % | C = 67,67 | H = 6,47 | N = 3,43 |

Exemple 85 : Préparation du N-(E)-[oxo-1 (acétylthiométhyl)-2 ène-2 phényl-3 propyl]-(RS)-(méthyldioxy-3,4 phényl)-3 alaninate de méthyle

On couple l'acide (E)acétylthiométhyl-2 cinnamique (exemple 81 C) avec le (RS)-(méthylènedioxy-3,4 phényl)-3 alaninate de méthyle selon le mode opératoire décrit à l'exemple 1 (étape f).
Rendement = 48%
F = 75°C
IR (nujol) : 3250, 1740, 1690, 1640 $cm^{-1}$

Microanalyse : $C_{23}H_{25}O_6NS$

| | | | | |
|---|---|---|---|---|
| Calc. | % | C = 62,57 | H = 5,25 | N = 3,17 |
| Tr. | % | C = 62,28 | H = 5,07 | N = 3,44 |

RMN$^1$H ($CDCl_3$/TMS) : 7,25(s,5H) ; 6,8(s,1H) ; 6,75 à 6,15(m,3H) ; 6,15 à 5,8(m,1H) ; 5,85(s,2H) ; 5,0 à 4,6(m,1H) ; 3,85(s,2H) ; 3,6(s,3H) ; 2,9(d,2H,J = 7,6Hz) ; 2,3(s,3H).

Exemple 86 : Préparation de la N-(E)-[oxo-1 (mercaptométhyl)-2 ène-2 phényl-3 propyl]-(R,S)-(méthylènedioxy-3,4 phényl)-3 alanine

On l'obtient par déprotection du composé de l'exemple 85 suivant le mode opératoire de l'exemple 82.
Rendement = 75%
F = 50°C
IR (nujol) : 3400, 1720, 1620 à 1600 $cm^{-1}$
RMN$^1$H ($CDCl_3$/TMS) : 8,3(m,1H) ; 7,25(s,5H) ; 6,7(s,1H) ; 6,55 à 6,0(m,4H) ; 5,85(s,2H) ; 5,0 à 4,65(m,1H) ; 3,45(d,2H,J = 8,2Hz) ; 2,9(d,2H,J = 5,1Hz) ; 1,75(t,1H,J = 8,2Hz).

Microanalyse : $C_{20}H_{19}O_5NS$

| | | | | |
|---|---|---|---|---|
| Calc. | % | C = 62,32 | H = 4,96 | N = 3,63 |
| Tr. | % | C = 62,18 | H = 5,06 | N = 3,39 |

Exemple 87 : Préparation du N-(Z)-[oxo-1(acétylthiométhyl)-2ène-2(méthylènedioxy-3,4 phényl)-3 propyl]-glycinate de benzyle

A. Préparation de l'acide (Z) bromométhyl-2(méthylènedioxy-3,4 phényl)-3 propénoïque

L'acide (E) méthyl-2(méthylènedioxy-3,4 phényl)-3 propénoïque (préparé par réaction de PERKIN à partir

du pipéronal) est substitué par le N-bromosuccinimide dans le chloroforme selon le mode opératoire décrit à l'exemple 81A.
Rendement = 75%
F = 158°C
RMN[1]H ($CDCl_3$/TMS) : 8,6(s,1H) ; 7,7(s,1H) ; 7,3 à 6,7(m,3H) ; 6,0(s,2H) ; 4,35(s,2H).

B. Préparation de l'acide (Z) acétylthiométhyl-2 (méthylènedioxy-3,4 phényl)-3 propénoïque

A une solution de 4,47 g d'acide préparé à l'étape A en solution dans 80 ml de THF, on ajoute à 0°C une solution de 1,26 g d'acide thioacétique (1,05 éq) et de 2,12 g de diisopropyléthylamine (1,05 éq) dans 80 ml de THF. On agite 30 minutes à 0°C. On évapore le THF, on reprend le résidu avec 100 ml d'éther. On lave par une solution aqueuse d'HCl 1N. La phase organique est séchée sur $MgSO_4$, filtrée et concentrée.
Masse obtenue = 4 g
Rendement = 86%
F = 142°C
RMN[1]H ($CDCl_3$/TMS) : 8,6(s,1H) ; 7,8(s,1H) ; 7,1 à 6,7(m,3H) ; 6,0(s,2H) ; 4,05(s,2H) ; 2,3(s,3H).

C. Préparation du N-(Z)-[oxo-1(acétylthiométhyl)-2ène-2(méthylènedioxy-3,4 phényl)-3 propyl]-glycinate de benzyle

On couple l'acide (Z) acétylthiométhyl-2(méthylènedioxy-3,4 phényl)-3 propénoïque obtenu sous B avec le glycinate de benzyle selon le mode opératoire décrit à l'exemple 1 (étape f).
Rendement = 55%
F = 102°C
RMN[1]H ($CDCl_3$/TMS) : 7,5(s,1H) ; 7,3(s,5H) ; 7,0 à 6,7(m,3H) ; 5,95(s,2H) ; 5,15(s,2H) ; 4,15(d,2H,J = 5,2Hz) ; 4,0(s,2H) ; 2,3(s,3H).

Exemple 88 : Préparation du N-(Z)-[oxo-1(mercaptométhyl)-2ène-2(méthylènedioxy-3,4 phényl)-3 propyl]-glycine

On effectue la déprotection du composé obtenu dans l'exemple 87 selon le mode opératoire décrit à l'exemple 82.
Rendement = 75%
F = 91°C
RMN[1]H (DMSO/TMS) : 7,20(s,1H) ; 7,0 à 6,7(m,3H) ; 6,0(s,2H) ; 4,10(d,2H,J = 4,5Hz) ; 3,65(d,2H,J = 7Hz) ; 2,0(t,1H, J = 7Hz).

Exemple 89 : Préparation du N-(E)-[oxo-1(acétylthiométhyl)-2ène -2(méthylènedioxy-3,4 phényl)-3 propyl]-glycinate de benzyle

A. Préparation de l'acide (E) acétylthiométhyl-2 (méthylènedioxy-3,4 phényl)-3 propénoïque

On irradie l'acide (Z) acétylthiométhyl-2(méthylènedioxy-3,4 phényl)-3 propénoïque préparé dans l'exemple 87 (stade B) selon le mode opératoire décrit à l'exemple 81 (stade C).
Rendement = 25%
RMN[1]H ($CDCl_3$/TMS) : 10,6(s,1H) ; 7,2 à 6,6(m,4H) ; 5,9(s,2H) ; 3,8(s,2H) ; 2,3(s,3H).

B. Préparation du N-(E)-[oxo-1(acétylthiométhyl)-2ène-2(méthylènedioxy-3,4 phényl)-3 propyl]-glycinate de benzyle

On couple l'acide (E) acétylthiométhyl-2(méthylènedioxy-3,4 phényl)-3 propénoïque obtenu sous A avec le glycinate de benzyle selon le mode opératoire décrit à l'exemple 1 (étape f).
Rendement = 58%
F = 113°C
RMN[1]H ($CDCl_3$/TMS) : 7,3(s,5H) ; 7,0 à 6,65(m,4H) ; 6,15(m,1H) ; 5,9(s,2H) ; 5,15(s,2H) ; 4,05(d,2H,J = 5Hz) ; 3,8(s,2H) ; 2,3(s,3H).

Exemple 90 : Préparation du N-[N-(RS)-[ter-butoxycarbonyl-1 phényl-2 éthyl]-(S)- phénylalanyl]-glycinate de benzyle

A. Préparation de l'acide (RS)-hydroxy-2 phényl-3 propanoïque

A une solution de 15 g (90,80 mmol) de (S)-phénylalanine dans 230 ml d'une solution aqueuse d'acide sulfurique à 10 %, on ajoute en une heure à -5°C une solution de 22,55 g (326,80 mmol) de nitrite de sodium en solution dans 90 ml d'eau. On laisse revenir à la température ambiante puis on chauffe le milieu réactionnel à 50°C pendant 3 heures.

On extrait, après retour à la température ambiante, la solution par 3 fois 100 ml d'acétate d'éthyle. Les phases organiques réunies sont lavées par 50 ml d'eau et par 50 ml d'une solution aqueuse saturée de chlorure de sodium. Après séchage sur sulfate de magnésium, filtration et évaporation, on obtient 9,25 g d'un résidu solide jaune. On recristallise ce solide dans un mélange de solvants éther/éther de pétrole. On obtient 6,44 g de solide blanc.
Rendement = 43 %
Rf : 0,55 (éluant 70/30 benzène/acide acétique)
RMN$^1$H (acétone $D_6$/TMS) : 7,2 (s, 5H), 7,2 à 5,4 (m, 1H), 4,55 à 4,3 (m,1H) ; 3,35 à 2,7 (m, 3H).

B. Préparation de l'acide (RS)-acétyloxy-2 phényl-3 propanoïque

On ajoute 4,83 g (61,53 mmol) de chlorure d'acétyle à 8,88 g (53,49 mmol) d'acide (RS)-hydroxy-2 phényl-3 propanoïque. On porte à reflux pendant 30 minutes. On évapore l'excès de chlorure d'acétyle. On obtient 11,18 g d'acide (RS)-acétyloxy-2 phényl-3 propanoïque.
Rendement = 98 %
RMN$^1$H ($CDCl_3$/TMS) : 11,0 (s, 1H) ; 7,2 (s, 5H) ; 5,25 (m, 1H) ; 3,4 à 2,8 (m, 2H) ; 2,0 (s, 3H).

C. Préparation du (RS)-acétyloxy-2 phényl-3 propanoate de tertiobutyle

A une solution de 11,4 g (54,80 mmol) d'acide obtenu sous B et de 4,06 g (54,80 mmol) de tertiobutanol en solution dans 27 ml de pyridine, on ajoute à une température inférieure à 40°C, une solution de 8,4 g (54,80 mmol) d'oxychlorure de phosphore dans 12 ml de dichlorométhane.

On refroidit à 5°C, on filtre et on lave le précipité au dichlorométhane. Le filtrat est successivement lavé à l'eau, 2 fois par une solution aqueuse saturée de $NaHCO_3$, 1 fois à l'eau, 2 fois par une solution aqueuse d'HCl 1N et 1 fois à l'eau. La phase organique est séchée sur $MgSO_4$, filtrée et concentrée. On obtient 10,5 g de (RS)-acétyloxy-2 phényl-3 propanoate de tertiobutyle.
Rendement = 73 %
RMN$^1$H ($CDCl_3$/TMS) : 7,2 (s, 5H) ; 5,1 (t, IH, J = 5,3 Hz) ; 3,05 (d, 2H, J = 5,3 Hz) ; 2,0 (s, 3H) ; 1,3 (s, 9H).

D. Préparation du (RS)-hydroxy-2 phényl-3 propanoate de tertiobutyle

On place dans un ballon 9,25 g (35,03 mmol) du produit obtenu sous C en solution dans 60 ml d'un mélange méthanol/$H_2O$ (70/30). On refroidit à 0°C et on ajoute 17,5 ml d'une solution aqueuse de NaOH 1N. On laisse agiter 1 heure.

Le méthanol est évaporé sous vide, et la phase aqueuse est extraite 2 fois à l'éther. Les phases organiques sont réunies, lavées 1 fois à l'eau, séchées sur MgSO4, filtrées et concentrées. On obtient 6,52 g d'une huile que l'on chromatographie sur silice (éluant : 10/90 éther/éther de pétrole).
Masse obtenue = 5,98 g
Rendement = 77 %
Rf : 0,36 (25/75 éther/éther de pétrole)
RMN$^1$H ($CDCl_3$/TMS) : 7,2 (s, 5H) ; 4,3 (m, 1H) ; 3,1 à 2,75 (m, 3H) ; 1,35 (s, 9H).

E. Préparation du triflate du (RS)-hydroxy-2 phényl-3 propionate de tertiobutyle

A une solution de 0,73 g (2,61 mmol) d'anhydride de trifluoromethane sulfonyle dans 3 ml de $CH_2Cl_2$, on ajoute à -20°C, 0,5 g (2,25 mmol) de (RS)-hydroxy-2 phényl-3 propanoate de tertiobutyle et 0,18 g (2,25 mmol) de pyridine en solution dans 1 ml de $CH_2Cl_2$. On agite pendant minutes.

On reprend le milieu réactionnel à l'eau et au $CH_2Cl_2$, on lave la phase organique par une solution aqueuse d'HCl 0,1 N puis par une solution aqueuse saturée de $NaHCO_3$ et ensuite à l'eau. La phase organique est sé-

chée sur $MgSO_4$, filtrée et concentrée. On obtient 0,7 g de triflate du (RS)-hydroxy-2 phenyl-3 propionate de tertiobutyle.
Rendement = 89 %
RMN$^1$H ($CDCl_3$/TMS) : 7,25 (s, 5H) ; 5,1 (m, 1H) ; 3,2 (m, 2H) ; 1,4 (s, 9H).

F. Préparation du N-(RS)-(ter-butoxycarbonyl-1 phényl-2 éthyl)-(S)-phénylalaninate de méthyle

A une solution de 0,66 g (1,86 mmol) de triflate du (RS)-hydroxy-2 phényl-3 propionate de tertiobutyle et de 1,86 mmol de bis-(diméthylamino)-1,8 naphtalène dans 6,5 ml de $CH_2Cl_2$, on ajoute à 0°C en 5 minutes, une solution de 0,4 g (1,86 mmol) de (S)-phénylalaninate de méthyle dans 6,5 ml de $CH_2Cl_2$. On agite à la température ambiante pendant 24 heures. On filtre, on lave le filtrat à l'eau et on sèche sur $MgSO_4$. On filtre et on concentre. On obtient 1 g de produit que l'on chromatographie sur silice.
Rendement = 82 %
Masse = 0,58 g
IR : 3320, 1735 $cm^{-1}$
RMN$^1$H ($CDCl_3$/TMS) : 7,1(m,10H) ; 3,55 et 3,5(s,3H) ; 3,5 à 3,15(m,2H) ; 3,0 à 2,7(m,4H) ; 2,05(m,1H) ; 1,25 et 1,2(s,9H).

G. Préparation de la N-(RS)-[ter-butoxycarbonyl-1 phényl-2 éthyl]-(S)-phénylalanine

On ajoute à 0°C un équivalent de NaOH 1N à une solution de 1,5 mmol du composé obtenu sous F en solution dans 5 ml de méthanol. On agite pendant 24 heures à température ambiante. On évapore le MeOH et on lave la phase aqueuse à l'éther. On acidifie par une solution d'HCl 1N et on extrait au $CHCl_3$. Les phases organiques réunies sont séchées sur $MgSO_4$, filtrées et concentrées.
Rendement = 87%
RMN$^1$H ($CDCl_3$/TMS) : 7,3 à 6,7(m,10H) ; 6,3(m,2H) ; 3,5 à 3,2(m,2H) ; 3,15 à 2,6(m,4H) ; 1,35 et 1,3(s,9H).

H. Préparation du N-[N-(RS)-[ter-butoxycarbonyl-1 phényl-2 éthyl]-(S)-phénylalanyl]-glycinate de benzyle

On couple le composé obtenu sous G avec le glycinate de benzyle selon le mode opératoire de l'exemple 1 (étape f).
Rendement = 74% (chromatographié)
IR : 3300, 1730, 1670 $cm^{-1}$
RMN$^1$H ($CDCl_3$/TMS) : 7,5 à 6,8(m,16H) ; 5,1(s,2H) ; 4,1 à 3,9(m,2H) ; 3,7 à 2,4(m,6H) ; 1,8(m,1H) ; 1,25 et 1,2(s,9H).

Microanalyse : $C_{31}H_{36}O_5N_2$
Calc. % C = 72,07 H = 7,02 N = 5,42
Tr. % C = 71,85 H = 6,91 N = 5,38

Exemple 91 : Préparation du chlorhydrate de N-[N-(RS)-[carboxy-1 phényl-2 éthyl]-(S)-phénylalanyl]-glycine

Une solution de 0,9 mmol du composé de l'exemple 90 H, dans 7 ml d'éthanol est hydrogénée pendant 15 heures à température ambiante en présence de 50 mg de charbon palladié 10%. On filtre, on évapore à sec et on reprend le résidu par 5 ml d'une solution d'HCl 4N dans de l'acétate d'éthyle. Après 15 heures d'agitation, on évapore à sec, on triture le solide obtenu dans l'éther et on sèche au dessicateur sur $P_2O_5$.
Rendement = 90%
F = 80°C
RMN$^1$H ($CDCl_3$/TMS) : 9,0 à 7,8(m,4H) ; 7,1(s,11H) ; 4,6 à 2,8(m,8H).

Microanalyse : $C_{20}H_{23}O_5N_2Cl$
Calc. % C = 59,14 H = 5,70 N = 6,89
Tr. % C = 59,40 H = 6,10 N = 6,50

Exemple 92 : Préparation du N-[N-(RS)-[ter-butoxycarbonyl -1 phényl-2 éthyl]-(RS) - (méthylènedioxy-3,4 phényl)-3 alanyl]-glycinate de benzyle

A. Préparation du N-(RS)-[ter-butoxycarbonyl-1 phényl-2 éthyl]-(RS)-(méthylènedioxy-3,4 phényl)-3 alaninate de méthyle

On substitue le triflate du (RS)-hydroxy-2 phényl-3 propionate de tertiobutyle préparé à l'exemple 90 D par le (RS)-(méthylènedioxy-3,4 phényl)-3 alaninate de méthyle selon le mode opératoire de l'exemple 90 F.
Rendement = 53% (chromatographié)
RMN$^1$ H ($CDCl_3$/TMS) : 7,15(s,1H) ; 6,7 à 6,4(m,3H) ; 5,8(s,2H) ; 3,6 et 3,55(s,3H) ; 3,6 à 3,2(m,2H) ; 2,9 à 2,6(m,4H) ; 2,1(m,1H) ; 1,3 et 1,25(s,9H).

B. Préparation de la N-(RS)-[ter-butoxycarboxyl-1 phényl-2 éthyl]-(RS)-(méthylènedioxy-3,4 phényl)-3 alanine

On déprotège le composé obtenu sous A selon le mode opératoire de l'exemple 90 G.
Rendement = 87%
F = 128°C
RMN$^1$H ($CDCl_3$/TMS) : 7,2(s,5H) ; 6,7(m,3H) ; 5,9(s,2H) ; 5,8 à 5,0(m,2H) ; 3,7 à 2,4(m,6H) ; 1,35 et 1,3(s,9H).

C. Préparation du N-[N-(RS)-[ter-butoxycarbonyl-1 phényl-2 éthyl]-(RS)-(méthylènedioxy-3,4 phényl)-3 alanyl]-glycinate de benzyle

On couple le produit obtenu sous B avec le glycinate de benzyle selon le mode opératoire de l'exemple 1 (étape f).
Rendement = 73%
IR : 3340, 1740 à 1710, 1660 $cm^{-1}$
RMN$^1$H ($CDCl_3$/TMS) : 7,3(s,5H) ; 7,1(s,5H) ; 6,8 à 6,3(m,4H) ; 5,8(s,2H) ; 5,1(s,2H) ; 4,1 à 3,9(m,2H) ; 3,7 à 2,3(m,6H) ; 1,8(m,1H) ; 1,25(s,9H).

Microanalyse : $C_{32}H_{36}O_7N_2$

| | | | | |
|---|---|---|---|---|
| Calc. | % | C = 68,56 | H = 6,47 | N = 5,00 |
| Tr. | % | C = 68,90 | H =6,62 | N = 5,09 |

Exemple 93 : Préparation du chlorhydrate de N-[N-(RS)-[carboxy-1 phényl-2 éthyl]-(RS)-(méthylènedioxy-3,4 phényl)-3 alanyl]-glycine

On déprotège le composé de l'exemple 92 C, selon le mode opératoire de l'exemple 91.
Rendement = 93%
F = 127°C
RMN$^1$H ($CDCl_3$/TMS) : 9,2 à 7,7(m,4H) ; 7,2(s,5H) ; 7,1 à 6,4(m,4H) ; 5,8(s,2H) ; 4,6 à 2,8(m,8H).

Microanalyse : $C_{21}H_{23}O_7N_2Cl$

| | | | | |
|---|---|---|---|---|
| Calc. | % | C = 55,94 | H = 5,14 | N = 6,21 |
| Tr. | % | C = 56,30 | H = 5,29 | N = 6,15 |

Exemple 94 : Préparation du N-[N-(RS)-[ter-butoxycarbonyl-1 pentyl]-(RS)-(méthylènedioxy-3,4 phényl)-3 alanyl]-glycinate de benzyle

A. Préparation de l'acide (RS)-hydroxy-2-hexanoïque

On le prépare en partant de la (RS) norleucine, suivant le mode opératoire de l'exemple 90 A.
Rendement = 53 %
Rf = 0,48 (éluant : 70/30 benzène/acide acétique)

RMN[1]H ($CDCl_3$/TMS) : 7,3 (s, 5H) ; 4,3 (m, 3H) ; 2,2 à 0,65 (m, 9H).

B. Préparation de l'acide (RS)-acétyloxy-2 hexanoïque

Le composé obtenu sous A est traité par le chlorure d'acétyle suivant le protocole de l'exemple 90 B.
Rendement = 98 %
RMN[1]H ($CDCl_3$/TMS) : 11,8 (s, 1H) ; 4,9 (t, IH, J = 6,4 Hz); 2,0 (s, 3H) ; 2,0 à 0,6 (m, 9H).

C. Préparation du (RS)-acétyloxy-2 hexanoate de tertiobutyle.

Le composé obtenu sous B est estérifié comme dans le cas de l'exemple 90°C.
Rendement = 82 %
RMN[1]H ($CDCl_3$/TMS) : 4,8 (t, 1H, J = 6,4 Hz) ; 2,1 (s, 3H) ; 2,0 à 1,6 (m, 2H) ; 1,4 (s, 9H) ; 1,6 à 0,6 (7H).

D. Préparation du (RS)-hydroxy-2 hexanoate de tertiobutyle

Le composé obtenu sous C est traité par NaOH 1N comme dans le cas de l'exemple 90 D.
Rendement = 67 %
RMN[1]H ($CDCl_3$/TMS) : 4,0 (m, 1H) ; 2,8 (d, 1H, J = 5,4 Hz) ; 1,9 à 0,6 (m, 18H).

E. Préparation du triflate du (RS)-hydroxy-2 hexanoate de tertiobutyle

Le composé obtenu sous D est traité par l'anhydride du trifluorométhane sulfonyle comme dans le cas de l'exemple 90 E.
Rendement = 46 %
RMN[1]H ($CDCl_3$/TMS) = 5,1 (m, 1H) ; 1,9 à 0,7 (m, 18H).

F. Préparation du N-(RS)-(ter-butoxycarbonyl-1 pentyl)-(RS)-(méthylènedioxy-3,4 phényl)-3 alaninate de méthyle.

On substitue le triflate du (RS)-hydroxy-2 hexanoate de tertiobutyle par le (RS)-(méthylènedioxy-3,4 phényl)-3 alaninate de méthyle selon le mode opératoire de l'exemple 90 F.
Rendement = 59% (chromatographié)
IR : 3340, 1735 cm-1
RMN[1]H ($CDCl_3$/TMS) : 6,6(m,3H) ; 5,9(s,2H) ; 3,6(s,3H) ; 3,6 à 3,2(m,2H) ; 3,2 à 2,7(m,4H) ; 1,9(m,1H) ; 1,3(s,9H) ; 1,7 à 0,6(m,7H)

G. Préparation de la N-(RS)-[ter-butoxycarbonyl-1 pentyl]-(RS)-(méthylènedioxy-3,4 phényl)-3 alanine

Le composé obtenu sous F est saponifié selon le mode opératoire de l'exemple 90 G.
Rendement = 82%
RMN[1]H ($CDCl_3$/TMS) : 6,7(m,3H) ; 5,9(s,2H) ; 5,8 à 5,3(m,2H) ; 3,6 à 2,8(m,6H) ; 1,4(s,9H) ; 2,1 à 0,5(m,7H).

H. Préparation du N-[N-(RS)-[ter.butoxycarbonyl-1 pentyl]-(RS)-(méthylènedioxy-3,4 phényl)-3 alanyl]-glycinate de benzyle

Le composé obtenu sous G est couplé avec le glycinate de benzyle selon le mode opératoire de l'exemple 1 (étape f).
Rendement = 80%
IR : 3360, 1720, 1660 $cm^{-1}$
RMN[1]H ($CDCl_3$/TMS) : 7,3(s,5H) ; 6,7(m,4H) ; 5,85(s,2H) ; 5,2(s,2H) ; 4,1 à 3,9(m,2H) ; 3,6 à 2,4(m,6H) ; 1,4(s,9H) ; 2,0 à 0,6(m,8H).

Microanalyse : $C_{29}H_{38}O_7N_2$

| | | | | |
|---|---|---|---|---|
| Calc. | % | C = 66,14 | H = 7,27 | N = 5,32 |
| Tr. | % | C = 65,88 | H = 7,03 | N = 5,44 |

Exemple 95 : Préparation du chlorhydrate de N-[N-(RS)- [carboxy-1 pentyl]-(RS)-(méthylènedioxy-3,4 phényl)-3 alanyl]-glycine

Le composé de l'exemple 94 H est déprotégé selon le mode opératoire de l'exemple 91.
Rendement = 94%
F = 85°C
RMN$^1$H ($CDCl_3$/TMS) : 9,2 à 7,8(m,4H) ; 7,2 à 6,4(m,4H) ; 5,8(s,2H) ; 4,8 à 3(m,8H) ; 2,3 à 0,6(m,7H).

Microanalyse : $C_{18}H_{25}O_7N_2Cl$

| | | | | |
|---|---|---|---|---|
| Calc. | % | C = 51,86 | H = 6,04 | N = 6,72 |
| Tr | % | C =51,68 | H = 6,21 | N = 6,53 |

Exemple 96 : Préparation du N-(R,S) [(diéthoxyphosphinyl)méthyl-2 oxo-1 (méthylènedioxy-3,4)phényl-3 propyl]-(S)-alaninate de benzyle

A. Préparation du chlorure d'acide ((méthylènedioxy-3,4 phényl)méthyl)-2 propénoïque

A 2,43 g du produit issu de l'exemple 1 (étape d) (11,8 mmol), on ajoute 1,3 ml de chlorure de thionyle (17,7 mmol ; 1,5 éq.) à 0°C et sous agitation. On laisse la température remonter à 20°C et, après 14 heures, on évapore le chlorure de thionyle résiduel. On obtient 2,65 g d'une huile jaune.
Rendement = 100%
RMN$^1$H = 3,50(s,2H) ; 5,60(s élargi,1H) ; 5,95(s,2H) ; 6,45(s, élargi,1H) ; 6,75(m,3H).

B. Préparation du N-[oxo-1 ((méthylènedioxy-3,4 phényl)méthyl)-2 propènyl]-(S)-alaninate de benzyle

A 1,12 g du produit issu de A (5 mmol ; 1 éq.) en solution dans 50 ml d'un mélange 50/50 de chloroforme et de tétrahydrofuranne, on ajoute 1 g de triéthylamine (10 mmol ; 2 éq.) en solution dans 20 ml de tétrahydrofuranne, à 0°C et sous agitation. Puis, on verse 1,8 g de paratoluène sulfonate d'alaninate de benzyle (5 mmol ; 1 éq.) en solution dans 50 ml d'un mélange 50/50 de chloroforme et de tétrahydrofuranne. On laisse la température remonter à 20°C et, après 14h, on évapore les solvants. Après avoir repris par 50 ml d'acétate d'éthyle et 50 ml d'eau, on effectue deux lavages par 100 ml d'une solution d'acide chlorhydrique 1N et par 100 ml d'une solution aqueuse saturée d'hydrogénocarbonate de sodium. La phase organique est recueillie, séchée sur sulfate de magnésium, filtrée et concentrée. Purification par chromatographie (éluant : chloroforme 97,5/méthanol 2,5).
Rendement = 90%.
On isole 1,50 g d'un solide beige.
F < 50°C
RMN$^1$H : 1,10 (d,3H,J = 6Hz) ; 3,60(s,2H) ; 4,60 à 4,65(m,1H) ; 5,05(s,2H) ; 5,10(s élargi,1H) ; 5,65(s élargi,1H) ; 6,00(s,2H) ; 6,20 à 6,40(s élargi,1H) ; 6,70 à 6,80(m,3H) ; 7,25(s élargi,5H).

C. Préparation du N-(R,S)-[(diéthoxyphosphinyl)méthyl-2 oxo-1 (méthylènedioxy-3,4)phényl-3 propyl]-(S)-alaninate de benzyle

On introduit sous atmosphère inerte et à 0°C, 630 mg de diéthylphosphite (4,6 mmol ; 1 éq.) en solution dans 50 ml de tétrahydrofuranne sur 220 mg d'hydrure de sodium (5,5 mmol ; 1,2 éq.) (à 60% dans l'huile minérale ; préalablement lavés par 2 x 10 ml d'éther de pétrole). Après que le dégagement d'hydrogène a cessé, on ajoute 1,50 g du produit issu de B (1 éq.) en solution dans 25 ml de tétrahydrofuranne. On laisse la température remonter à 20°C et, après 14 heures, on ajoute 2 ml d'éthanol. On évapore les solvants. Après avoir repris par 50 ml d'acétate d'éthyle et 50 ml d'eau, on effectue deux lavages par 50 ml d'une solution d'acide chlorhydrique 1N et par 50 ml d'une solution aqueuse saturée d'hydrogénocarbonate de sodium. La phase organique est recueillie, séchée sur sulfate de magnésium, filtrée et concentrée. On isole 1,50 g d'une huile par chromatographie (éluant : chloroforme 96/méthanol 4).
Rendement = 72%
RMN$^1$H : 1,10 à 1,40(m,9H) ; 1,70 à 2,65(m,2H) ; 2,70 à 3,25(m,3H) ; 3,40 à 4,20(m,4H) ; 4,55 à 4,65(m,1H) ; 5,10(s élargi,2H) ; 6,00(s,2H) ; 6,20 à 6,80(m,4H) ; 7,20(s élargi,5H).
IR : 3280, 1740, 1675, 1550, 1255, 1065, 1030 $cm^{-1}$

Microanalyse : $C_{25}H_{32}NO_8P$

Calc. % C = 59,41 N = 2,92 H = 6,31

Tr. % C = 59,58 N = 2,79 H = 6,25

SM : 505 ; 496, 468, 420, 401, 380, 353, 311, 273, 234.

Exemple 97 : Préparation de la N-(R,S)-[(dihydroxyphosphinyl)méthyl-2 oxo-1 (méthylènedioxy-3,4 phényl)-3 propyl]-(S)-alanine

A température ambiante, sous atmosphère d'argon et sous agitation, on ajoute 3,33 ml de bromotriméthylsilane (24 mmol ; 5,5 éq.) à une solution de 1,50 g du produit de l'exemple 96 C, (3,31 mmol ; 1 éq.) dans 7 ml de chlorure de méthylène. Après 4 heures, le mélange est concentré. On ajoute alors 15 ml d'acide chlorhydrique 6N. Après 14 heures, l'eau de la phase aqueuse et les produits volatils sont évaporés et 657 mg d'un solide hygroscopique orange sont récupérés.

Rendement = 99%

RMN$^1$H : (CD3OD) 1,20(d,3H,J = 5Hz) ; 1,70 à 2,55(m,2H) ; 2,65 à 3,35(m,3H) ; 4,50 à 4,60(m,1H) ; 4,80 (s élargi,4H) ; 5,00(s,2H) ; 6,20 à 6,80(m,3H).

Microanalyse : $C_{14}H_{18}NO_8P.2H_2O$

Calc. % C = 42,53 N = 3,54 H = 5,57

Tr. % C = 43,27 N = 3,68 H = 5,43

SM : 359, 302, 287, 251, 196.

Exemple 98 : Préparation du mono sel calcique de la N-(R,S)-[(dihydroxyphosphinyl)méthyl-2 oxo-1 (méthylènedioxy-3,4phenyl)-3 propyl]-(S)-alanine

Un mélange de 325 mg du produit issu de l'exemple 97 (1,19 mmol), de 33,6 mg de monoxyde de calcium (0,60 mmol) et de 5 ml d'eau sont soumis aux ultra-sons jusqu'à complète dissolution du solide. L'eau est évaporée et on récupère 350 mg d'un sel blanchâtre.

Rendement = 100%

Microanalyse : $C_{14}H_{17}NO_8P,Ca1/2$

Calc. % C = 44,35 N = 3,86 H = 4,72

Tr. % C = 44,24 N = 3,74 H = 4,65

Exemple 99 : Préparation du N-(R,S)-[(diéthoxyphosphinyl)méthyl-2 oxo-1 (méthylènedioxy-3,4 phényl)-3 propyl]-glycinate de benzyle

A. Préparation du N-(oxo-1(méthylènedioxy-3,4 phényl)méthyl-2 propényl]-glycinate de benzyle

Le mode opératoire est identique à celui de l'exemple 96 B, à ceci près que le paratoluène sulfonate de glycinate de benzyle remplace le paratoluène sulfonate d'alaninate de benzyle. Purification par chromatographie (éluant : chloroforme 97,5/méthanol 2,5)

Rendement = 88%.

On isole un solide beige.

RMN$^1$H : 3,60(s,2H) ; 4,05(s élargi,2H) ; 5,05(s,2H) ; 5,10(s,1H) ; 5,65(s élargi, 1H) ; 6,00(s,2H) 6,20 à 6,40(s élargi,1H) ; 6,70 à 6,80(m,3H) ; 7,25(s élargi,5H).

B. Préparation du N-(R,S)-[(diéthoxyphosphinyl)méthyl-2 oxo-1(méthylènedioxy-3,4 phényl)-3 propyl]-glycinate de benzyle

Le mode opératoire est identique à celui de l'exemple 96 C à ceci près que le glycinate de benzyle remplace l'alaninate de benzyle. On obtient une poudre beige. Purification par chromatographie (éluant : chloroforme 96/méthanol 4).
Rendement = 69%
F < 50°C
RMN$^1$H : 1,10 à 1,35(t,6H,J = 7Hz) ; 1,70 à 2,65(m,2H) ; 2,70 à 3,25(m,3H) ; 3,40 à 4,20(m,6H) ; 5,10(s,2H) ; 6,00(s,2H) ; 6,20 à 6,80(m,4H) ; 7,20(s élargi,5H).

Microanalyse : $C_{24}H_{30}O_8P$

| | | | | |
|---|---|---|---|---|
| Calc. | % | C = 58,66 | N = 2,85 | H = 6,31 |
| Tr. | % | C = 58,77 | N = 2,79 | H = 6,25 |

SM : 491, 400, 372, 344, 312, 286.

Exemple 100 : Préparation de la N-(R,S)-[(dihydroxyphosphinyl)méthyl-2 oxo-1(méthylènedioxy-3,4 phényl)-3 propyl]-glycine

Le mode opératoire est identique à celui de l'exemple 97 à ceci près que le produit issu de l'exemple 99B remplace le produit issu de l'exemple 96C. On obtient un solide hygroscopique orange.
Rendement = 99%
RMN$^1$H :(CD3OD) 1,70 à 2,55(m,2H) ; 2,65 à 3,30(m,3H) ; 4,05(s élargi,2H) ; 4,80(s élargi,4H) ; 6,00(s,2H) ; 6,20 à 6,80(m,4H).

Microanalyse : $C_{13}H_{16}NO_8P.2H_2O$

| | | | | |
|---|---|---|---|---|
| Calc. | % | C = 40,94 | N = 3,67 | H = 5,25 |
| Tr. | % | C = 41,19 | N = 3,68 | H = 5,03 |

SM = 345, 300, 271, 243, 211, 175, 121.

Exemple 101 : Préparation du mono sel calcique de la N-(R,S)-[(dihydroxyphosphinyl)méthyl-2 oxo-1(méthylènedioxy-3,4 phényl]-3 propyl]-glycine

Le mode opératoire est identique à celui de l'exemple 98 à ceci près que le produit issu de l'exemple 100 remplace le produit issu de l'exemple 97. On obtient une poudre blanche.
Rendement = 100%

Microanalyse : $C_{13}H_{16}NO_8P,Ca1/2$

| | | | | |
|---|---|---|---|---|
| Calc. | % | C = 42,86 | N = 3,85 | H = 4,67 |
| Tr. | % | C = 43,14 | N = 3,74 | H = 4,75 |

Exemple 102 : Préparation du N-(R,S)-[(diéthoxyphosphinyl)méthyl-2 oxo-1 (phényl-4 phényl)-3 propyl]-(S)-alaninate de benzyle

A. Préparation du chlorure de l'acide ((phényl-4 phényl)méthyl)-2 propénoïque

On prépare le chlorure d'acide par réaction de l'acide ((phényl-4)benzyl)-2 acrylique avec le chlorure de thionyle selon le mode opératoire de l'exemple 96 A. On obtient une huile jaune.
Rendement = 100%
RMN$^1$H : 3,50(s,2H) ; 5,64(s,1H) ; 6,48(s,1H) ; 7,20 à 7,70(m,9H)

B. Préparation du N-[(phényl-4 phényl)méthyl-2 oxo-1 propényl]-(S)-alaninate de benzyle

On couple le produit obtenu sous A selon le mode opératoire de l'exemple 96 B.
Chromatographie (éluant : chloroforme 97,5/méthanol 2,5)
Rendement = 91%
On isole 1,50 g d'un solide beige.
F < 50°C
RMN$^1$H : 1,10(d,3H,J = 6Hz) ; 3,60(s,2H) ; 4,50 à 4,60(m,1H) ; 5,00(s,2H) ; 5,10(s,1H) ; 5,60(s élargi,1H) ; 6,20 à 6,40 (s élargi,1H) ; 7,20 à 7,70(m,14H).

C. Préparation du N-(R,S)-[(diéthoxyphosphinyl)méthyl-2 oxo-1 (phényl-4 phényl)-3 propyl]-(S)-alaninate de benzyle

On le prépare à partir du composé obtenu sous B selon le mode opératoire de l'exemple 96 C.
Chromatographie (éluant : chloroforme 96/méthanol 4)
On obtient une huile.
Rendement = 69%
RMN$^1$H : 1,10 à 1,40(m,9H) ; 1,70 à 2,65(m,2H) ; 2,70 à 3,30(m,3H) ; 3,40 à 3,80(q,4H,J = 6Hz) ; 4,50 à 4,60(m,1H) ; 5,10(s,2H) ; 6,20 à 6,50(s élargi, 1H) ; 7,20 à 7,80(m,14H).
IR : 3280, 1740, 1675, 1550, 1255, 1065, 1030 $cm^{-1}$

Microanalyse : $C_{30}H_{36}NO_6P$

| | | | |
|---|---|---|---|
| Calc. % | C = 67,03 | N = 2,61 | H = 6,75 |
| Tr. % | C = 67,58 | N = 2,74 | H = 6,75 |

SM : 537, 446, 418, 390, 341, 318, 285, 247.

Exemple 103 : Préparation de la N-(R,S)-[(dihydroxyphosphinyl)méthyl-2 oxo-1 (phényl-4 phényl)-3 propyl]-(S)-alanine

Le produit de l'exemple 102 C est déprotégé selon le mode opératoire de l'exemple 97.
On isole une huile par chromatographie (éluant : chloroforme 96/méthanol 4).
Rendement = 73%
RMN$^1$H (CD3OD) : 1,25(d,3H,J = 5Hz) ; 1,70 à 2,65(m,2H) ; 2,70 à 3,20(s élargi,3H) ; 4,55 à 4,60(m,1H) ; 4,80(s élargi,4H) ; 7,20 à 7,80(m,9H).

Microanalyse : $C_{19}H_{22}NO_6P.2H_2O$

| | | | |
|---|---|---|---|
| Calc. % | C = 53,40 | N = 3,28 | H = 6,13 |
| Tr. % | C = 54,27 | N = 3,39 | H = 6,02 |

SM : 391, 342, 319, 256, 194, 176.

Exemple 104 : Préparation du mono sel calcique de la N-(R,S)-[(dihydroxyphosphinyl)méthyl-2 oxo-1 (phényl-4 phényl)-3 propyl]-(S)-alanine

Le produit de l'exemple 103 est traité comme décrit dans l'exemple 98.
On obtient une poudre blanche.
Rendement = 100%

Microanalyse : $C_{19}H_{21}NO_6P,Ca_{1/2}$

| | | | |
|---|---|---|---|
| Calc. % | C = 55,61 | N = 3,41 | H = 5,16 |
| Tr. % | C = 55,49 | N = 3,54 | H = 5,26 |

Exemple 105 : Préparation du N-(R,S)-[(diéthoxyphosphinyl)méthyl-2 oxo-1 (phényl-4 phényl)-3 propyl]-glycinate de benzyle

A.Préparation du N-[(phényl-4 phényl)méthyl-2 oxo-1 propényl]-glycinate de benzyle

Le composé de l'exemple 102 A est couplé avec le glycinate de benzyle selon le mode opératoire de l'exemple 96 B. On obtient une poudre blanche. Purification par chromatographie (éluant : chloroforme 97,5/méthanol 2,5).
Rendement = 92%
RMN$^1$H : 3,60(s,2H) ; 4,05(s élargi,2H) ; 5,05(s,2H) ; 5,10(s,1H) ; 5,60(s élargi,1H) ; 6,20 à 6,40(s élargi,1H) ; 7,20 à 7,75(m,14H).

B. Préparation du N-(R,S)-[(diéthoxyphosphinyl)méthyl-2 oxo-1 (phényl-4 phényl)-3 propyl]-glycinate de benzyle

Il est préparé comme dans le cas de l'exemple 96 C. On obtient une poudre beige qui est purifiée par chromatographie (éluant : chloroforme 96/méthanol 4).
Rendement = 70%
F < 50°C
RMN$^1$H : 1,10 à 1,35(t,6H,J = 7Hz) ; 1,70 à 2,65(m,2H) ; 2,70 à 3,25(m,3H) ; 3,40 à 4,20(m,6H) ; 5,10(s élargi, 2H) ; 6,30 à 6,70(s élargi,1H) ; 7,20 à 7,80(m,14H).

Microanalyse : $C_{29}H_{34}NO_6P$

| | | | |
|---|---|---|---|
| Calc. % | C = 66,53 | N = 2,68 | H = 6,55 |
| Exp. % | C = 66,58 | N = 2,73 | H = 6,45 |

SM : 523, 432, 404, 376, 317, 281.

Exemple 106 : Préparation de la N-(R,S)-[(dihydroxyphosphinyl)méthyl-2 oxo-1 (phényl-4 phényl)-3 propyl]-glycine

Le composé de l'exemple 105 B est traité comme décrit dans l'exemple 97. On obtient un solide hygroscopique orange.
Rendement = 98%
RMN$^1$H : ($CD_3OD$) 1,95 à 2,55(m,2H) ; 2,65 à 3,30(m,3H) ; 4,05(s élargi,2H) ; 4,80(s élargi,4H) ; 6,20 à 6,50(s élargi,1H).

Microanalyse : $C_{18}H_{20}NO_6P.2H_2O$

| | | | |
|---|---|---|---|
| Calc. % | C = 52,30 | N = 3,39 | H = 5,85 |
| Tr. % | C = 53,13 | N = 3,48 | H = 5,73 |

SM : 333, 254, 188, 164

Exemple 107 : Préparation du mono sel calcique de la N-(R,S)-[(dihydroxyphosphinyl)méthyl-2 oxo-1 (phényl 4 phényl)-3 propyl]-glycine

Le produit de l'exemple 106 est traité comme décrit dans l'exemple 98. On obtient une poudre blanche.
Rendement = 100%

Microanalyse : $C_{18}H_{19}NO_6P,Ca_{1/2}$

| | | | | |
|---|---|---|---|---|
| Calc. | % | C = 54,55 | N = 3,53 | H = 4,83 |
| Tr. | % | C = 54,69 | N = 3,44 | H = 4,92 |

ETUDE BIOLOGIQUE

On a procédé aux dosages de l'activité inhibitrice de l'enképhalinase (Enkase) et de l'activité inhibitrice de l'ACE (Giros et al;, J. Pharmac. Exp. Ther., 1987,243, 666) des composés I précités.

On a rassemblé dans le tableau I les résultats obtenus avec les composés I sous forme racémique et dans le tableau II les résultats obtenus avec certains des composés I sous forme optiquement pure.

TABLEAU I

INHIBITEURS MIXTES 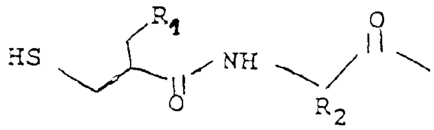 (R,S) (S)

| COMPOSE | $CH_2R_1$ | $R_2$ | IC 50(nm) EDC | ACE |
|---|---|---|---|---|
| EX 1 | | H | 8 | 8 |
| EX 12 | | nPr | 10 | 6 |
| EX 14 | | nBu | 4 | 10 |
| EX 18 | 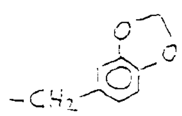 | 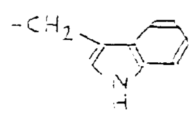 | 4 | 4 |
| EX 20 | | $-CH_2-Ph$ | 10 | 8 |
| EX 22 | | 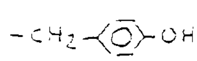 | 6 | 1,5 |
| EX 32 | 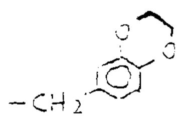 | H | 5 | 10 |
| EX 49 | 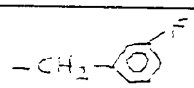 | H | 3 | 10 |
| EX 57 | 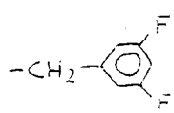 | H | 4 | 8 |
| EX 59 | | $CH_3$ | 2,5 | 10 |
| EX 64 | 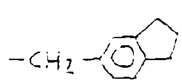 | $CH_3$ | 8 | 10 |
| EX 68 | 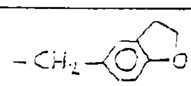 | $CH_3$ | 5 | 9 |

TABLEAU II

INHIBITEURS MIXTES OPTIQUEMENT PURS

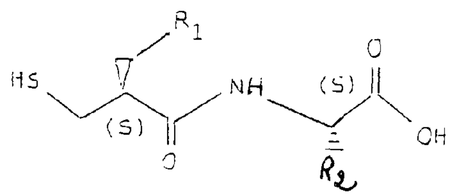

| COMPOSES | $CH_2-R_1$ | $R_2$ | IC50(nM) EDC | ACE |
|---|---|---|---|---|
| EX 4 | 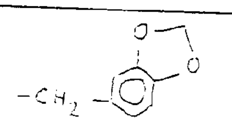 | H | 5 | 5 |
| EX 8 | 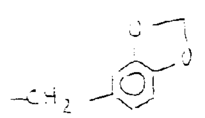 | $CH_3$ | 2 | 10 |
| EX 24 | | $CH_2-OH$ | 3 | 11,7 |
| EX 26 | | $CH_2-CH_2-SCH_3$ | 0,75 | 4,8 |
| EX 45 | 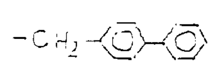 | $CH_3$ | 0,2 | 10 |
| EX 60 | 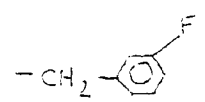 | $CH_3$ | 1,5 | 9 |

On voit que les composés qui figurent dans ces tableaux ont des concentrations inhibitrices IC50 comprises entre 0,1 et 1 nM et ont un rapport des activités inhibitrices des deux enzymes inférieur à 3-4. Ces composés sont d'excellents inhibiteurs mixtes des enzymes enképhalinase et ACE.

On note que l'utilisation des composés Ia ou Ib sous forme optiquement pure permet d'améliorer encore leurs propriétés inhibitrices vis-à-vis des l'enképhalinase et de l'ACE. (Voir exemple 4).

En accord avec leur double activité inhibitrice in vitro, on a pu constater que les composés qui figurent dans les tableaux I et II ou leurs dérivés estérifiés ou thioestérifiés administrés par voie orale à la dose de 3 mg/kg entraînent : un ralentissement de la dégradation de l'ANF $^{125}I$ chez la souris, une augmentation de la natriurèse et de la diurèse chez le rat normo ou spontanément hypertendu, une baisse significative de la pression artérielle moyenne.

## Revendications

1. Dérivés d'amino-acides, caractérisés en ce qu'ils répondent à la formule générale :

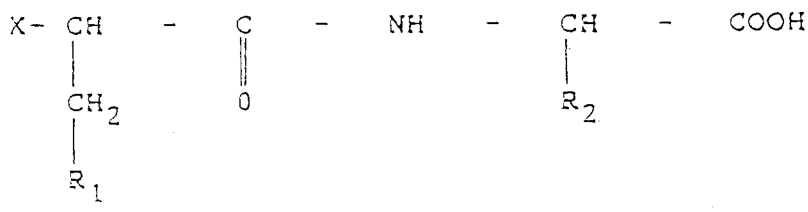

(Ia)

ou

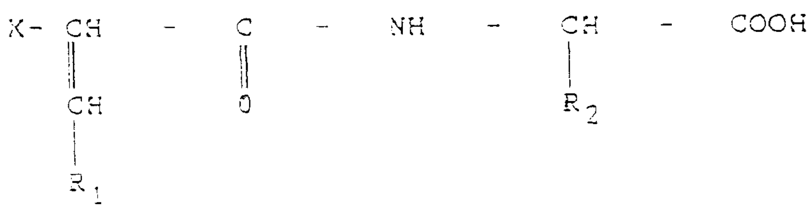

(Ib)

dans laquelle $R_1$ représente pour la formule (Ia) un groupe biphényle ou l'un des groupements suivants :

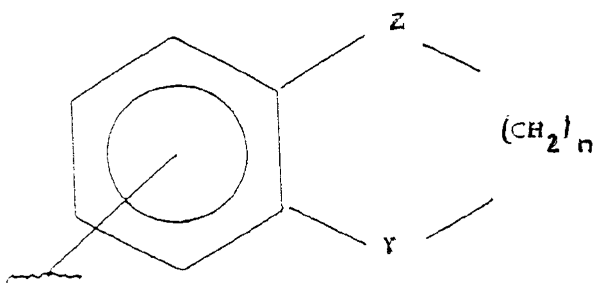

où Z, Y et n ont les significations données ci-dessous :

| Z | Y | n |
|---|---|---|
| 0 | 0 | 1 |
| 0 | $CH_2$ | 1 |
| $CH_2$ | $CH_2$ | 1 |
| 0 | 0 | 2 |
| $CH_2$ | $CH_2$ | 2 |
| 0 | $CH_2$ | 2 |

ou

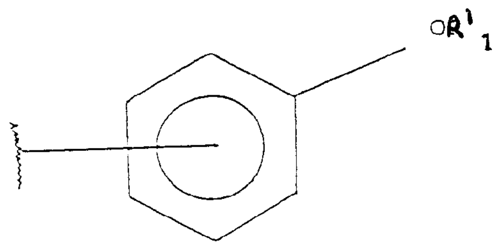

où $R'_1$ représente un groupe alkyle inférieur ; un groupe phényle alkylène inférieur.

Pour la formule (Ib), $R_1$ a la définition précitée et peut être également un groupe phényle,

$R_2$ représente un atome d'hydrogène ; un groupe alkyle inférieur ; un groupe hydroxyalkylène inférieur ; un groupe phényle ; un groupe phénylalkylène inférieur ; un groupe hydroxyphénylalkylène inférieur ; un groupe aminoalkylène inférieur ; un groupe guanidinoalkylène inférieur ; un groupe mercaptoalkylène inférieur ; un groupe alkyle inférieur thioalkylène inférieur ; un groupe imidazolylakylène inférieur ; un groupe indolylalkylène inférieur ; un groupe carbamylalkylène inférieur ; un groupe carboxyalkylène inférieur ou l'un des groupements suivants

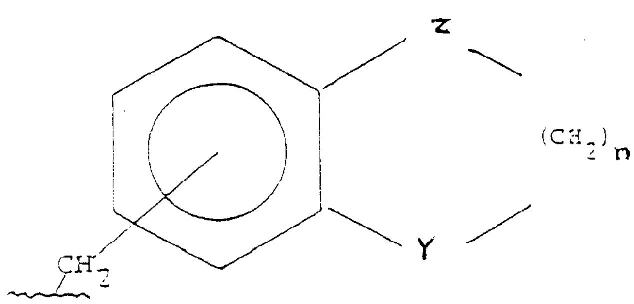

où Z, Y et n ont les significations données ci-dessous :

| Z | Y | n |
|---|---|---|
| O | O | 1 |
| O | O | 2 |
| O | $CH_2$ | 1 |
| $CH_2$ | $CH_2$ | 1 |
| $CH_2$ | $CH_2$ | 2 |

X désigne un groupement responsable de la chélation de l'atome de zinc des enzymes, enképhalinase et ACE, et peut être choisi dans le groupe constitué par un mercaptométhyle; un N-carboxyalkyle de formule

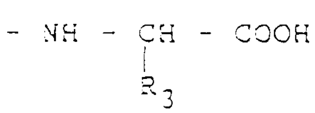

où $R_3$ représente un radical alkyle inférieur, un radical alkyle inférieur benzyle ou un radical alcoxy inférieur benzyle ; des dérivés phosphorés de formule

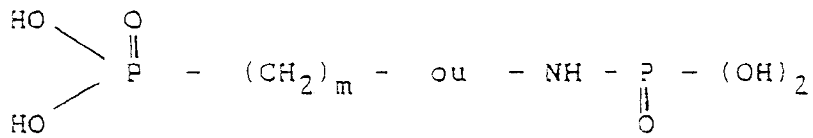

m = 0 ou 1

sous réserve que, lorsque dans la formule (Ia), X représente un groupe mercaptométhyle, l'on n'ait pas simultanément $R_1$ représentant un groupe phényle substitué par un groupe alcoxy en $C_1$-$C_3$ et $R_2$ un groupe alkyle en $C_1$-$C_2$ thioalkylène en $C_1$-$C_3$, et leurs sels d'addition pharmaceutiquement acceptables.

**2.** Dérivés d'amino-acides selon la revendication 1, caractérisés en ce que X représente le groupe mercaptométhyle.

**3.** Dérivés d'amino-acides selon la revendication 2, caractérisés en ce qu'ils sont choisis parmi :

- la N-(RS)- [oxo-1 (mercaptométhyl)-2 (méthylènedioxy-3,4 phényl)-3 propyl]glycine et ses formes optiquement pures,
- la N-(RS)-[oxo-1 (mercaptométhyl)-2 (methylènedioxy-3,4 phényl)-3 propyl]-(S)-alanine, et ses formes optiquement pures,
- l'acide N-(RS)-[oxo-1 (mercaptométhyl)-2 (methylènedioxy-3,4 phényl)-3 propyl] -(S)-amino-2-butyrique,
- la N-(RS)-[oxo-1 (mercaptométhyl)-2 (méthylènedioxy-3,4 phényl)-3 propyl]-(S)- norvaline,
- la N-(RS)-[oxo-1(mercaptométhyl)-2 (méthylènedioxy-3,4 phényl)-3 propyl]-(S)-norleucine,
- la N-(RS)-[oxo-1 (mercaptométhyl)-2 (méthylènedioxy-3,4 phényl)-3 propyl]-(S)-leucine,
- le N-(RS)-[oxo-1 (mercaptométhyl)-2 (méthylènedioxy-3,4 phényl)-3 propyl]-(S)-tryptophane,
- la N-(RS)-[oxo-1 (mercaptométhyl)-2 (méthylènedioxy-3,4 phényl)-3 propyl]-(S)-phénylalanine,
- la N-(RS)-[oxo-1 (mercaptométhyl)-2 (méthylènedioxy-3,4 phényl)-3 propyl]-(S)-tyrosine,
- la N-(S)-[oxo-1 (mercaptométhyl)-2 (méthylènedioxy-3,4 phényl)-3 propyl]-(S)-sérine,
- la N-(S)-[oxo-1 (mercaptométhyl)-2 (méthylènedioxy-3,4 phényl)-3 propyl]-(S)-méthionine,
- la N-(S)-[oxo-1 (mercaptométhyl)-2 (méthylènedioxy-3,4 phényl)-3 propyl]-(RS)-methionine sulfoxyde,
- la N-(S)-[oxo-1 (mercaptométhyl)-2 (méthylènedioxy-3,4 phényl)-3 propyl]-(RS)-(méthylénedioxy-3,4 phényl)-3 alanine,
- la N-(RS)-[oxo-1 (mercaptométhyl)-2 (éthylènedioxy-3,4 phényl)-3 propyl]-glycine,
- la N-(RS)-[oxo-1 (mercaptométhyl)-2 (éthylènedioxy-3,4 phényl) -3 propyl]-(S)-alanine,
- la N-(RS)-[oxo-1 (mercaptométhyl)-2 (méthylènedioxy-2,3 phényl)-3 propyl]-glycine,
- la N-(RS)-[oxo-1 (mercaptométhyl)-2 (phénoxy-4 phényl)-3 propyl]-glycine,
- la N-(RS)-[oxo-1 (mercaptométhyl)-2 (phénoxy-4 phényl)-3 propyl]-(S)-alanine,
- la N-(RS)-[oxo-1 (mercaptométhyl)-2 (phényl-4 phényl)-3 propyl]-glycine, et ses formes optiquement pures,
- la N-[oxo-1 (mercaptométhyl)-2 (phényl-4 phényl)-3 propyl]-(S)-alanine et ses formes optiquement pures,
- la N-[oxo-1 (mercaptométhyl)-2 (phényl-4 phényl)-3 propyl]-(S)-leucine,
- la N-(RS)-[oxo-1 (mercaptométhyl)-2 (indanyl-5')-3 propyl]-glycine,
- la N-(RS)-[oxo-1 (mercaptométhyl)-2 (indanyl-5')-3 propyl]-(S)-alanine,
- la N-(RS)-[oxo-1 (mercaptométhyl)-2 (dihydro-2',3'benzofuranyl-5')-3 propyl]-glycine,
- la N-(RS)-[oxo-1 (mercaptométhyl)-2 (dihydro-2',3'benzofuranyl-5')-3 propyl]-(S)-alanine,
- la N-(RS)-[oxo-1(mercaptométhyl)-2(méthoxy-4 phényl)-3 propyl]glycine,
- la N-(RS)-[oxo-1(mercaptométhyl)-2(méthoxy-4 phényl) -3 propyl]-(S)-alanine,
- la N-(S)-[oxo-1(mercaptométhyl)-2(méthoxy-4 phényl)-3 propyl]-glycine,
- la N-(S)-[oxo-1(mercaptométhyl)-2(méthoxy-4 phényl)-3 propyl]-(S)-alanine,
- la N-(RS)-[oxo-1(mercaptométhyl)-2(éthoxy-4 phényl)-3 propyl]-glycine,
- la N-(RS)-[oxo-1(mercaptométhyl)-2(éthoxy-4 phényl)-3 propyl]-(S)-alanine,
- la N-(E)-[oxo-1 mercaptométhyl)-2 ène-2 phényl-3 propyl]-(S)-alanine,
- la N-(E)-[oxo-1 (mercaptométhyl)-2 ène-2 phényl-3 propyl]-(S)-norvaline,

- la N-(E)-[oxo-1 (mercaptométhyl)-2 ène-2 phényl-3 propyl]-(S)-norleucine,
- la N-(E)-[oxo-1 (mercaptométhyl)-2 ène-2 phényl-3 propyl]-(RS)-(méthylènedioxy-3,4 phényl)-3 alanine,
- la N-(Z)-[oxo-1(mercaptométhyl)-2ène-2(méthylènedioxy-3,4 phényl)-3 propyl]glycine,
- le chlorhydrate de N-[N-(RS)-(carboxy-1 pentyl)-(RS)-(méthylènedioxy-3,4 phényl)-3 alanyl]-glycine,
- le chlorhydrate de N-[N-(RS)-(carboxy-1 phényl-2 éthyl)-(S)-phénylalanyl]-glycine,
- le chlorhydrate de N-[N-(RS)-(carboxy-1 phényl-2 éthyl)-(RS)-(méthylènedioxy-3,4 phényl)-3 alanyl]-glycine,
- la N-(RS)-[(dihydroxyphosphinyl) méthyl-2 oxo-1 (méthylènedioxy-3,4 phényl)-3 propyl]-(S)-alanine et son monosel de calcium,
- la N-(RS)-[(dihydroxyphosphinyl) méthyl-2 oxo-1 (méthylènedioxy-3,4 phényl)-3 propyl]-glycine et son monosel de calcium,
- la N-(RS)-[(dihydroxyphosphinyl) méthyl-2 oxo-1 (phényl-4 phényl)-3 propyl]-(S) alanine et son monosel de calcium,
- la N-(RS)-[(dihydroxyphosphinyl) méthyl-2 oxo-1 (phényl-4 phényl)-3 propyl]-glycine et son monosel de calcium.

**4.** Procédé de préparation d'amino-acides de formule (Ib) dans laquelle $R_1$ et $R_2$ ont la signification donnée dans la revendication 1 et X représente un groupe mercaptométhyle, caractérisé en ce qu'il consiste successivement

a) à effectuer une bromation allylique d'un acide éthylénique (E) de formule (VIII)

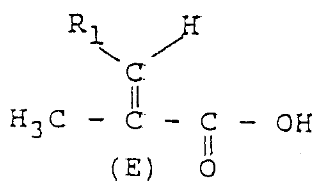

(VIII)

dans laquelle $R_1$ a la signification précitée, avec un agent de bromation tel que le N-bromo-succinimide, en présence d'une quantité catalytique de péroxyde de benzoyle, pour former un acide de formule (IX)

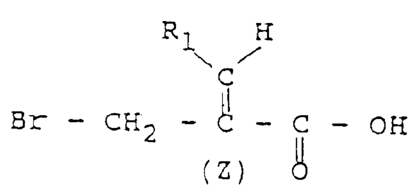

(IX)

b) à substituer le brome de l'acide éthylénique de formule (IX) par l'acide thioacétique pour former l'acide éthylénique thioacétylé (Z) de formule (X)

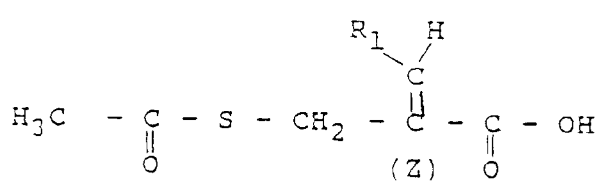

(X)

c) à isomériser l'acide de formule (X)

puis à séparer le mélange d'isoméres (E/Z) obtenu par une amine telle que la cyclohexylamine pour obtenir l'acide éthylénique thioacétylé (E) de formule (XI)

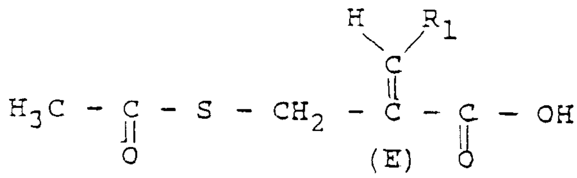

(XI)

d) à coupler l'acide éthylénique thioacétylé (E) de formule (XI), avec l'amino-ester désiré de formule (VI) en présence d'un agent de couplage tel que le dicyclohexylcarbadiimide pour obtenir le composé de formule (XII)

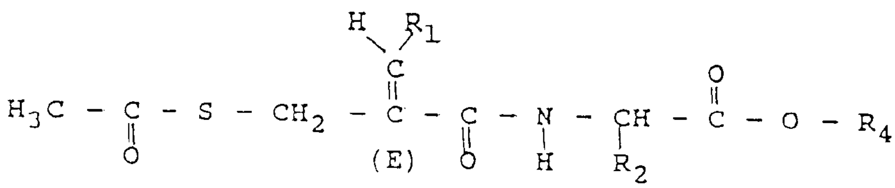

(XII)

e) puis à soumettre le composé de formule (XII) a une déprotection alcaline pour former les inhibiteurs mixtes de formule (Ib)

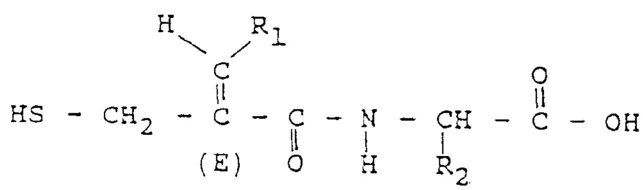

(Ib)

**5.** Procédé de préparation d'amino-acides de formule (Ia) dans alquelle $R_1$ et $R_2$ ont la signification donnée dans la revendication 1 et X représente le groupe N-carboxyalkyle, caractérisé en ce qu'il consiste successivement

a) à effectuer une diazotation puis une hydrolyse d'un amino-acide de formule (XIII)

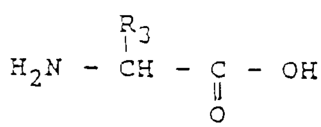

(XIII)

où $R_3$ a la définition précité, puis à former un hydroxyacide de formule (XIV)

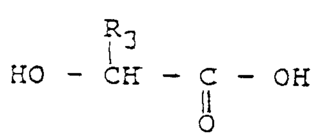

(XIV)

b) à effectuer une protection du groupe hydroxy du composé de formule (XIV) avec le chlorure d'acétyle pour former le composé de formule (XV)

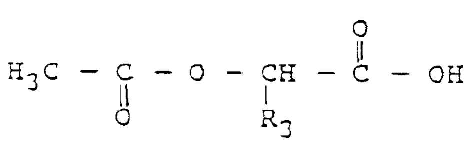

(XV)

c) à estérifier le composé de formule (XV), plus particulièrement par le tertiobutanol en présence d'oxychlorure de phosphore pour former l'ester de formule (XVI)

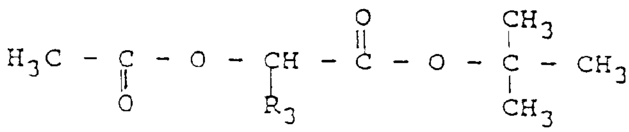 (XVI)

d) à libérer le groupe acétyle du composé (XVI) par une déprotection alcaline pour former l'hydroxyester de formule (XVII)

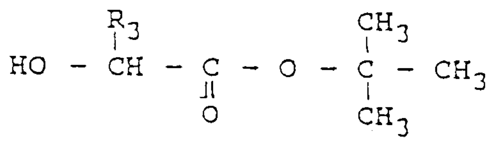 (XVII)

e) à activer la fonction alcool du composé de formule (XVII), par exemple par l'anhydride de trifluorométhane sulfonique, en présence de pyridine, pour former le composé de formule (XVIII)

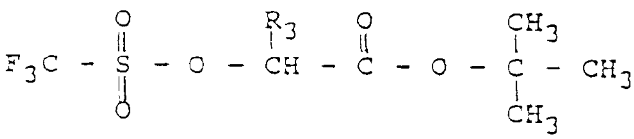 (XVIII)

f) à substituer le groupe trifluorométhanesulfonique du composé de formule (XVIII) par un amino-ester de formule (XIX) :

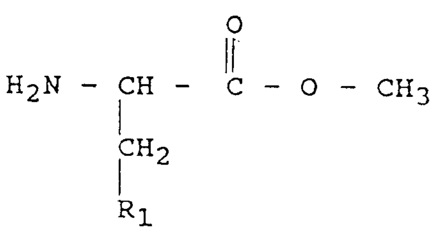 (XIX)

où $R_1$ a la définition précitée, en présence de bis (diméthylamino)-1,8 naphtalène, pour former le composé de formule (XX) :

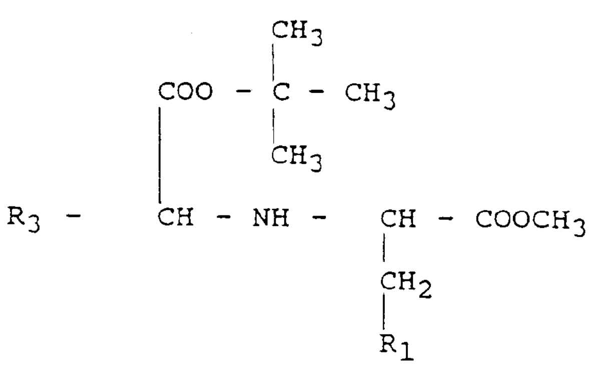 (XX)

g) à soumettre le composé de formule (XX) à une déprotection alcaline sélective pour former le composé de formule (XXI) :

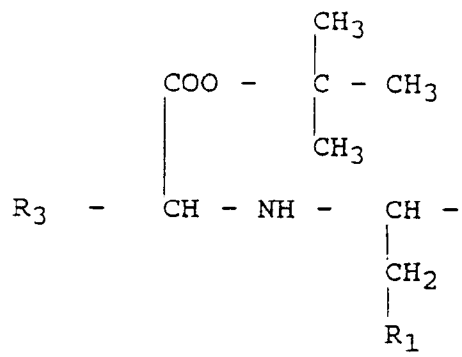

(XXI)

h) à coupler l'acide de formule (XXI) avec l'amino-ester désiré de formule (VI) où $R_4$ est un groupe benzyle, en présence d'un agent de couplage tel que le dicyclohexylcarbodiimide pour former le composé de formule (XXII) :

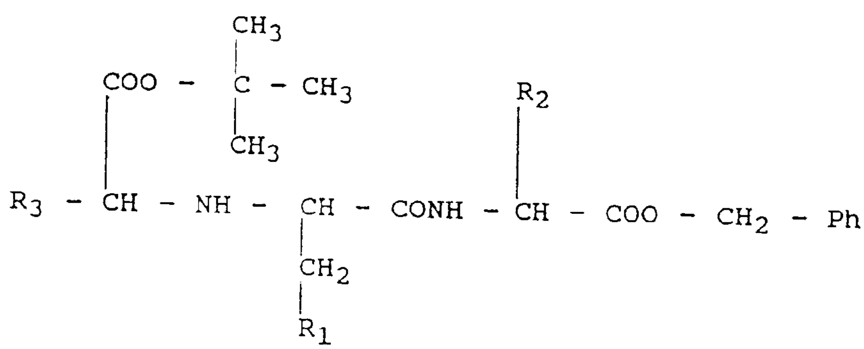

(XXII)

i) à hydrogéner le composé de formule (XXII) pour former le composé de formule (XXIII)

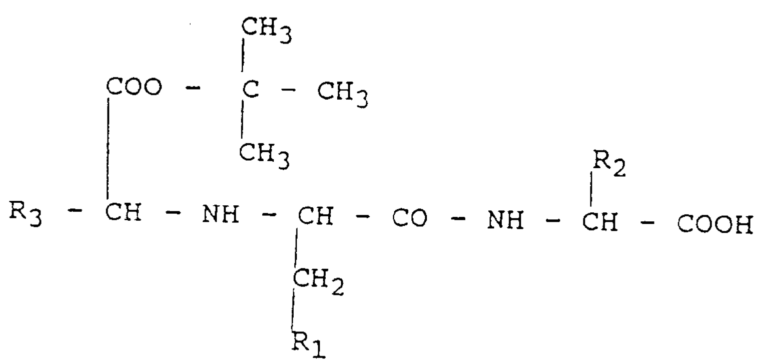

(XXIII)

j) puis, à hydrolyser la fonction ester tertiobulytique du composé de formule (XXIII), pour former le diacide de formule (Ia)

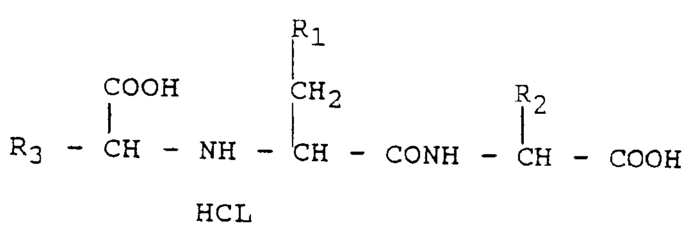

(Ia)

6. Procédé de préparation d'amino-acides de formule (Ia), dans laquelle $R_1$ et $R_2$ ont la signification donnée

dans la revendication 1 et X représente le groupe phosphonate, caractérisé en ce qu'il consiste successivement

a) à saponifier l'ester acrylique de formule (IV),

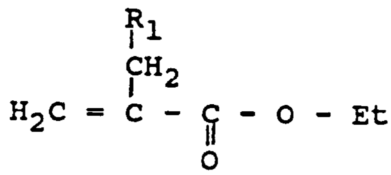

(IV)

et à faire suivre la saponification par l'action du chlorure de thionyle pour former le chlorure d'acide acrylique de formule (XXIV)

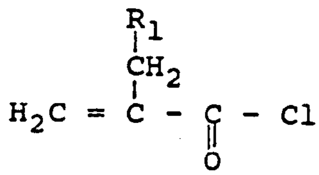

(XXIV)

b) à coupler le chlorure d'acide de formule (XXIV) avec l'amino-ester désiré de formule (VI), en présence par exemple de triéthylamine pour former le composé de formule (XXV)

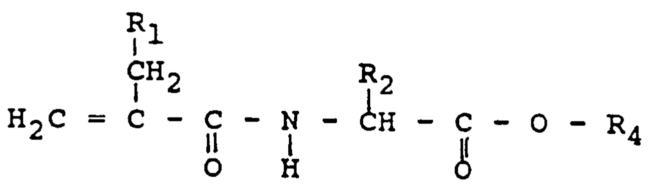

(XXV)

c) à effectuer une addition de Michaël avec un dialkylphosphite, par exemple avec le diéthylphosphite en présence d'hydrure de sodium pour former le composé de formule (XXVI)

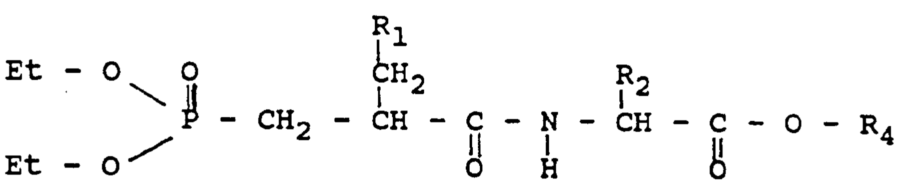

(XXVI)

d) puis à hydrolyser les fonctions protectrices du composé (XXVI) pour former les inhibiteurs de formule (Ia)

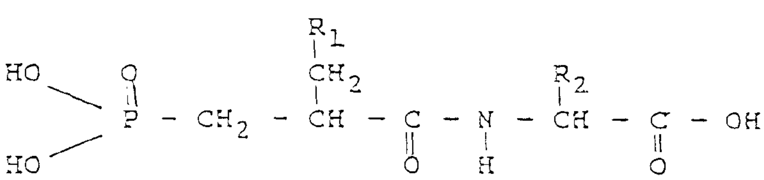

(Ia)

7. Dérivés d'amino-acides de formule générale (Ia) ou (Ib) dans laquelle $R_1$, $R_2$, X et n ont les significations données dans la revendication 1, caractérisés en ce qu'ils présentent une activité inhibitrice des enzymes, enképhalinase et ACE, exprimée en concentration inhibitrice $IC_{50}$, inférieure à 10 nM.

8. Dérivés d'amino-acides selon la revendication 7, caractérisés en ce que, lorsque la concentration inhibitrice $IC_{50}$ pour les deux activités est comprise entre 1 et 10 nM, le rapport de la concentration inhibitrice $IC_{50}$ de l'enképhalinase sur la concentration inhibitrice $IC_{50}$ de l'ACE est inférieur à 4, pour que le dérivé

soit équipotent.

**9.** Dérivés d'amino-acides de formule générale (Ia) ou (Ib) dans laquelle X représente le groupe mercaptométhyle, $R_1$ et $R_2$ ont les significations données dans la revendication 1, caractérisés en ce qu'ils sont utilisés après protection des fonctions mercaptométhyle et carboxylique.

**10.** Dérivés d'amino-acides selon la revendication 9, caractérisés en ce qu'ils répondent aux formules générales.

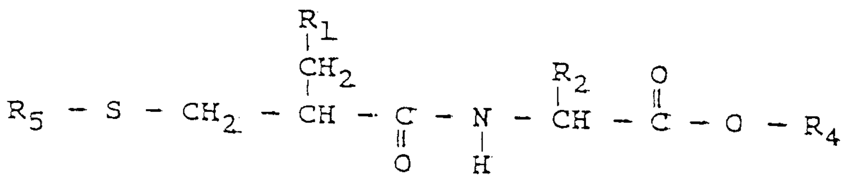

ou

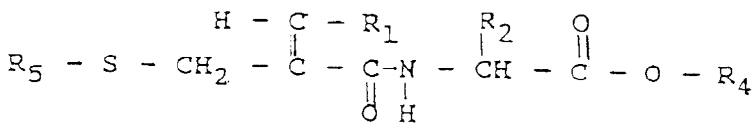

où $R_4$ représente plus particulièrement un groupe alkyle linéaire ou ramifié, un groupe phényle ou un groupe phénylalkyle, ces deux derniers groupes étant éventuellement mono- ou polysubstitués sur le noyau phényle, ou des substituants linéaires ou ramifiés comportant un ou plusieurs atomes d'oxygène et où $R_5$ représente, plus particulèrement, un radical acyle aliphatique linéaire ou ramifié, un radical acyle aromatique éventuellement mono- ou polysubstitué ou un radical acyle linéaire ou ramifié comportant un ou plusieurs atomes d'oxygène.

**11.** Médicament présentant une activité inhibitrice des enzymes enképhalinase et ACE, caractérisé en ce qu'il contient, à titre de principe actif, un composé tel que défini dans l'une des revendications 1, 2 et 3.

**12.** Utilisation des composés de formules principales

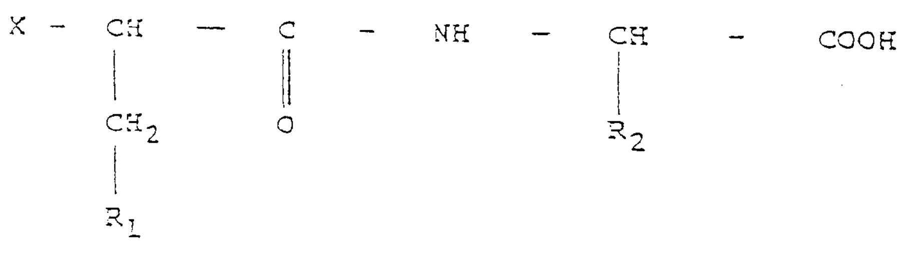

(Ia)

ou

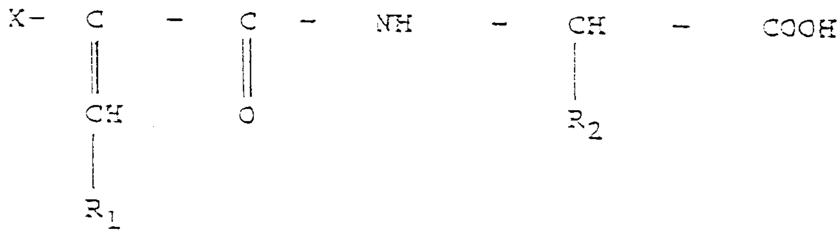

(Ib)

dans laquelle $R_1$ représente pour la formule (Ia) un groupe phényle mono- ou polysubstitué par un atome d'halogène, notamment le fluor ; un groupe biphényle ou l'un des groupements suivants :

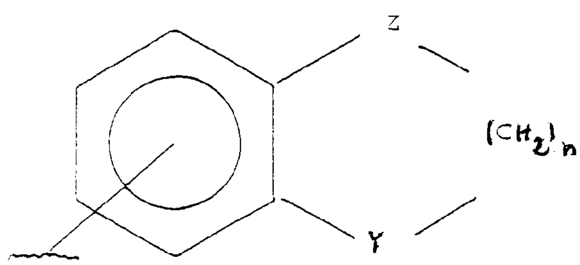

où Z, Y et n ont la signification donnée ci-dessous :

| Z | Y | n |
|---|---|---|
| O | O | 1 |
| O | $CH_2$ | 1 |
| $CH_2$ | $CH_2$ | 1 |
| O | O | 2 |
| $CH_2$ | $CH_2$ | 2 |
| O | $CH_2$ | 2 |

ou

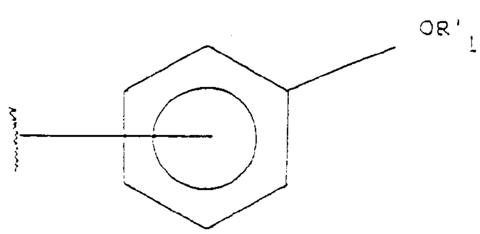

où

$R'_1$ représente un atome d'hydrogène, un groupe alkyle inférieur ; un groupe phényle ; un groupe phénylalkylène inférieur.

Pour la formule (Ib), $R_1$ a la définition précitée et peut être également un groupe phényle,

$R_2$ représente un atome d'hydrogène ; un groupe alkyle inférieur ; un groupe hydroxyalkylène inférieur ; un groupe phényle ; un groupe phénylalkyléne inférieur ; un groupe hydroxyphénylalkylène inférieur ; un groupe aminoalkylène inférieur ; un groupe guanidinoalkylène inférieur ; un groupe mercaptoalkylène inférieur ; un groupe alkyle inférieur thioalkylène inférieur ; un groupe imidazolylalkylène inférieur ; un groupe indolylalkylène inférieur ; un groupe carbamylalkylène inférieur ; un groupe carboxyalkylène inférieur ou l'un des groupements suivants :

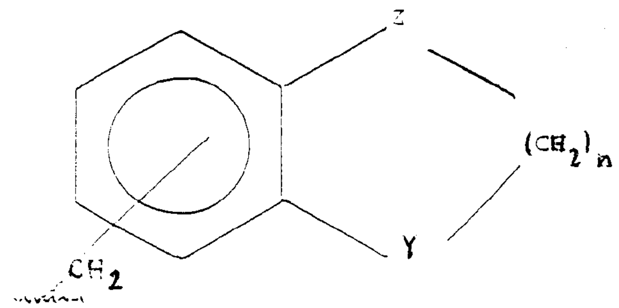

où Z et n ont les significations données ci-dessous :

| Z | Y | n |
|---|---|---|
| O | O | 1 |
| O | O | 2 |
| O | $CH_2$ | 1 |
| O | $CH_2$ | 2 |
| $CH_2$ | $CH_2$ | 1 |
| $CH_2$ | $CH_2$ | 2 |

X désigne un groupement responsable de la chélation de l'atome de zinc des enzymes, enképhalinase et ACE, et peut être choisi dans le groupe constitué par un mercaptométhyle ; l'acide hydroxamique ; un N-carboxyalkyle de formule

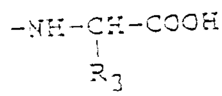

$R_3$ représente un radical alkyle inférieur, un radical alkyle inférieur benzyle ou un radical alcoxy inférieur benzyle des dérivés phosphorés de formule

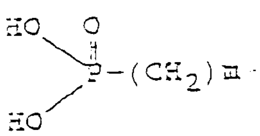 ou 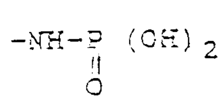

m = 0 ou 1
et des sels d'addition pharmaceutiquement acceptables de ces composés,
pour la préparation d'un médicament agissant en tant qu'inhibiteur mixte des enzymes enképhalinase et ACE.

**13.** Utilisation des composés selon la revendication 12, caractérisé en ce que lesdits composés sont choisis parmi :
- la N-(RS)-[oxo-1 (mercaptométhyl)-2 (fluoro-3 phényl)-3 propyl]-glycine,
- la N-(RS)-[oxo-1 (mercaptométhyl)-2 (fluoro-3 phényl)-3 propyl]-(S)-alanine,

- la N-(RS)-[oxo-1 (mercaptométhyl)-2 (difluoro-3,4 phényl)-3 propyl]-(S)-alanine,
- la N-(RS)-[oxo-1 (mercaptométhyl)-2 (difluoro-3,5 phényl)-3 propyl]-glycine et ses formes optiquement pures;
- la N-(RS)-[oxo-1 (mercaptométhyl)-2 (difluoro-3,5 phényl)-3 propyl]-(S)-alanine.

**14.** Utilisation des composés de formule (Ia) ou (Ib) selon la revendication 12 dans laquelle X représente un groupe mercaptométhyle et $R_1$ et $R_2$ ont les significations données dans la revendication 12, caractérisée en ce que lesdits composés sont mis en oeuvre après protection des fonctions mercaptométhyle et carboxylique.

**15.** Utilisation des composés selon la revendication 14, caractérisée en ce qu'ils répondent aux formules générales données dans la revendication 10, dans lesquelles $R_1$ et $R_2$ ont les significations données dans la revendication 12 et $R_4$ et $R_5$ ont les significations données dans la revendication 10.

## Claims

**1.** Amino acid derivatives characterized in that they correspond to the general formula :

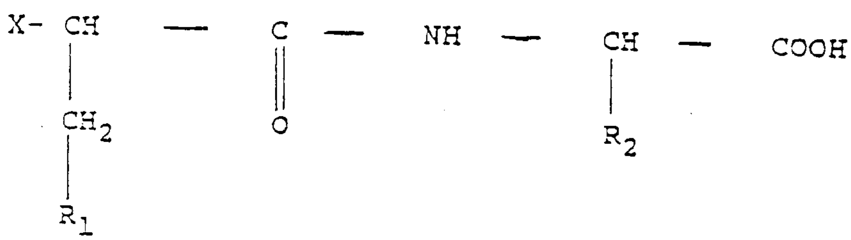

(Ia)

or

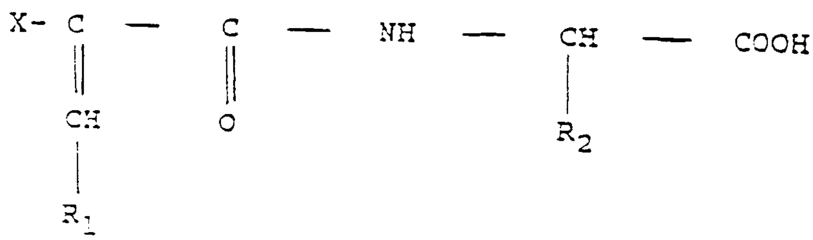

(Ib)

wherein $R_1$ represents in formula (Ia), a biphenyl group or one of the following groups :

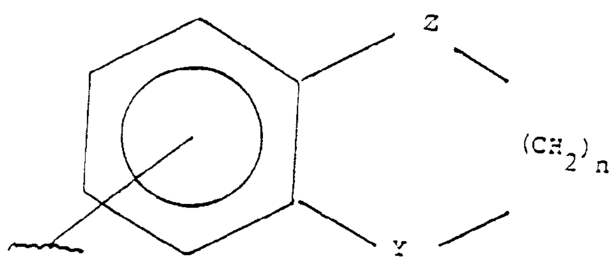

wherein Z, Y and n have the meanings defined below :

| Z | Y | n |
|---|---|---|
| O | O | 1 |
| O | $CH_2$ | 1 |
| $CH_2$ | $CH_2$ | 1 |
| O | O | 2 |
| $CH_2$ | $CH_2$ | 2 |
| O | $CH_2$ | 2 |

or

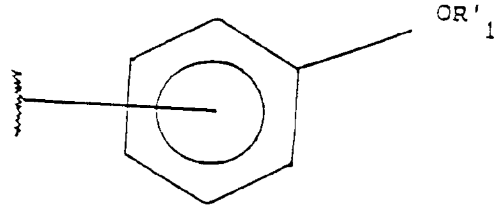

wherein R′, represents a lower alkyl group, a lower phenylalkylene group.

In formula (Ib), $R_1$ has the meaning defined hereinabove and can be also a phenyl group,

$R_2$ represents a hydrogen atom, a lower alkyl group, a lower hydroxyalkylene group, a phenyl group, a lower phenylalkylene group, a lower hydroxyphenylalkylene group, a lower aminoalkylene groupe, a lower guanidinoalkylene group, a lower mercaptoalkylene group, a lower alkyl lower thioalkylene group, a lower imidazolylalkylene group, a lower indolylalkylene group, a lower carbamylalkylene group, a lower carboxyalkylene group or one of the following groups :

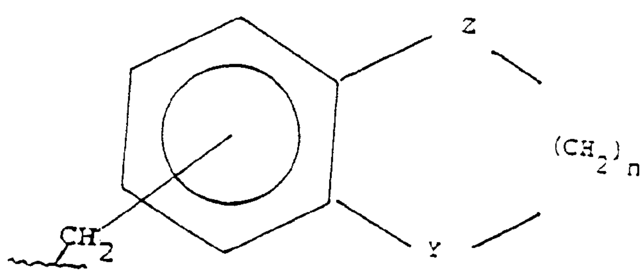

wherein Z, Y and n have the meanings defined below :

| Z | Y | n |
|---|---|---|
| O | O | 1 |
| O | O | 2 |
| O | $CH_2$ | 1 |
| $CH_2$ | $CH_2$ | 1 |
| $CH_2$ | $CH_2$ | 2 |

X designates a group responsible for chelating the zinc atom of the enzymes, enkephalinase and ACE, and can be chosen from the group consisting of a mercaptomethyl, an N-carboxyalkyl of formula

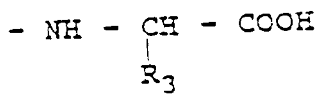

wherein $R_3$ represents a lower alkyl radical, a lower alkyl benzyl radical or a lower alkoxy benzyl radical, phosphorated derivatives of formula

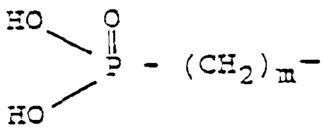

or

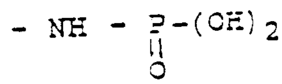

m = 0 or 1

with the proviso that, when in formula (5a), X represents a mercaptomethyl group, $R_1$ does not simultaneously represent a phenyl group substituted with a ($C_1$ - $C_3$) alkoxy group and $R_2$ a ($C_1$ - $C_2$) alkyl ($C_1$ - $C_3$) thioalkylene, and the pharmaceutically acceptable addition salts thereof.

**2.** Amino acid derivatives according to claim 1, characterized in that X represents the mercaptomethyl group.

**3.** Amino acid derivatives according to claim 2, characterized in that they are chosen from :

- N-(RS)-[1-oxo- 2-(mercaptomethyl)- 3-(3,4-methylenedioxy phenyl) propyl] glycine and its optically pure forms,
- N-(RS)-[1-oxo- 2-(mercaptomethyl)- 3-(3,4-methylenedioxy phenyl) propyl]-(S)-alanine and its optically pure forms,
- N-(RS)-[1-oxo- 2-(mercaptomethyl)- 3-(3,4-methylenedioxy phenyl) propyl]-(S)-2-aminobutyric acid,
- N-(RS)-[1-oxo-- 2-(mercaptomethyl)- 3-(3,4-methylenedioxy phenyl) propyl]-(S)-norvaline,
- N-(RS)-[1-oxo - 2-(mercaptomethyl)- 3-(3,4-methylenedioxy phenyl) propyl]-(S)-norleucine,
- N-(RS)-[1-oxo- 2-(mercaptomethyl)- 3-(3,4-methylenedioxy phenyl) propyl]-(S)-leucine,
- N-(RS)-[1-oxo- 2-(mercaptomethyl)- 3-(3,4-methylenedioxy phenyl) propyl]-(S)-tryptophan,
- N-(RS)-[1-oxo- 2-(mercaptomethyl)- 3-(3,4-methylenedioxy phenyl) propyl]-(S)-phenylalanine,
- N-(RS)-[1-oxo- 2-(mercaptomethyl)- 3-(3,4-methylenedioxy phenyl) propyl]-(S)-tyrosine,
- N-(S)[1-oxo 2-(mercaptomethyl)- 3-(3,4-methylenedioxy phenyl) propyl]-(S)-serine
- N-(S)-[1-oxo - 2-(mercaptomethyl)- 3-(3,4-methylenedioxy phenyl) propyl]-(S)-methionine,
- N-(S)-[1-oxo- 2-(mercaptomethyl)- 3-(3,4-methylenedioxy phenyl) propyl]-(RS)-methionine sulfoxide,
- N-(S)-[1-oxo- 2-(mercaptomethyl) - 3-(3,4-methylenedioxy phenyl) propyl]-(RS)-3-(3,4-methylenedioxy phenyl)-alanine,
- N-(RS)-[1-oxo- 2-(mercaptomethyl)- 3-(3,4-ethylenedioxy phenyl) propyl]-glycine,
- N-(RS)-[1-oxo- 2-(mercaptomethyl)- 3-(3,4-ethylenedioxy phenyl) propyl]-(S)-alanine,
- N-(RS)-[1-oxo- 2-(mercaptomethyl)- 3-(2,3-methylenedioxy phenyl) propyl]-glycine,
- N-(RS)-[1-oxo- 2-(mercaptomethyl)- 3-(4-phenoxy phenyl) propyl]-glycine,
- N-(RS)-[1-oxo-2-(mercaptomethyl)-3-(4-phenoxy phenyl) propyl]-(S)-alanine,
- N-(RS)-[1-oxo-2-(mercaptomethyl)--3-(4-phenyl phenyl) propyl]-glycine and its optically pure forms,
- N-[1-oxo-2-(mercaptomethyl)-3-(4-phenyl phenyl) propyl]-(S)-alanine and its optically pure forms,
- N-[1-oxo-2-(mercaptomethyl)-3-(4-phenyl phenyl) propyl]-(S)-leucine,
- N-(RS)-(1-oxo-2-(mercaptomethyl)-3-(5′-indanyl) propyl]-glycine,
- N-(RS)-[1-oxo-2-(mercaptomethyl)-3-(5′-indanyl) propyl]-(S)-alanine,
- N-(RS)-[1-oxo-2-(mercaptomethyl)-3-(2′,3′-dihydro-5′-benzofuranyl) propyl]-glycine,
- N-(RS)-[1-oxo-2-(mercaptomethyl)-3-(2′,3′-dihydro-5′-benzofuranyl) propyl]-(S)-alanine,
- N-(RS)-[1-oxo- 2-(mercaptomethyl)- 3-(4-methoxy phenyl) propyl]-glycine,
- N-(RS)-[1-oxo- 2-(mercaptomethyl)- 3-(4-methoxy phenyl) propyl]-(S)-alanine,
- N-(S)-[1-axo- 2-(mercaptomethyl)- 3-(4-methoxy phenyl) propyl]-glycine,
- N-(S)-[1-oxo-2-(mercaptomethyl)- 3-(4-methoxy phenyl) propyl]-(S)-alanine,
- N-(RS)-[1-oxo- 2-(mercaptomethyl)- 3-(4-ethoxy phenyl) propyl]-glycine,
- N-(RS)-[1-oxo- 2-(mercaptomethyl)- 3-(4-ethoxy phenyl) propyl]-(S)-alanine,

- N-(E)-[1-oxo-2-(mercaptomethyl)-2-ene-3-phenyl propyl]-(S)-alanine,
- N-(E)-[1-oxo- 2-(mercaptomethyl)-2-ene- 3-phenyl propyl]-(S)-norvaline,
- N-(E)-[1-oxo- 2-(mercaptomethyl)-2-ene- 3-phenyl propyl]-(S)-norleucine,
- N-(E)-[1-oxo-2-(mercaptomethyl)-2-ene-3-phenyl propyl]-(RS)-3-(3,4-methylenedioxy phenyl)-alanine,
- N-(Z)-[1-oxo-2-(mercaptomethyl)-2-ene-3-(3,4-methylenedioxy phenyl) propyl]-glycine,
- N-[N-(RS)-(1-carboxy pentyl)-(RS)-3-(3,4-methylenedioxy phenyl) alanyl]-glycine hydrochloride,
- N-[N-(RS)(1-carboxy-2-phenyl ethyl) -(S)- phenylalanyl]-glycine hydrocloride,
- N-[N-(RS)-(1-carboxy-2-phenyl ethyl) -(RS)- 3-(3,4-methylenedioxy phenyl) alanyl]-glycine hydrochloride,
- N-(RS)-[(dihydroxyphosphinyl)-2-methyl-1-oxo-3-(3,4-methylenedioxy phenyl) propyl]-(S)-alanine and its calcium monosalt,
- N-(RS)-[(dihydroxyphosphinyl)-2-methyl-1-oxo-3-(3,4-methylenedioxy phenyl) propyl]-glycine and its calcium monosalt,
- N-(RS)-[(dihydroxyphosphinyl)-2-methyl-1-oxo-3-(4-phenyl phenyl) propyl]-(S)-alanine and its calcium monosalt,
- N-(RS)-[(dlhydroxyphosphinyl)-2-methyl-1-oxo-3-(4-phenyl phenyl) propyl]-glycine and its calcium monosalt.

**4.** Process for the preparation of amino acids of formula (Ib) wherein $R_1$ and $R_2$ have the meanings defined in claim 1 and X represents a mercaptomethyl group, characterized in that it successively consists in :

a) carrying out allylic bromination of an ethylenic acid (E) of formula (VIII)

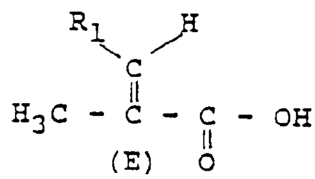

(VIII)

wherein $R_1$ has the meaning defined hereinabove, with a bromination agent such as N-bromo-succinimide, in the presence of a catalytic amount of benzoyl peroxide, in order to form an acid of formula (IX)

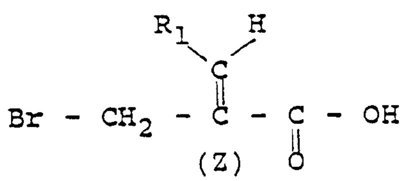

(IX)

b) substituting the bromine in ethylenic acid of formula (IX) with thioacetic acid in order to form thioacetylated (Z) ethylenic acid of formula (X)

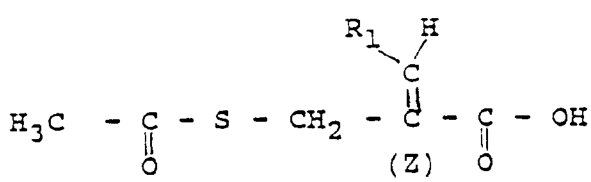

(X)

c) isomerizing the acid of formula (X) then separating the isomer mixture (E/Z) obtained with an amine, such as cyclohexylamine, in order to obtain thioacetylated (E) ethylenic acid of formula (XI)

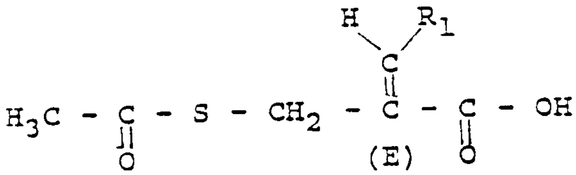

(XI)

d) coupling thioacetylated (E) ethylenic acid of formula (XI) with the desired aminoester of formula (VI) in the presence of a coupling agent such as dicyclohexylcarbodiimide, in order to obtain the compound of formula (XII)

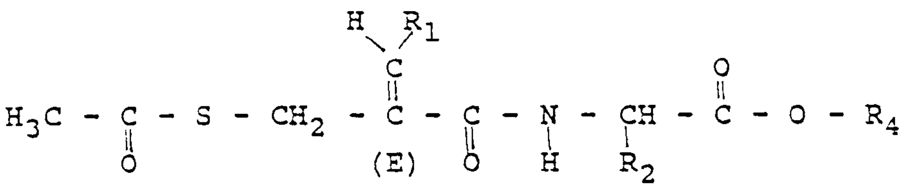

(XII)

e) then subjecting the compound of formula (XII) to alkaline deprotection in order to form the mixed inhibitors of formula (Ib)

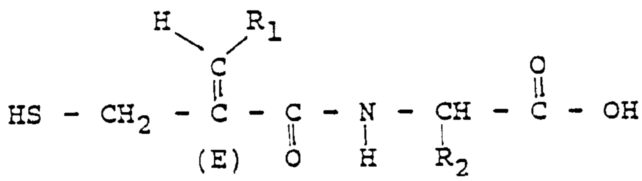

(Ib)

**5.** Process for the preparation of amino acids of formula (Ia) wherein $R_1$ and $R_2$ have the meanings defined in claim 1 and X represents a N-carboxyalkyl group, characterized in that it successively consists in :

a) carrying out diazotization then hydrolysis of an amino acid of formula (XIII)

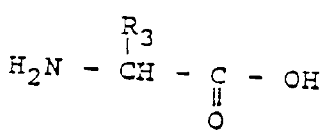

(XIII)

wherein $R_3$ has the meaning defined hereinabove, then forming a hydroxyacid of formula (XIV)

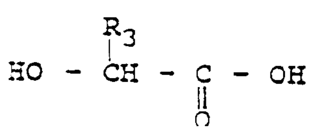

(XIV)

b) carrying out protection of the hydroxy group of the compound of formula (XIV) with acetyl chloride in order to form the compound of formula (XV)

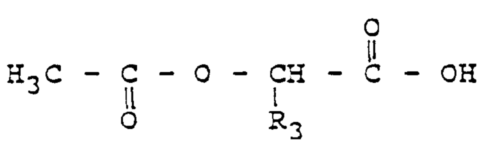

(XV)

c) esterifying the compound of formula (XV), more particularly with tertiary butanol in the presence of

phosphorous oxychloride, in order to form the ester of formula (XVI)

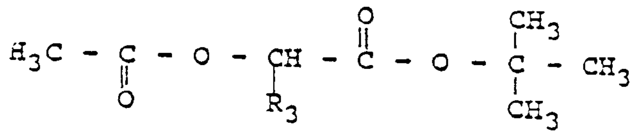

(XVI)

d) releasing the acetyl group of compound (XIV) by alkaline deprotection in order to form the hydroxyester of formula (XVII)

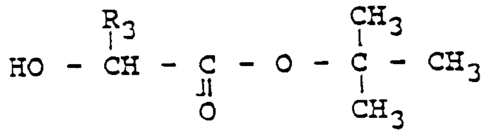

(XVII)

e) activating the alcohol function of the compound of formula (XVII), for example with trifluoromethane sulfonic anhydride, in the presence of pyridine, in order to form the compound of formula (XVIII)

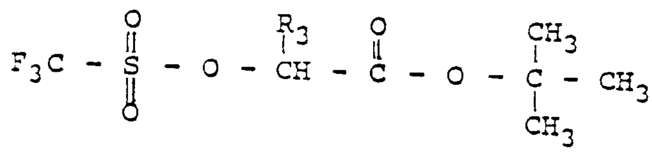

(XVIII)

f) substituting the trifluoromethane sulfonic group of the compound of formula (XVIII) with an aminoester of formula (XIX)

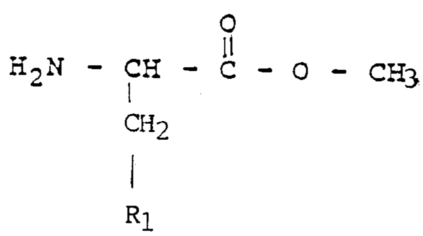

(XIX)

wherein $R_1$ has the meaning defined hereinabove, in the presence of bis-1,8-(dimethylamino)-naphthalene in order to form the compound of formula (XX) :

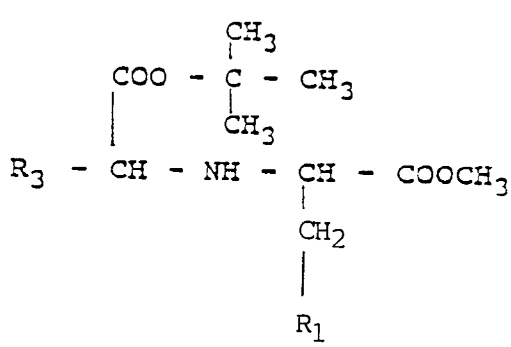

(XX)

g) subjecting the compound of formula (XX) to selective alkaline deprotection in order to form the compound of formula (XXI) :

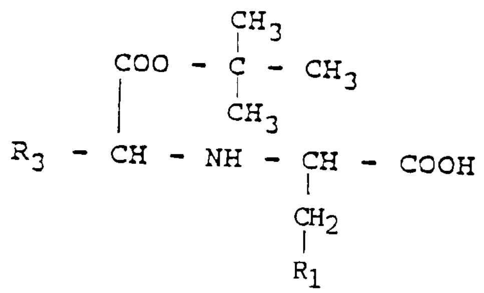

(XXI)

h) coupling the acid of formula (XXI) with the desired aminoester of formula (VI) wherein $R_4$ is a benzyl group, in the presence of a coupling agent such as dicyclohexylcarbodiimide in order to form the compound of formula (XXII)

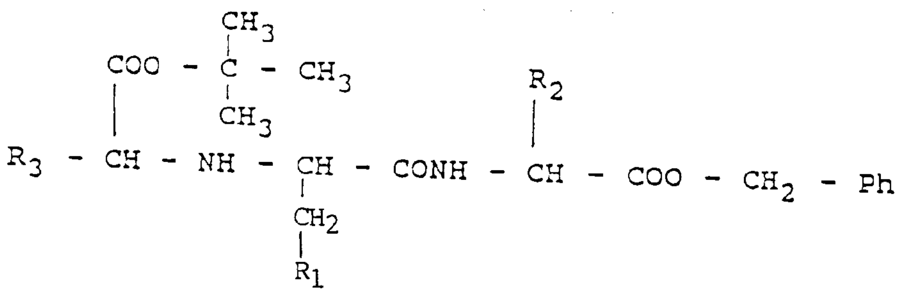

(XXII)

i) hydrogenating the compound of formula (XXII) in order to form the compound of formula (XXIII)

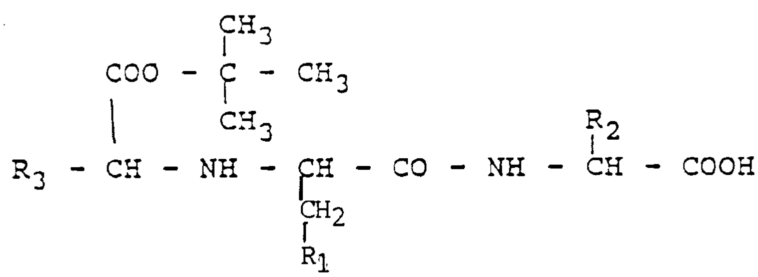

(XXIII)

j) then hydrolyzing the tertiary butyl ester function of the compound of formula (XXIII) in order to form the diacid of formula (Ia)

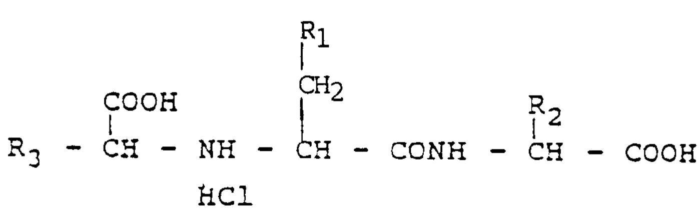

(Ia)

**6.** Process for the preparation of amino acids of formula (Ia) wherein $R_1$ and $R_2$ have the meanings defined in claim 1 and X represents a phosphonate group, characterized in that it successively consists in :

a) saponifying the acrylic ester of formula (IV),

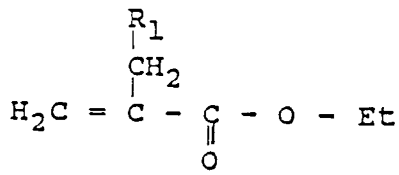 (IV)

and following saponification with the addition of thionyl chloride in order to form the acrylic acid chloride of formula (XXIV)

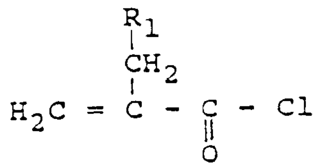 (XXIV)

b) coupling the acid chloride of formula (XXIV) with the desired aminoester of formula (VI), in the presence of triethylamine for example, in order to form the compound of formula (XXV)

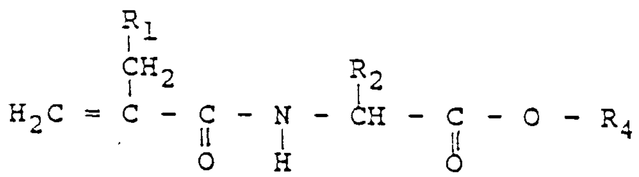 (XXV)

c) carrying out a Michaël addition with a dialkylphosphite, for example with diethylphosphite, in the presence of sodium hydride in order to form the compound of formula (XXVI)

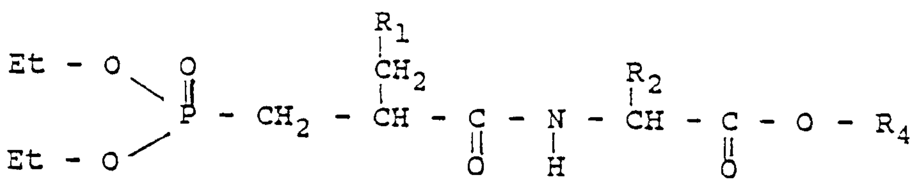 (XXVI)

d) then hydrolyzing the protective functions of compound (XXVI) in order to form the inhibitors of formula (Ia)

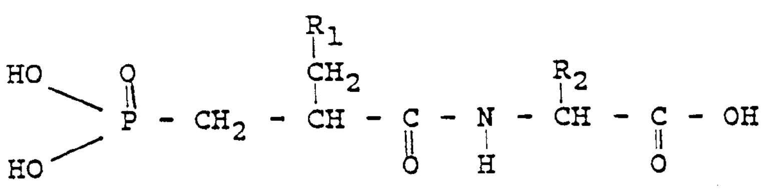 (Ia)

**7.** Amino acid derivatives of general formula (Ia) or (Ib) wherein $R_1$, $R_2$, X and n have the meanings defined in claim 1 characterized in that they show inhibiting activity of the enzymes enkephalinase and ACE, expressed as 50 % inhibiting concentration $IC_{50}$, lower than 10 nM.

**8.** Amino acid derivatives according to claim 7 characterized in that, when the inhibiting concentration $IC_{50}$ for the two activitites ranges from 1 to 10 nM, the ratio of enkephalinase-inhibiting concentration $IC_{50}$ to the ACE-inhibiting concentration $IC_{50}$ is lower than 4 for the compound to be equipotent.

**9.** Amino acid derivatives of general formula (Ia) or (Ib) wherein X represents the mercaptomethyl group, $R_1$, $R_2$ have the meanings defined in claim 1, characterized in that they are used after protection of the mercaptomethyl and carboxyl functions has been carried out.

**10.** Amino acid derivatives according to claim 9, characterized in that they correspond to the general formulae

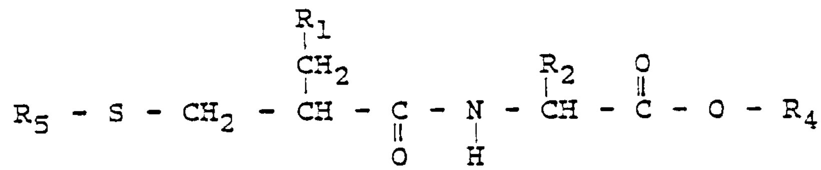

or

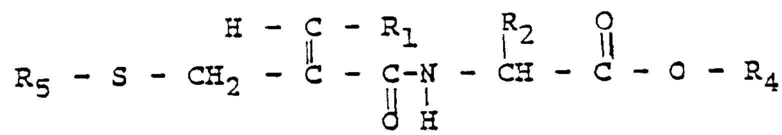

wherein $R_4$ more particularly represents a linear or branched alkyl group, a phenyl group or a phenylalkyl group, the two latter groups possibly being mono- or polysubstituted on the phenyl ring, or linear or branched substituents including one ore more oxygen atoms and wherein $R_5$ more particularly represents a linear or branched aliphatic acyl radical, an aromatic acyl radical possibly mono- or polysubstituted, or a linear or branched acyl radical including one or more oxygen atoms.

**11.** Drug showing enkephalinase- and ACE-inhibiting activities characterized in that it contains a compound as defined in one of claims 1, 2 and 3 as the active principle.

**12.** Use of the compounds of general formulae

I(a)

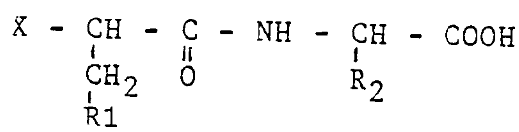

or

I(b)

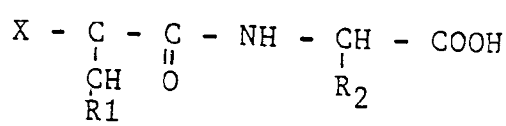

wherein $R_1$ represents, in formula (Ia), a phenyl group mono- or polysubstituted with a halogen atom, in particular fluorine ; a biphenyl group or one of the folowing groups :

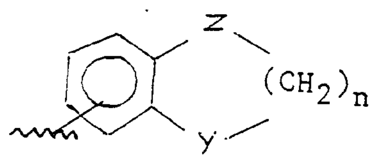

wherein Z, Y and N have the meanings defined below

| Z | Y | n |
|---|---|---|
| O | O | 1 |
| O | $CH_2$ | 1 |
| $CH_2$ | $CH_2$ | 1 |
| O | O | 2 |
| $CH_2$ | $CH_2$ | 2 |
| O | $CH_2$ | 2 |

or

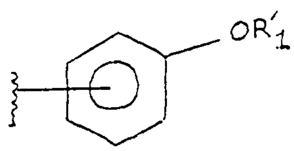

OR'1

wherein

$R'_1$ represents as hydrogen atom, a lower alkyl group ; a phenyl group ; a lower phenylalkylene group.

In formula (Ib), $R_1$ has the meaning defined herein above and can be also a phenyl group, $R_2$ represents a hydrogen atom ; a lower alkyl group ; a lower hydroxyalkylene group ; a phenyl group ; a lower phenylalkylene group ; a lower hydroxyphenylalkylene group; a lower aminoalkylene group ; a lower guanidinoalkylene group, a lower mercapto alkylene group ; a lower alkyl lower thioalkylene group ; a lower imidazolylalkylene group; a lower indolylalkylene group ; a lower carbanylalkylene group ; as lower carboxyalkylene group or one of the following groups :

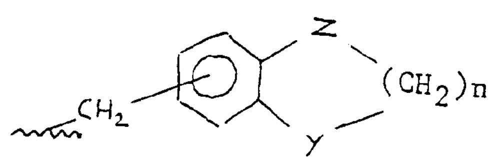

wherein Z, Y and n have the meanings defined below :

| Z | Y | n |
|---|---|---|
| O | O | 1 |
| O | O | 2 |
| O | $CH_2$ | 1 |
| O | $CH_2$ | 2 |
| $CH_2$ | $CH_2$ | 1 |
| $CH_2$ | $CH_2$ | 2 |

X designates a group responsible for chelating the zinc atom of the enzymes, enkephalinase and ACE, and can be chosen from the group consisting of a mercaptomethyl ; hydroxamic acid ; a N-carboxyalkyl of formula

$$- NH - \underset{R_3}{CH} - COOH$$

$R_3$ represents a lover alkyl radical, a lower alkyl benzyl radical or a lower alkoxy benzyl radical ; phosphorated derivatives of formula

$$\overset{HO\ \ O}{\ } \underset{HO}{\overset{\|}{P}} - (CH_2)_m - \quad ou \quad - NH - \underset{O}{\overset{\ }{\underset{\|}{P}}} (OH)_2 \qquad m = 0 \text{ ou } 1$$

and the pharmaceutically acceptable addition salts of the compounds, for preparing a drug acting as mixed inhibitors of the enkephalinase and ACE enzymes.

**13.** Use of the compounds according to claim 12, characterized in that said compounds are chosen from :
- N-(RS)-[1-oxo-2-(mercaptomethyl)-3-(3-fluoro phenyl) propyl]-glycine,
- N-(RS)-[1-oxo-2-(mercaptomethyl)-3-(3-fluoro phenyl) propyl]-(S)-alanine,
- N-(RS)-[1-oxo-2-(mercaptomethyl)-3-(3,4-difluoro phenyl) propyl]-(S)-alanine,
- N-(RS)-[1-oxo-2-(mercaptomethyl)-3-(3,5-difluoro phenyl) propyl]-glycine and its optically pure forms,
- N-(RS)-[1-oxo-2-(mercaptomethyl)-3- (3,5-difluoro phenyl) propyl]-(S)-alanine.

**14.** Use of the compounds of formula (Ia) or (Ib) according to claim 12, wherein X represents a mercaptomethyl group and $R_1$ and $R_2$ have the meanings defined in claim 12, characterized in that said compounds are used after protection of the mercaptomethyl and carboxyl functions has been carried out.

**15.** Use of the compounds according to claim 14, characterized in that they correspond to the general formulae given in claim 10, wherein $R_1$ and $R_2$ have the meanings defined in claim 12 and $R_4$ and $R_5$ have the meanungs defined in claim 10.

## Patentansprüche

**1.** Aminosäurederivate, gekennzeichnet durch die allgemeine Formel:

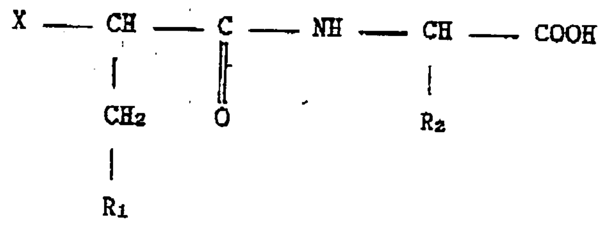

(Ia)

oder

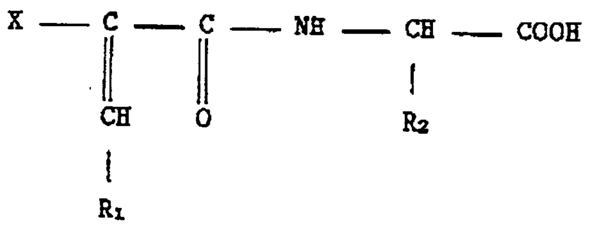

(Ib)

worin $R_1$ in der Formel (Ia) eine Biphenylgruppe oder eine der folgenden Gruppen bedeutet:

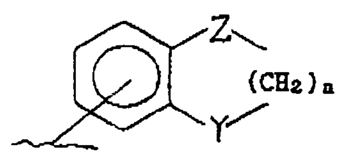

worin Z, Y und n die folgenden Bedeutungen aufweisen:

| Z | Y | n |
|---|---|---|
| O | O | 1 |
| O | $CH_2$ | 1 |
| $CH_2$ | $CH_2$ | 1 |
| O | O | 2 |
| $CH_2$ | $CH_2$ | 2 |
| O | $CH_2$ | 2 |

oder

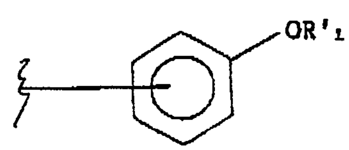

worin $R'_1$ eine niedere Alkylgruppe, eine niedere Phenylalkylengruppe bedeutet,

in der Formel (Ib) weist $R_1$ die zuvor definierte Bedeutung auf und kann darüber hinaus eine Phenylgruppe sein,

$R_2$ bedeutet ein Wasserstoffatom, eine niedrige Alkylgruppe, eine niedrige Hydroxyalkylengruppe, eine Phenylgruppe, eine niedrige Phenylalkylengruppe, eine niedrige Hydroxyphenylalkylengruppe, eine niedrige Aminoalkylengruppe, eine niedrige Guanidinoalkylengruppe, eine niedrige Mercaptoalkylengruppe, eine niedrige Alkyl-niedrige-Thioalkylengruppe, eine niedrige Imidazolylalkylengruppe, eine niedrige Indolylalkylengruppe, eine niedrige Carbamylalkylengruppe, eine niedrige Carboxyalkylengruppe oder eine der folgenden Gruppen:

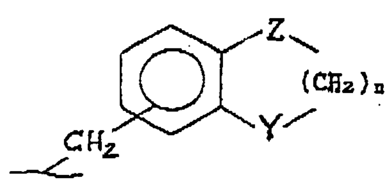

worin Z, Y unde n die folgenden Bedeutungen aufweisen:

| Z | Y | n |
|---|---|---|
| O | O | 1 |
| O | O | 2 |
| O | $CH_2$ | 1 |
| $CH_2$ | $CH_2$ | 1 |
| $CH_2$ | $CH_2$ | 2 |

X bedentet eine Gruppe, die für die Bildung eines Chelats mit dem Zinkatom der Enzyme Enkephalinase und ACE verantworlicht ist, und kann ausgewählt sein aus der Gruppe bestehend aus Mercaptmethyl, einem N-Carboxyalkyl der Formel

$$-NH-CH-COOH$$
$$|$$
$$R_3$$

worin $R_3$ steht für einen niedrigen Alkylrest, einen niedrigen Alkylbenzylrest oder einen niedrigen Alkoxybenzylrest, Phosphoderivate der Formel

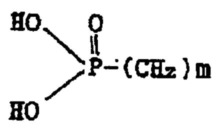 oder $-NH-\underset{\|}{P}-(OH)_2$
O

m = 0 oder 1

mit der Maßgabe, daß, wenn in der Formel (Ia) X eine Mercaptomethylgruppe ist, nicht gleichzeitig $R_1$ für eine mit einer $C_1$-$C_3$-Alkoxygruppe substituierte Phenylgruppe und $R_2$für eine $C_1$-$C_2$-Alkyl-$C_1$-$C_3$-Thioalkylengruppe stehen kann,

sowie deren pharmazeutisch verträgliche Additionssalze.

2. Aminosäurederivate nach Anspruch 1, dadurch gekennzeichnet, daß X für eine Mercaptomethylgruppe steht.

3. Aminosäurederivate nach Anspruch 2, dadurch gekennzeichnet, daß sie ausgewählt sind aus:
   - N-(RS)-[1-Oxo-2-(mercaptomethyl)-3-(3,4-Methylendioxy-phenyl)-propyl] glycin und dessen optisch reinen Formen,
   - N-(RS)-[1-Oxo-2-(mercaptomethyl)-3(3,4-methylendioxy-phenyl)-propyl]-(S)-alanin und dessen optisch reinen Formen,
   - N-(RS)-[1-Oxo-2-(mercaptomethyl)-3-(3,4-methylendioxy-phenyl)-propyl]-(S)-2-amino-buttersäure,
   - N-(RS)-[1-Oxo-2-(mercaptomethyl)-3-(3,4-methylendioxy-phenyl)-propyl]-(S)-norvalin,
   - N-(RS)-[1-Oxo-2-(mercaptomethyl)-3-(3,4-methylendioxy-phenyl)-propyl]-(S)-norleucin,
   - N-(RS)-[1-Oxo-2-(mercaptomethyl)-3-(3,4-methylendioxy-phenyl)-propyl]-(S)-leucin,
   - N-(RS)-[1-Oxo-2-(mercaptomethyl)-3-(3,4-methylendioxy-phenyl)-propyl]-(S)-tryptophan,
   - N-(RS)-[1-Oxo-2-(mercaptomethyl)-3-(3,4-methylendioxy-phenyl)-propyl]-(S)-phenylalanin,
   - N-(RS)-[1-Oxo-2-(mercaptomethyl)-3-(3,4-methylendioxy-phenyl)-propyl]-(S)-tyrosin,

- N-(S)-[1-Oxo-2-(mercaptomethyl)-3-(3,4-methylendioxy-phenyl)-propyl]-(S)-serin,
- N-(S)-[1-Oxo-2-(mercaptomethyl)-3-(3,4-methylendioxy-phenyl)-propyl]-(S)-methionin,
- N-(S)-[1-Oxo-2-(mercaptomethyl)-3-(3,4-methylendioxy-phenyl)-propyl]-(RS)-methioninsulfoxyd,
- N-(S)-[1-Oxo-2-(mercaptomethyl)-3-(3,4-methylendioxy-phenyl)-propyl]-(RS)-3-(3,4-methylendioxy-phenyl)-alanin,
- N-(RS)-[1-Oxo-2-(mercaptomethyl)-3-(3,4-ethylendioxy-phenyl)-propyl]-glycin,
- N-(RS)-[1-Oxo-2-(mercaptomethyl)-3-(3,4-ethylendioxy-phenyl)-propyl]-(S)-alanin,
- N-(RS)-[1-Oxo-2-(mercaptomethyl)-3-(2,3-methylendioxy-phenyl)-propyl]-glycin,
- N-(RS)-[1-Oxo-2-(mercaptomethyl)-3-(4-phenoxy-phenyl)-propyl]-glycin,
- N-(RS)-[1-Oxo-2-(mercaptomethyl)-3-(4-phenoxy-phenyl)-propyl]-(S)-alanin,
- N-(RS)-[1-Oxo-2-(mercaptomethyl)-3-(4-phenyl-phenyl)-propyl]-glycin und dessein optisch reinen Formen,
- N-[1-Oxo-2-(mercaptomethyl)-3-(4-phenyl-phenyl)-propyl]-(S)-alanin und dessen optisch reinen Formen,
- N-[1-Oxo-2-(mercaptomethyl)-3-(4-phenyl-phenyl)-propyl]-(S)-leucin,
- N-(RS)-[1-Oxo-2-(mercaptomethyl)-3-(5′-indanyl)-propyl]-glycin,
- N-(RS)-[1-Oxo-2-(mercaptomethyl)-3-(5′-indanyl)-propyl]-(S)-alanin,
- N-(RS)-[1-Oxo-2-(mercaptomethyl)-3-(2′,3′-dihydro-5′-benzofuranyl-propyl]-glycin,
- N-(RS)-[1-Oxo-2-(mercaptomethyl)-3-(1′,3′-dihydro-5′-benzofuranyl)-propyl]-(S)-alanin,
- N-(RS)-[1-Oxo-2-(mercaptomethyl)-3-(4-methoxyphenyl)-propyl]-glycin,
- N-(RS)-[1-Oxo-2-(mercaptomethyl)-3-(4-methoxyphenyl)-propyl]-(S)-alanin,
- N-(S)-[1-Oxo-2-(mercaptomethyl)-3-(4-methoxphenyl)-propyl]-glycin,
- N-(S)-[1-Oxo-2-(mercaptomethyl)-3-(4-methoxyphenyl)-propyl]-(S)-alanin,
- N-(RS)-[1-Oxo-2-(mercaptomethyl)-3-(4-ethoxyphenyl)-propyl]-glycin,
- N-(RS)-[1-Oxo-2-(mercaptomethyl)-3-(4-ethoxyphenyl)-propyl]-(S)-alanin,
- N-(E)-[1-Oxo-2-mercaptomethyl)-2-en-3-phenylpropyl]-(S)-alanin,
- N-(E)-[1-Oxo-2-mercaptomethyl)-2-en-3-phenylpropyl]-(S)-norvalin,
- N-(E)-[1-Oxo-2-mercaptomethyl)-2-en-3-phenylpropyl]-(S)-norleucin,
- N-(E)-[1-Oxo-2-mercaptomethyl)-2-en-3-phenylpropyl]-(RS)-3-(3,4-methylendioxy-phenyl)-alanin,
- N-(Z)-[1-Oxo-2-(mercaptomethyl)-2-en-3-(3,4-methylendioxy-phenyl)-propryl]-glycin,
- dem Chlorhydrat von N-[N-(RS)-(1-carboxy-pentyl)-(RS)-3-(3,4-methylendioxy-phenyl)-alanyl]-glycin,
- dem Chlorhydrat von N-[N-(RS)-(1-carboxy-2-phenylethyl)-(S)-phenyl-alanyl]-glycin,
- dem Chlorhydrat von N-[N-(RS)-(1-carboxy-2-phenylethyl)-(RS)-3-(3,4-methylendioxy-phenyl)-alanyl]-glycin,
- N-(RS)-[(Dihydroxyphosphinyl)-2-methyl-1-oxo-3(3,4-methylendioxy-phenyl)-propyl]-(S)-alanin und dessen Monocalciumsalz,
- N-(RS)-[(Dihydroxyphosphinyl)-2-methyl-1-oxo-3-(3,4-methylendixoxy-phenyl)-propyl]-glycin un dessen Monocalciumsalz,
- N-(RS)-[(Dihydroxyphosphinyl)-2-methyl-1-oxo-3-(4-phenyl-phenyl)-propyl]-(S)-alanin und dessen Monocalciumsalz,
- N-(RS)-[(Dihydroxyphosphinyl)-2-methyl-1-oxo-3-(4-phenyl-phenyl)-propyl]-glycin und dessen Monocalciumsalz.

**4.** Verfahren zur Herstellung von Aminosäuren der Formel (Ib), in denen $R_1$ und $R_2$ die in Anspruch 1 angegebene Bedeutung aufweisen und X eine Mercaptomethylgruppe bedeutet, dadurch gekennzeichnet, daß man nacheinander die folgenden Schritte ausführt:

a) Durchführen einer Allylbromierung einer Ethylensäure (E) der Formel (VIII)

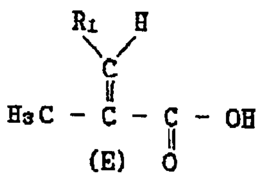

(VIII)

in der $R_1$ die zuvor definierte Bedeutung aufweist, mit einem Bromierungsmittel, wie N-Bromsuccinimid, in Gegenwart einer katalytischen Menge Benzoylperoxid, um eine Säure der Formel (IX) zu bilden

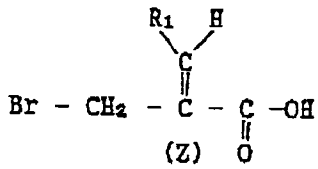 (IX)

b) Substituieren des Broms der Ethylensäure von Formel (IX) durch Thioessigsäure, ume eine Thioacetylethylensäure (Z) der Formel (X) zu bilden

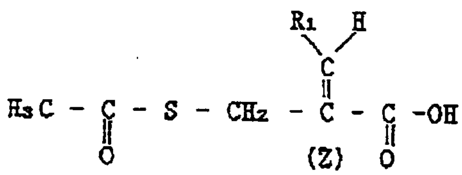 (X)

c) Isomerisieren der Säure der Formel (X), dann Auftrennen der so erhaltenen Isomerenmischung (E/Z) mittels eines Amins, wie Cyclohexylamin, um die Thioacetylethylensäure (E) der Formel (XI) zu erhalten

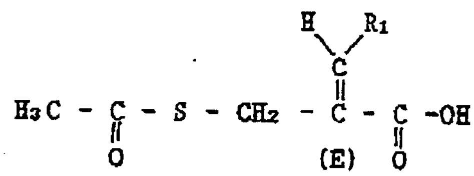 (XI)

d) Koppeln der thioacetylierten Ethylensäure (E) der Formel (XI) mit dem gewünschten Aminoester der Formel (VI) in Gegenwart eines Kopplungs- mittels, wie Dicyclohexylcarbodiimid, um eine Verbinding der Formel (XII) zu erhalten

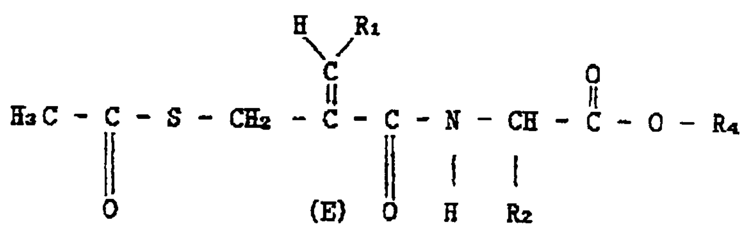 (XII)

e) dann die Verbindung der Formel (XII) einer alkalischen Entschützung unterzieht, um die Kombinationsinhibitoren der Formel (Ib) zu bilden

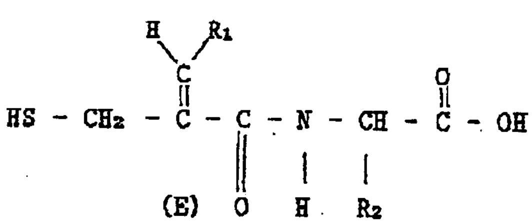 (Ib)

**5.** Verfahren zur Herstellung von Aminosäuren der Formel (Ia), in denen $R_1$ und $R_2$ die in Anspruch 1 angegebene Bedeutung aufweisen und X für eine N-Carboxyalkylgruppe steht, dadurch gekennzeichnet, daß man nacheinander

a) eine Diazotierung durchführt, dann eine Hydrolyse einer Aminosäure der Formel (XIII)

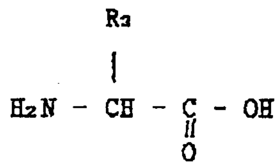 (XIII)

worin $R_3$ die zuvor definierte Bedeutung aufweist, dann eine Hydroxysäure der Formel (XIV) bildet

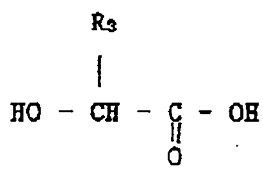 (XIV)

b) mittels Acetylchlorid die Hydroxygruppe der Verbindung der Formel (XIV) schützt, um die Verbindung der Formel (XV) zu bilden

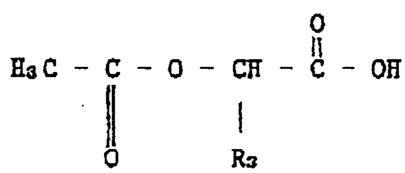 (XV)

c) die Verbindung der Formel (XV) im besonderen mittels Tertiärbutanol in Gegenwart von Phosphoroxychlorid verestert, um einen Ester der Formel (XVI) zu bilden:

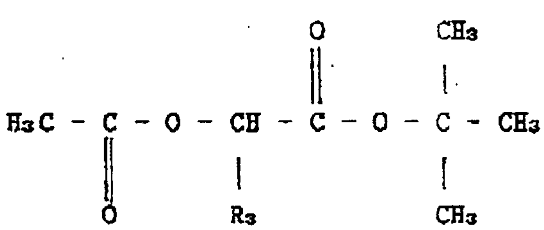 (XVI)

d) die Acetylgruppe der Verbindung (XVI) mittels eineralkalischen Entschützung freisetzt, um den Hydroxyester der Formel (XVII) zu bilden

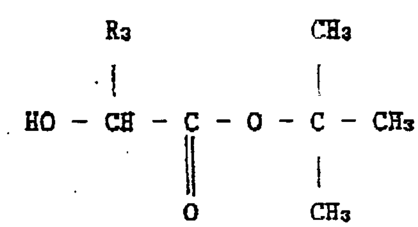 (XVII)

e) die Alkoholgruppe der Verbindung der Formel (XVII) beispielsweise mittels Trifluomethansulfonsäureanhydrid in Gegenwart von Pyridin akti- viert, um die Verbindnng der Formel (XVIII) zu bilden

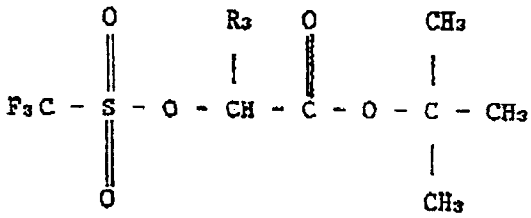

(XVIII)

f) die Trifluormethansulfonsäuregruppe der Verbindung der Formel (XVIII) mittels einem Aminoester der Formel (XIX):

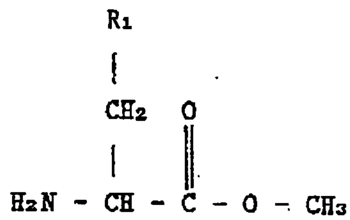

(XIX)

worin $R_1$ die zuvor definierte Bedeutung aufweist, in Gegenwart von bis-1,8-(dimethylamino)-naphthalen substituiert, um die Verbindung der Formel (XX) zu bilden:

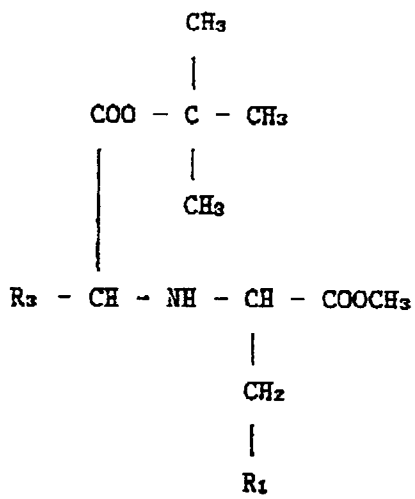

(XX)

g) die Verbindung der Formel (XX) einer selektiven alkalischen Entschützung unterwirft, um die Verbindung der Formel (XXI) zu bilden:

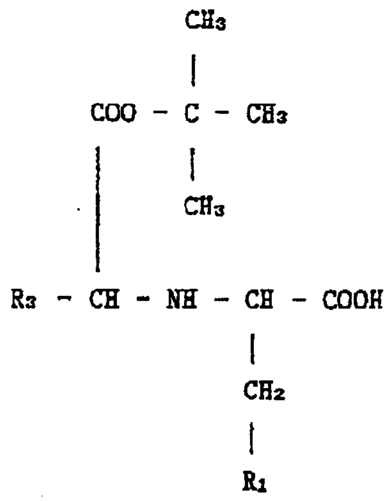

(XXI)

h) die Säure von Formel (XXI) mit dem gewünschten Aminoester der Formel (VI), worin $R_4$ eine Benzylgruppe ist, in Gegenwart eines Kopplungsagens, wie Dicyclohexylcarbodiimid koppelt, um die Verbindung der Formel (XXXI) zu bilden:

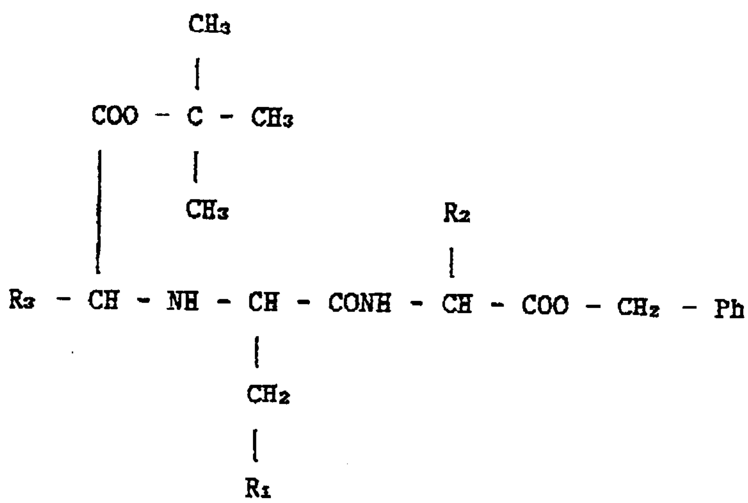

(XXII)

i) die Verbindung der Formel (XXII) hydriert, um die Verbindung der Formel (XXIII) zu bilden

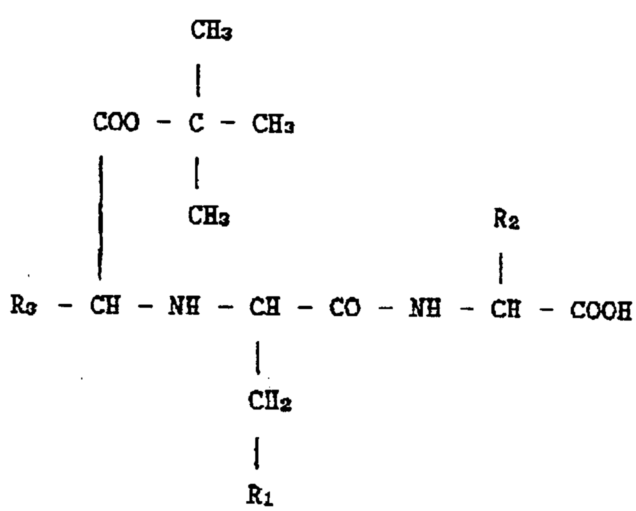

(XXIII)

j) dann die tert.-Butylestergruppe der Verbindung der Formel (XXIII) hydrolysiert, um die Disäure der Formel (Ia) zu bilden

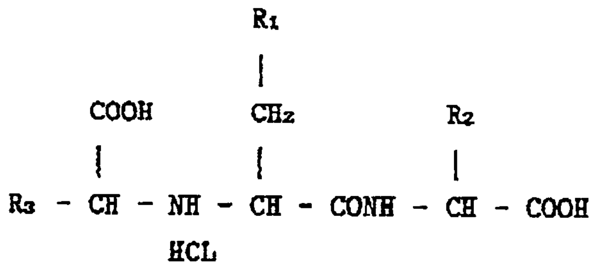

(Ia)

6. Verfahren zur Herstellung von Aminosäuren der Formel (Ia), in denen $R_1$ und $R_2$ die in Anspruch 1 angegebene Bedeutung aufweisen und X die Phosphonatgruppe bedeutet, dadurch gekennzeichnet, daß man nacheinander

a) den Acrylester der Formel (IV) verseift,

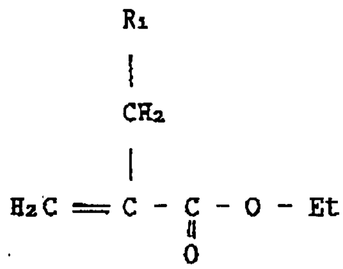

(IV)

und wobei auf die Verseifung eine Behandlung mit Thionylchlorid folgt, um das Acrylsäurechlorid der Formel (XXIV) zu bilden

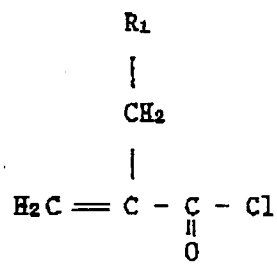

(XXIV)

b) das Säurechlorid der Formel (XXIV) mit dem gewünschten Aminosäureester der Formel (VI) in Gegenwart beispielsweise von Triethylamin koppelt, um die Verbindung der Formel (XXV) zu bilden

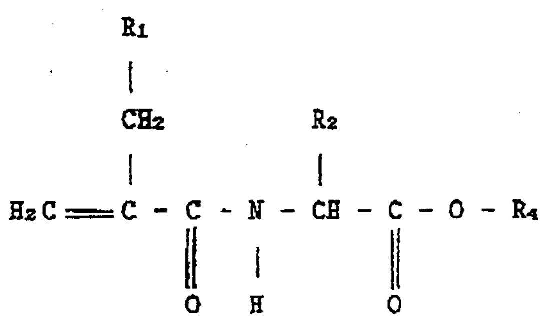

(XXV)

c) eine Michael-Addition mit einem Dialkylphosphit, beispielsweise mit Diethylphosphit, in Gegenwart von Natriumhydroxid durchführt, um die Verbindung der Formel (XXVI) zu bilden

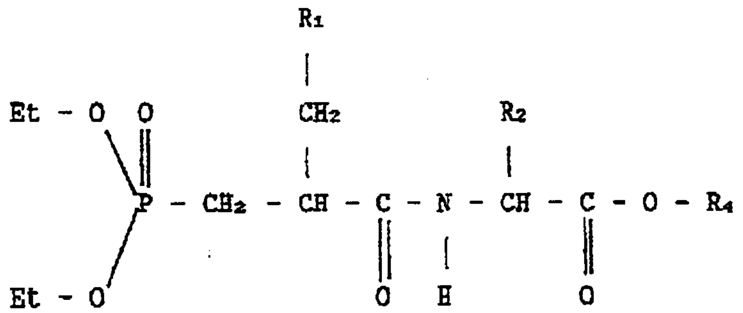

(XXVI)

d) dann die Scbutzgruppen der Verbindung (XXVI) hydrolysiert, um die Inhibitoren der Formel (Ia) zu bilden

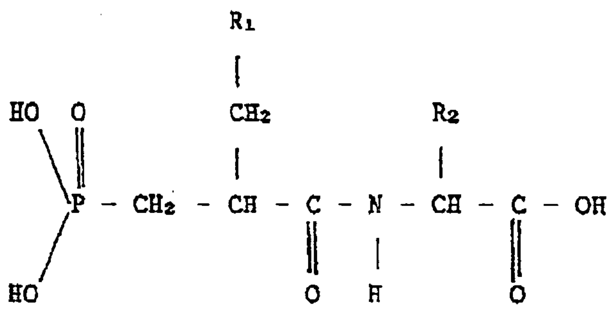

(Ia)

**7.** Aminosäurederivate der allgemeinen Formel (Ia) oder (Ib), in denen $R_1$, $R_2$, X und n die im Anspruch 1 angegebene Bedeutung aufweisen, dadurch gekennzeichnet, daß sie eine inhibierende Wirkung auf die Enzyme Enkephalinase und ACE aufweisen, die eine Inhibitorkonzentration $IC_{50}$ von unterhalb 10 nM aufweist.

**8.** Aminosäurederivate nach Anspruch 7, dadurch gekennzeichnet, daß, falls die Inhibitorkonzentration $IC_{50}$ für die beiden Wirkungen zwischen 1 und 10 nM liegt, das Verhältnis der Inhibitorkonzentration $IC_{50}$ von Enkephalinase zur Inhibitorkonzentration $IC_{50}$ von ACE kleiner als 4 ist, damit das Derivat gleichwirksam ist.

**9.** Aminosäurederivate der allgemeinen Formal (Ia) oder (Ib), in denen X eine Mercaptomethylgruppe bedeutet, $R_1$ und $R_2$ die in Anspruch 1 angegebenen Bedeutangen aufweisen, dadurch gekennzeichnet, daß nach Entschützen der Mercaptomethyl- und Carboxylgruppen verwendet werden.

**10.** Aminosäurederivate nach Anspruch 9, dadurch gekennzeichnet, daß sie die allgemeinen Formeln aufweisen:

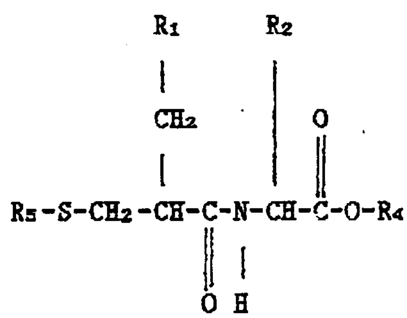

oder

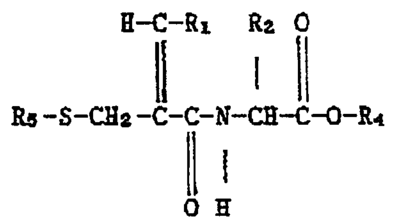

worin $R_4$ im besonderen steht für eine lineare oder verzweigte Alkylgruppe, eine Phenyl- oder Phenylalkylgruppe, wobei die beiden letzten Gruppen gegebenenfalls am Phenylkern einfach oder mehrfach substituiert sind, oder lineare oder verzweigte Substituenten, die ein oder mehrere Sauerstoffatome aufweisen und worin $R_5$ im besonderen steht für einen linearen oder verzweigten aliphatischen Acylrest, einen aromatischen Acylrest, der gegebenenfalls einfach oder mehrfach substituiert ist, oder einen linearen oder verzweigten Acylrest, der ein oder mehrere Sauerstoffatome enthält.

**11.** Medikament mit einer die Enzyme Enkephalinase und ACE inhibierenden Wirkung, dadurch gekennzeichnet, daß es als Wirkstoff eine Verbindung aufweist, wie sie in einem der Ansprüche 1, 2 und 3 definiert ist.

**12.** Verwendung der Verbindungen der allgemeinen Formeln:

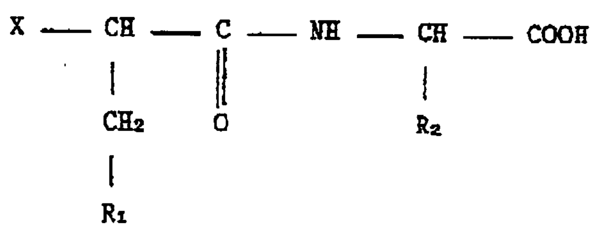

(Ia)

oder

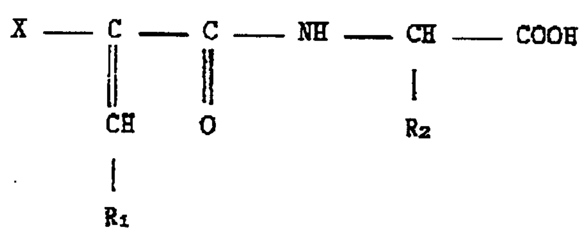

(Ib)

worin $R_1$ in der Formel (Ia) eine mit einem Halogenatom, insbesondere mit Fluor, einfach oder mehrfach substituierte Phenylgruppe, eine Biphenyl- gruppe oder eine der folgenden Gruppen bedeutet:

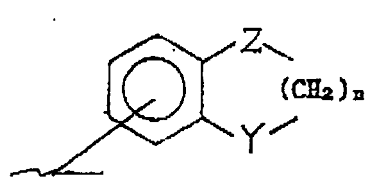

worin Z, Y und n die folgenden Bedeutungen aufweisen:

| Z | Y | n |
|---|---|---|
| O | O | 1 |
| O | $CH_2$ | 1 |
| $CH_2$ | $CH_2$ | 1 |
| O | O | 2 |
| $CH_2$ | $CH_2$ | 2 |
| O | $CH_2$ | 2 |

oder

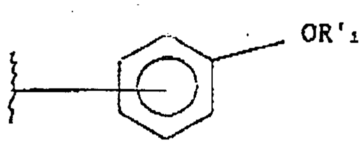

worin $R'_1$ ein Wassertoffatom, eine niedere Alkylgruppe, eine Phenylgruppe, eine niedere Phenylalkylengruppe bedeutet,

in der Formel (Ib) weist $R_1$ die zuvor definierte Bedeutung auf und kann darüber hinaus eine Phenylgruppe sein,

$R_2$ bedeutet ein Wasserstoffatom, eine niedrige Alkylgruppe, eine niedrige Hydroxyalkylengruppe, eine Phenylgruppe, eine niedrige Phenylalkylengruppe, eine niedrige Hydroxyphenylalkylengruppe, eine nied- rige Aminoalkylengruppe, eine niedrige Guanidinoalkylengruppe, eine nied- rige Mercaptoalkylengruppe, eine niedrige Alkyl-niedrige-Thioalkylengruppe, eine niedrige Imidazolylalkylengruppe, eine niedrige Indolylalkylengruppe, eine niedrige Carbamylalkylengruppe, eine niedrige Carboxyalkylengruppe oder eine der folgenden Gruppen:

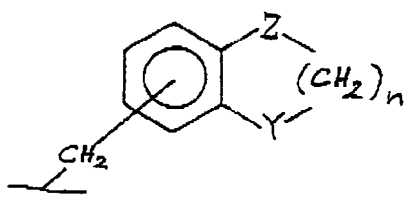

worin Z und n die folgenden Bedeutungen aufweisen:

| Z | Y | n |
|---|---|---|
| O | O | 1 |
| O | O | 2 |
| O | $CH_2$ | 1 |
| O | $CH_2$ | 2 |
| $CH_2$ | $CH_2$ | 1 |
| $CH_2$ | $CH_2$ | 2 |

X bedeutet eine Gruppe, die fur die Bildung eines Chelats mit dem Zinkatom der Enzyme Enkephalinase und ACE verantwortlich ist, und kann ausgewählt sein aus der Gruppe bestehend aus Mercaptomethyl, Hydroxamsäure, einem N-Carboxyalkyl der Formel

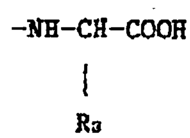

worin $R_3$ bedeutet einen niedrigen Alkylrest, einen niedrigen Alkylbenzylrest oder einen niedrigen Alkoxybenzylrest, Phosphoderivate der Formel:

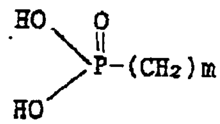

oder

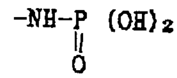

m = 0 oder 1

und pharmazeutisch verträgliche Additionssalze dieser Verbindungen,

zur Herstellung eines Medikamentes, das als Komkinationsinhibitor der Enzyme Enkephalinase und ACE wirkt.

13. Verwendung der Verbindungen nach Anspruch 12, dadurch gekenn- zeichnet, daß diese Verbindungen ansgewählt sind aus
   - N-(RS)-[1-Oxo-2-(mercaptomethyl)-3-(3- fluoro-phenyl)-propyl]-glycin,
   - N-(RS)-[1-Oxo-2-(mercaptomethyl)-3(3-fluoro-phenyl)-propyl]-(S)-alanin,
   - N-(RS)-[1-Oxo-2-(mercaptomethyl)-3-(3, 4-difluoro-phenyl)-propyl]-(S)-alanin,
   - N-(RS)-[1-Oxo-2-(mercaptomethyl)-3-(3-,5-difluoro-phenyl)-propyl]-glycin und dessen optisch reinen Formen,
   - N-(RS)[1-Oxo-2-(mercaptomethyl)-3-(3, 5-difluoro-phenyl)-propyl]- (S)-alanin.

14. Verwendung der Verbindungen der Formeln (Ia) oder (Ib) gemäß Anspruch 12, worin X eine Mercaptomethylgruppe bedeutet und $R_1$ und $R_2$ die in Anspruch 12 angegebenen Bedeutungen aufweisen, dadurch gekennzeichnet, daß diese Verbindungen nach Schützen der Mercaptomethyl- und Carboxylgruppen bereitgestellt werden.

15. Verwendung der Verbindungen nach Anspruch 14, dadurch gekenn- zeichnet, daß sie die in Anspruch 10 angegebenen allgemeinen Formeln aufweisen, in denen $R_1$ und $R_2$ die in dem Anspruch 12 angegebene Bedeutung aufweisen und $R_4$ und $R_5$ die in Anspruch 10 angegebenen Bedeutungen aufweisen.